# EUROPEAN PATENT SPECIFICATION

(11) **EP 2 217 697 B1**
(45) Date of publication and mention of the grant of the patent: **10.06.2015**
(21) Application number: 08848257.5
(22) Date of filing: 10.11.2008
(51) Int. Cl.: C12N 5/00, A01N 37/18, A61K 31/70, C07K 14/705, A61K 38/17, C07K 14/46

(54) **USE OF LINGO-4 ANTAGONISTS IN THE TREATMENT OF CONDITIONS INVOLVING DEMYELINATION**
VERWENDUNG VON LINGO-4-ANTAGONISTEN BEI DER BEHANDLUNG VON LEIDEN MIT DEMYELINIERUNG
UTILISATION DES ANTAGONISTES DE LINGO-4 DANS LE TRAITEMENT D'ÉTATS METTANT EN JEU UNE DÉMYÉLINATION

(30) Priority: 08.11.2007 US 986492 P
(43) Date of publication of application: 18.08.2010
(73) Proprietor: Biogen MA Inc., Cambridge, MA 02142 (US)
(72) Inventor: MI, Sha, Belmont, MA 02478 (US); PEPINSKY, R., Blake, Arlington, MA 02474 (US); MCCOY, John, Reading, MA 01867 (US)
(74) Representative: Adams, Harvey Vaughan John
(86) International application number: PCT/US2008/012620
(87) International publication number: WO 2009/061500

(56) References cited:
- WO-A1-2006/136006
- WO-A2-03/054152
- WO-A2-2007/092370
- US-A- 5 605 690
- US-A1- 2006 067 935
- US-A1- 2007 178 088
- US-A1- 2007 213 290
- MI S ET AL: "LINGO-1 Negatively Regulates Myelination by Oligodendrocytes", NATURE NEUROSCIENCE, NATURE AMERICA, INC, US, vol. 8, no. 6, 1 June 2005 (2005-06-01), pages 745-751, XP003005584, ISSN: 1097-6256, DOI: 10.1038/NN1460
- MI SHA ET AL: "LINGO-1 antagonist promotes spinal cord remyelination and axonal integrity in MOG-induced experimental autoimmune encephalomyelitis", NATURE MEDICINE, NATURE PUBLISHING GROUP, NEW YORK, NY, US, vol. 13, no. 10, 30 September 2007 (2007-09-30), pages 1228-1233, XP002525677, ISSN: 1078-8956, DOI: 10.1038/NM1664 [retrieved on 2007-09-30]
- LEE XINHUA ET AL: "NGF REGULATES THE EXPRESSION OF AXONAL LINGO-1 TO INHIBIT OLIGODENDROCYTE DIFFERENTIATION AND MYELINATION", JOURNAL OF NEUROSCIENCE, NEW YORK, NY, US, vol. 27, no. 1, 3 January 2007 (2007-01-03), pages 220-225, XP002559457, ISSN: 0270-6474, DOI: 10.1523/JNEUROSCI.4175-06.2007
- HAINES ET AL: "Expression of the Lingo/LERN gene family during mouse embryogenesis", GENE EXPRESSION PATTERNS, ELSEVIER, AMSTERDAM, NL, vol. 8, no. 2, 22 October 2007 (2007-10-22), pages 79-86, XP022418642, ISSN: 1567-133X, DOI: 10.1016/J.MODGEP.2007.10.003

## Description

### BACKGROUND OF THE INVENTION

### Field of the Invention

This invention relates to neurobiology, neurology and pharmacology. More particularly, it relates to a LINGO-4 antagonist for use in treating demyelination, dysmyelination, and central nervous system (CNS) diseases, such as multiple sclerosis.

The invention also relates to in vitro methods for promoting proliferation, differentiation, or survival of oligodendrocytes and myelination of neurons by the administration of a LINGO-4 antagonist.

### Background Art

Nerve cell function is influenced by contact between neurons and other cells in their immediate environment (Rutishauser et al., 1988, Physiol. Rev. 68:819). These cells include specialized glial cells, oligodendrocytes in the central nervous system (CNS), and Schwann cells in the peripheral nervous system (PNS), which sheathe the neuronal axon with myelin (Lemke, 1992, in An Introduction to Molecular Neurobiology, Z. Hall, Ed., p. 281, Sinauer).

The formation of the myelin sheath is an exquisite and dynamic example of cell-cell interaction that involves the myelin-forming cell and the neuronal axon. It is generally thought that during development axons control whether they will become myelinated by expressing appropriate signals to either promote or inhibit this process (Colello and Pott, Mol. Neurobiol. 15:83-100 (1997)).

CNS neurons have the inherent potential to regenerate after injury, but they are inhibited from doing so by inhibitory proteins present in myelin (Brittis et al., 2001, Neuron 30:11-14; Jones et al, 2002, J. Neurosci. 22:2792-2803; Grimpe et al, 2002, J. Neurosci.:22:3144-3160).

Several myelin inhibitory proteins found on oligodendrocytes have been characterized. Known examples of myelin inhibitory proteins include NogoA (Chen et al., Nature, 2000, 403, 434-439; Grandpre et al., Nature 2000, 403, 439-444), myelin associated glycoprotein (MAG) (McKerracher et al., 1994, Neuron 13:805-811; Mukhopadhyay et al., 1994, Neuron 13:757-767) and oligodendrocyte-myelin glycoprotein (OM-gp), Mikol et al., 1988, J. Cell. Biol.106:1273-1279). Each of these proteins has been separately shown to be a ligand for the neuronal Nogo receptor-1 (NgR1) (Wang et al., Nature 2002, 417, 941-944; Grandpre et al., Nature 2000, 403, 439-444; Chen et al., Nature, 2000, 403, 434-439; Domeniconi et al., Neuron 2002, published online June 28, 2002).

Many diseases of the nervous system are associated with demyelination and dysmyelination, including multiple sclerosis (MS), progressive multifocal leukoencephalopathy (PML), encephalomyelitis (EPL), central pontine myelolysis (CPM), Wallerian Degeneration and some inherited diseases such as adrenoleukodystrophy, Alexander's disease, and Pelizaeus Merzbacher disease (PMZ). Among these diseases, MS is the most widespread, affecting approximately 2.5 million people worldwide.

MS generally begins with a relapsing-remitting pattern of neurologic involvement, which then progresses to a chronic phase with increasing neurological damage. MS is associated with the destruction of myelin, oligodendrocytes and axons localized to chronic lesions. The demyelination observed in MS is not always permanent and remyelination has been documented in early stages of the disease. Remyelination of neurons requires oligodendrocytes.

Various disease-modifying treatments are available for MS, including the use of corticosteroids and immunomodulators such as interferon beta. In addition, because of the central role of oligodendrocytes and myelination in MS, there have been efforts to develop therapies to increase oligodendrocyte numbers or enhance myelination. *See, e.g*., Cohen et al., U.S. Pat. No. 5,574,009; Chang et al., N. Engl. J. Med. 346:165-73 (2002). However, there remains an urgent need to devise additional therapies for MS.

### BRIEF SUMMARY OF THE INVENTION

The present disclosure is based on the discovery that LINGO-4 (DAAT9248, Leucine Rich Repeat Neuronal 6D, LRRN6D, PRO34002, or Q6UY18) is expressed in oligodendrocytes and negatively regulates oligodendrocyte differentiation and axon myelination. Based on the disclosure that is contained herein, the present invention provides a composition comprising a LINGO-4 antagonist for use in the treatment of a disease, disorder, or injury involving demyelination or dysmyelination selected from the group consisting of multiple sclerosis (MS), progressive multifocal leukoencephalopathy (PML), encephalomyelitis (EPL), central pontine myelinolysis (CPM), adrenoleukodystrophy, Alexander's disease, Pelizaeus Merzbacher disease (PMZ), Wallerian Degeneration, optic neuritis, transverse myelitis, amylotrophic lateral sclerosis (ALS), Huntington's disease, Alzheimer's disease, Parkinson's disease, spinal cord injury, traumatic brain injury, post radiation injury, neurologic complications of chemotherapy, stroke, acute ischemic optic neuropathy, isolated vitamin E deficiency syndrome, Bassen-Kornzweig syndrome, Marchiafava-Bignami syndrome, metachromatic leukodystrophy, trigeminal neuralgia, and Bell's palsy, wherein the LINGO-4 antagonist is selected from the group consisting of:
(i) a soluble LINGO-4 polypeptide, optionally fused to a non-LINGO-4 moiety;
(ii) a LINGO-4 antibody or an antigen-binding fragment thereof;
(iii) a LINGO-4 antagonist polynucleotide selected from the group consisting of
   (a) an antisense polynucleotide;
   (b) a ribozyme;
   (c) a small interfering RNA (siRNA); and
   (d) a small-hairpin RNA (shRNA);
(iv) a LINGO-4 aptamer; and
(v) a combination of two or more of said LINGO-4 antagonists;
wherein the non-LINGO-4 moiety is selected from the group consisting of immunoglobulin, serum albumin, a targeting polypeptide, a reporter polypeptide, a purification-facilitating polypeptide, polyalkylene glycol, a sugar polymer, and a combination of two or more of said non-LINGO-4 moieties.

In a related aspect, the present invention provides a polynucleotide that encodes the soluble LINGO-4 polypeptide, the LINGO-4 antibody or fragment thereof, or the LINGO-4 aptamer of the invention, for use in the treatment of a disease, disorder, or injury involving demyelination or dysmyelination selected from the group consisting of multiple sclerosis (MS), progressive multifocal leukoencephalopathy (PML), encephalomyelitis (EPL), central pontine myelinolysis (CPM), adrenoleukodystrophy, Alexander's disease, Pelizaeus Merzbacher disease (PMZ), Wallerian Degeneration, optic neuritis, transverse myelitis, amylotrophic lateral sclerosis (ALS), Huntington's disease, Alzheimer's disease, Parkinson's disease, spinal cord injury, traumatic brain injury, post radiation injury, neurologic complications of chemotherapy, stroke, acute ischemic optic neuropathy, isolated vitamin E deficiency syndrome, Bassen-Kornzweig syndrome, Marchiafava-Bignami syndrome, metachromatic leukodystrophy, trigeminal neuralgia, and Bell's palsy, where the polynucleotide is operably linked to an expression control sequence, allowing expression of said LINGO-4 antagonist.

In a related aspect, the present invention provides an *in vitro* method of promoting differentiation or survival of oligodendrocytes comprising contacting oligodendrocytes with a composition comprising a LINGO-4 antagonist selected from the group consisting of:
(i) a soluble LINGO-4 polypeptide, optionally fused to a non-LINGO-4 moiety;
(ii) a LINGO-4 antibody or an antigen-binding fragment thereof;
(iii) a LINGO-4 antagonist polynucleotide selected from the group consisting of
   (a) an antisense polynucleotide;
   (b) a ribozyme;
   (c) a small interfering RNA (siRNA); and
   (d) a small-hairpin RNA (shRNA);
(iv) a LINGO-4 aptamer; and
(v) a combination of two or more of said LINGO-4 antagonists;
wherein the non-LINGO-4 moiety is selected from the group consisting of immunoglobulin, serum albumin, a targeting polypeptide, a reporter polypeptide, a purification-facilitating polypeptide, polyalkylene glycol, a sugar polymer, and a combination of two or more of said non-LINGO-4 moieties.

In yet another aspect, the present invention provides an *in vitro* method of promoting myelination of neurons or of preventing demyelination of neurons comprising contacting a mixture of neurons and oligodendrocytes with a composition comprising a LINGO-4 antagonist selected from the group consisting of:
(i) a soluble LINGO-4 polypeptide, optionally fused to a non-LINGO-4 moiety;
(ii) a LINGO-4 antibody or an antigen-binding fragment thereof;
(iii) a LINGO-4 antagonist polynucleotide selected from the group consisting of
   (a) an antisense polynucleotide;
   (b) a ribozyme;
   (c) a small interfering RNA (siRNA); and
   (d) a small-hairpin RNA (shRNA);
(iv) a LINGO-4 aptamer; and
(v) a combination of two or more of said LINGO-4 antagonists;
wherein the non-LINGO-4 moiety is selected from the group consisting of immunoglobulin, serum albumin, a targeting polypeptide, a reporter polypeptide, a purification-facilitating polypeptide, polyalkylene glycol, a sugar polymer, and a combination of two or more of said non-LINGO-4 moieties.

The present invention and embodiments thereof are set out in the appended claims.

In various embodiments, the LINGO-4 antagonist may be any molecule which interferes with the ability of LINGO-4 to negatively regulate survival, proliferation and differentiation of oligodendrocytes as well as myelination of neurons. In certain embodiments, the LINGO-4 antagonist is selected from the group consisting of a soluble LINGO-4 polypeptide, a LINGO-4 antibody, a LINGO-4 antagonist polynucleotide (*e.g.* RNA interference) and a LINGO-4 aptamer.

Certain soluble LINGO-4 polypeptides include, but are not limited to, LINGO-4 polypeptide fragments, variants, or derivatives thereof which lack a transmembrane domain. Soluble LINGO-4 polypeptides include polypeptides comprising (i) a LINGO-4 immunoglobulin (Ig) domain and (ii) a LINGO-4 Leucine-Rich Repeat (LRR) domain. In some embodiments, the soluble LINGO-4 polypeptide lacks a LINGO-4 Ig domain, a LINGO-4 LRR domain, and a transmembrane domain. In some embodiments, the soluble LINGO-4 polypeptide lacks a LINGO-4 Ig domain and a LINGO-4 transmembrane domain. Yet in some embodiments, the soluble LINGO-4 polypeptide comprises a LINGO-4 LRR domain. In some embodiments, the soluble LINGO-4 polypeptide comprises a LINGO-4 Ig domain. In some embodiments, the soluble LINGO-4 polypeptide comprises amino acid residues 30-486 or 30-491 of SEQ ID NO: 2.

In some instances, the LINGO-4 antagonist is administered by bolus injection or chronic infusion. In some instances, the soluble LINGO-4 polypeptide is administered directly into the central nervous system. In some instances, the soluble LINGO-4 polypeptide is administered directly into a chronic lesion of MS.

In some embodiments, the LINGO-4 antagonist is a fusion polypeptide comprising a non-LINGO-4 moiety. In some embodiments, the non-LINGO-4 moiety is selected from the group consisting of an antibody Ig moiety, a serum albumin moiety, a targeting moiety, a brain targeting moiety, a reporter moiety, and a purification-facilitating moiety. In some embodiments, the antibody Ig moiety is a hinge and Fc moiety.

In some embodiments, the soluble LINGO-4 polypeptides are conjugated to a polymer. In some embodiments, the polymer is selected from the group consisting of a polyalkylene glycol, a sugar polymer, and a polypeptide. In some embodiments, the polyalkylene glycol is polyethylene glycol (PEG). In some embodiments, the polypeptides and antibodies of the present invention are conjugated to 1, 2, 3 or 4 polymers. In some embodiments, the total molecular weight of the polymers is from 5,000 Da to 100,000 Da.

In some embodiments, the soluble LINGO-4 polypeptide is a cyclic peptide. In some embodiments, the cyclic peptide comprises a biotin molecule attached to the N-terminus and a cysteine residue attached to the C-terminus of said cyclic peptide. In some embodiments, the cyclic peptide comprises a cysteine residue attached to the N- and C-terminus of said cyclic peptide, wherein said N-terminal cysteine residue is acetylated.

In some embodiments, the LINGO antagonist comprises a LINGO-4 antibody, or fragment thereof. In some embodiments, the LINGO-4 antagonist comprises a LINGO-4 antagonist polynucleotide. In some embodiments, the LINGO-4 antagonist polynucleotide is selected from the group consisting of an antisense polynucleotide, a ribozyme, a small interfering RNA (siRNA), and a small-hairpin RNA (shRNA). In some embodiments, the LINGO-4 antagonist polynucleotide is an antisense polynucleotide comprising at least 10 bases complementary to the coding portion of the LINGO-4 mRNA. In another embodiment, the LINGO-4 antagonist is an aptamer.

### BRIEF DESCRIPTION OF THE DRAWINGS

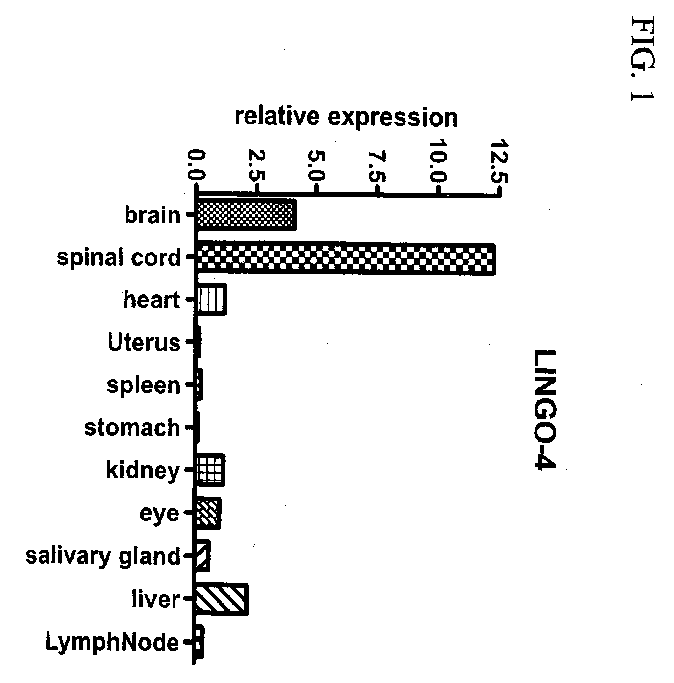

Figure 1 - Graph of transcription levels showing that LINGO-4 is highly expressed both in brain and spinal cord.

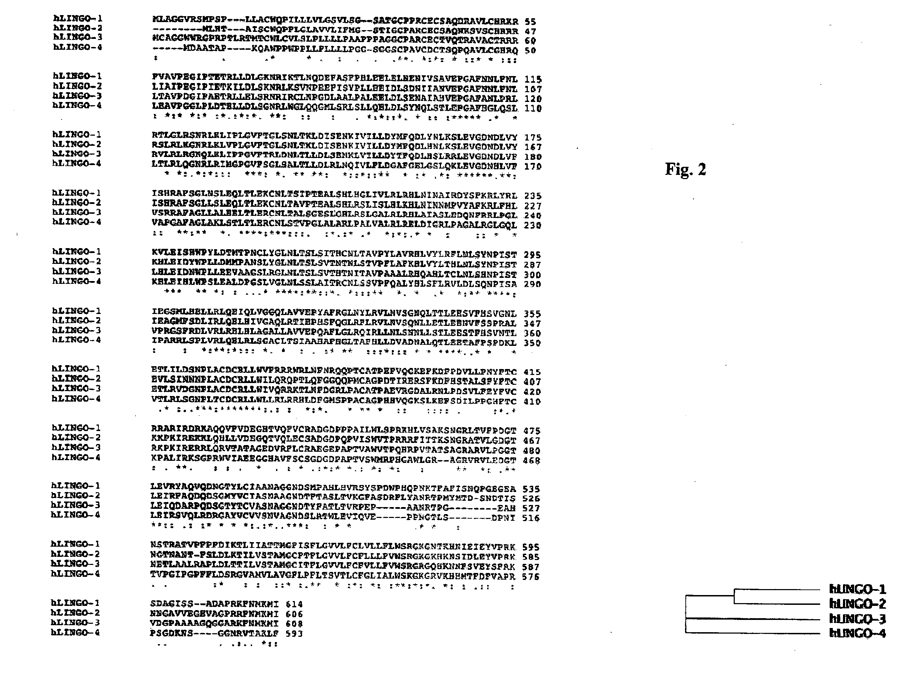

Figure 2 - The percent similarity and identity between hLINGO-1 and hLINGO-2 are 70.4% and 60.7%, respectively. The percent similarity and identity between hLINGO-1 and hLINGO-3 are 66.4% and 55.4%, respectively. The percent similarity and identity between hLINGO-1 and hLINGO-4 are 52.1% and 44.3%, respectively.

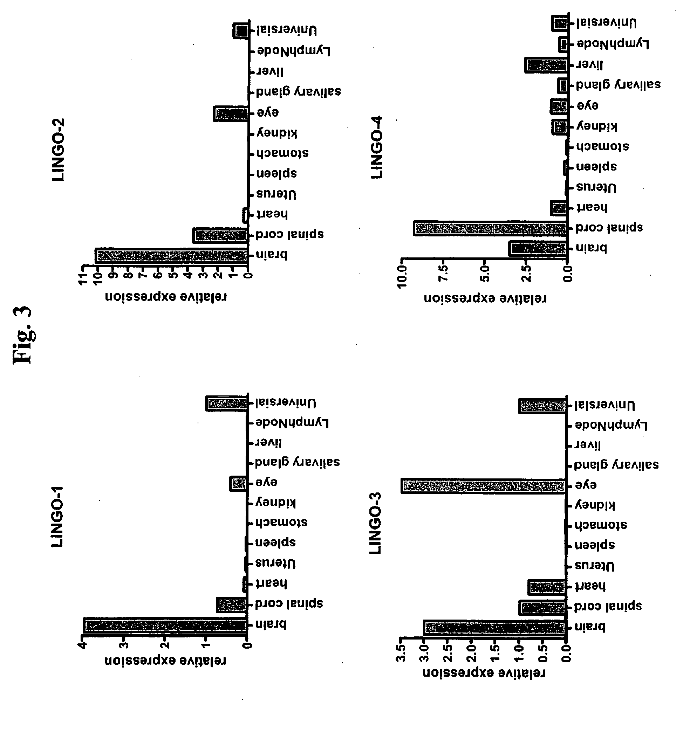

Figure 3 - Q-PCR of adult mouse tissues. LINGO-4 is highly expressed in brain and spinal cord of adult mouse tissues. Quantitation of mRNA expression of LINGO-4 was carried out by Q-PCR.

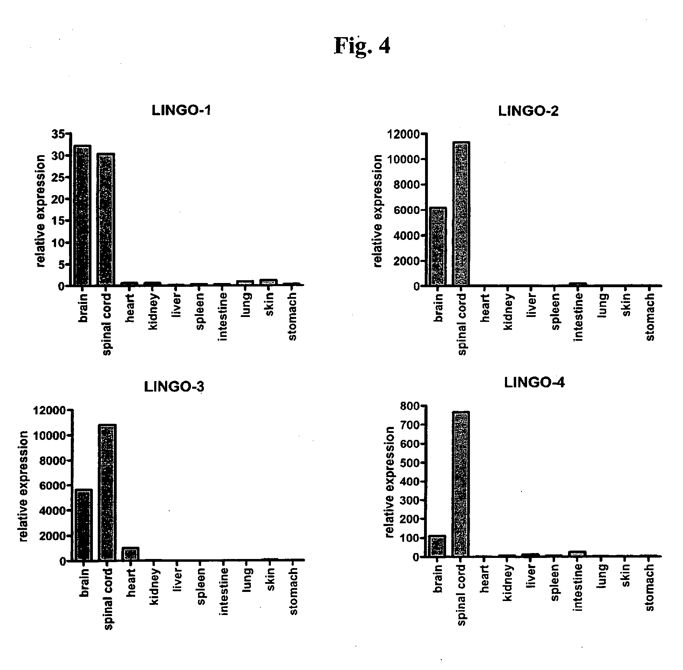

Figures 4 - Q-PCR of P6 mouse tissues. LINGO-4 is highly expressed in brain and spinal cord of postnatal 6 days (P6) mouse tissues. Quantitation of mRNA expression of LINGO-4 was carried out by Q-PCR.

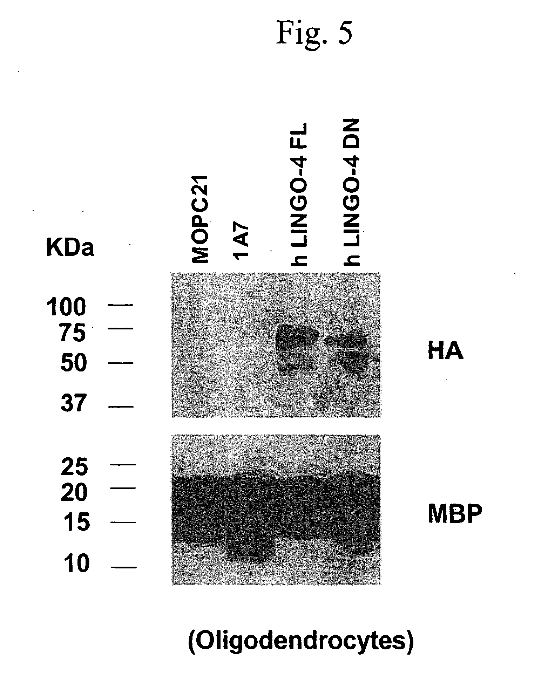

Figure 5 - DN LINGO-4 promotes oligodendrocyte differentiation. Western blots from rat oligodendrocyte cultures treated with exogenous MOPC21 (control) and 1A7 (anti-LINGO-1) monoclonal antibodies and oligodendrocytes cultures infected with hLINGO-4FL (full-length) and hLINGO-4 DN (dominant negative) lentivirus using anti-MBP and anti-HA antibodies (internal lentiviral control) to detect relative levels of myelin basic protein (MBP) expression.

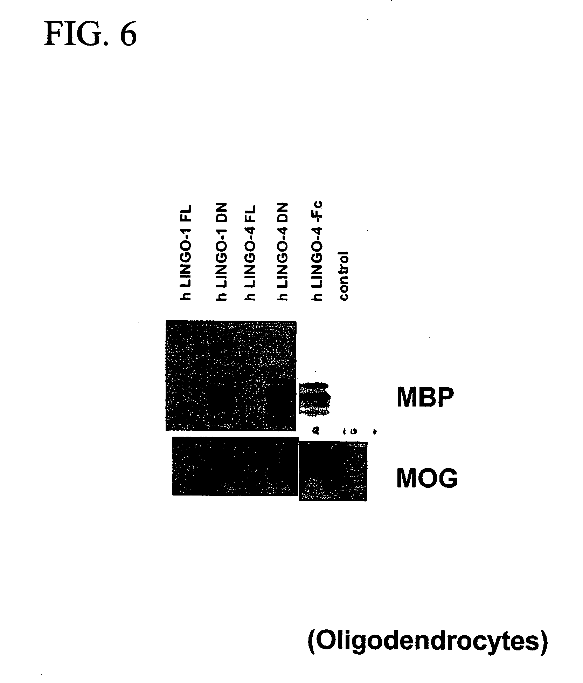

Figure 6 - DN LINGO-4 and LINGO-4-Fc promotes oligodendrocyte differentiation. Western blots from oligodendrocytes cultures infected with hLINGO-1 FL (full length), hLINGO-1 DN (dominant negative), hLINGO-4 FL (full length), and hLINGO-4-DN (dominant negative) lentivirus, and exogenous treatment of oligodendrocytes with hLINGO-4-Fc, and a control polypeptide using anti-MBP (mature oligodendrocytes) antibody and MOG antibody to detect the presence of both MBP and myelin-oligodendrocyte glycoprotein (MOG) proteins.

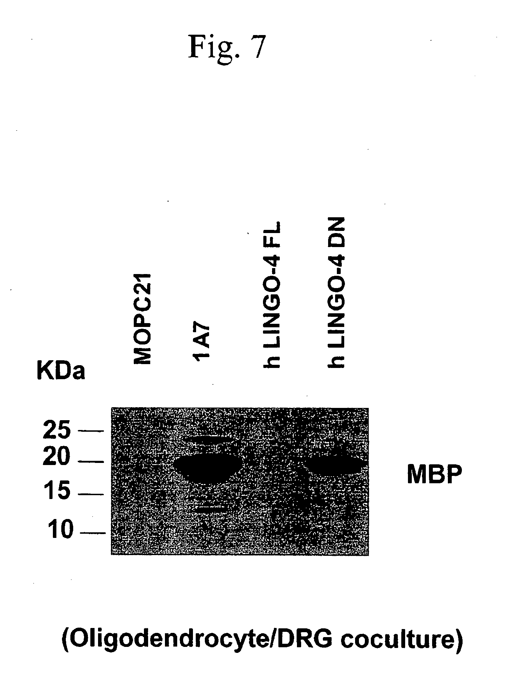

Figure 7 - DN LINGO-4 promotes oligodendrocyte myelination of neurons in co-culture. Western blot of cocultures of dorsal root ganglion (DRG) and oligodendrocytes treated with exogenous MOPC21 (negative control) and 1A7 (positive control) antibodies and cocultures infected with hLINGO-4FL (full-length), and hLINGO-4 DN (dominant negative) lentivirus using anti-MBP to detect the presence of the MBP protein.

### DETAILED DESCRIPTION OF THE INVENTION

### Definitions

Unless defined otherwise, all technical and scientific terms used herein have the same meaning as commonly understood by one of ordinary skill in the art to which this invention belongs. In case of conflict, the present application including the definitions will control. Unless otherwise required by context, singular terms shall include pluralities and plural terms shall include the singular.

Although methods and materials similar or equivalent to those described herein can be used in practice or testing of the present invention, suitable methods and materials are described below. The materials, methods and examples are illustrative only and are not intended to be limiting. Other features and advantages of the invention will be apparent from the detailed description and from the claims.

In order to further define this invention, the following terms and definitions are provided.

It is to be noted that the term "a" or "an" entity, refers to one or more of that entity; for example, "an immunoglobulin molecule," is understood to represent one or more immunoglobulin molecules. As such, the terms "a" (or "an"), "one or more," and "at least one" can be used interchangeably herein.

Throughout this specification and claims, the word "comprise," or variations such as "comprises" or "comprising," indicate the inclusion of any recited integer or group of integers but not the exclusion of any other integer or group of integers.

As used herein, the term "consists of," or variations such as "consist of" or "consisting of," as used throughout the specification and claims, indicate the inclusion of any recited integer or group of integers, but that no additional integer or group of integers may be added to the specified method, structure or composition.

As used herein, the term "consists essentially of," or variations such as "consist essentially of' or "consisting essentially of," as used throughout the specification and claims, indicate the inclusion of any recited integer or group of integers, and the optional inclusion of any recited integer or group of integers that do not materially change the basic or novel properties of the specified method, structure or composition.

As used herein, a "therapeutically effective amount" refers to an amount effective, at dosages and for periods of time necessary, to achieve a desired therapeutic result. A therapeutic result may be, e.g., lessening of symptoms, prolonged survival, improved mobility, and the like. A therapeutic result need not be a "cure".

As used herein, a "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

As used herein, a "polynucleotide" can contain the nucleotide sequence of the full length cDNA sequence, including the untranslated 5' and 3' sequences, the coding sequences, as well as fragments, epitopes, domains, and variants of the nucleic acid sequence. The polynucleotide can be composed of any polyribonucleotide or polydeoxyribonucleotide, which may be unmodified RNA or DNA or modified RNA or DNA. For example, polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotides can be composed of triple-stranded regions comprising RNA or DNA or both RNA and DNA. Polynucleotides may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

In the present invention, a polypeptide can be composed of amino acids joined to each other by peptide bonds or modified peptide bonds, *i.e.,* peptide isosteres, and may contain amino acids other than the 20 gene-encoded amino acids (*e.g.* non-naturally occurring amino acids). The polypeptides of the present invention may be modified by either natural processes, such as posttranslational processing, or by chemical modification techniques which are well known in the art. Such modifications are well described in basic texts and in more detailed monographs, as well as in a voluminous research literature. Modifications can occur anywhere in the polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched , for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (*See,* for instance, Proteins - Structure And Molecular Properties, 2nd Ed., T.E. Creighton, W.H. Freeman and Company, New York (1993); Posttranslational Covalent Modification of Proteins, B.C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

The terms "fragment," "variant," "derivative" and "analog" when referring to a LINGO-4 antagonist include any antagonist molecules which promote proliferation, differentiation or survival of oligodendrocytes and neurite outgrowth or survival of a CNS neuron. These terms also include any antagonist molecules which promote myelination of neurons. Soluble LINGO-4 polypeptides may include LINGO-4 proteolytic fragments, deletion fragments and in particular, fragments which more easily reach the site of action when delivered to an animal. Polypeptide fragments further include any portion of the polypeptide which comprises an antigenic or immunogenic epitope of the native polypeptide, including linear as well as three-dimensional epitopes. Soluble LINGO-4 polypeptides may comprise variant LINGO-4 regions, including fragments as described above, and also polypeptides with altered amino acid sequences due to amino acid substitutions, deletions, or insertions. Variants may occur naturally, such as an allelic variant. By an "allelic variant" is intended alternate forms of a gene occupying a given locus on a chromosome of an organism. Genes II, Lewin, B., ed., John Wiley & Sons, New York (1985). Non-naturally occurring variants may be produced using art-known mutagenesis techniques. Soluble LINGO-4 polypeptides may comprise conservative or non-conservative amino acid substitutions, deletions or additions. LINGO-4 antagonists may also include derivative molecules. For example, soluble LINGO-4 polypeptides may include LINGO-4 regions which have been altered so as to exhibit additional features not found on the native polypeptide. Examples include fusion proteins and protein conjugates.

In the present invention, a "polypeptide fragment" refers to a short amino acid sequence of a LINGO-4 polypeptide. Protein fragments may be "free-standing," or comprised within a larger polypeptide of which the fragment forms a part of region. Representative examples of polypeptide fragments of the invention, include, for example, fragments comprising about 5 amino acids, about 10 amino acids, about 15 amino acids, about 20 amino acids, about 30 amino acids, about 40 amino acids, about 50 amino acids, about 60 amino acids, about 70 amino acids, about 80 amino acids, about 90 amino acids, and about 100 amino acids in length.

*Antibody or Immunoglobulin.* In one embodiment, the LINGO-4 antagonists for use in the treatment methods disclosed herein are "antibody" or "immunoglobulin" molecules, or immunospecific fragments thereof, *e.g.,* naturally occurring antibody or immunoglobulin molecules or engineered antibody molecules or fragments that bind antigen in a manner similar to antibody molecules. The terms "antibody" and "immunoglobulin" are used interchangeably herein. An antibody or immunoglobulin comprises at least the variable domain of a heavy chain, and normally comprises at least the variable domains of a heavy chain and a light chain. Basic immunoglobulin structures in vertebrate systems are relatively well understood. *See, e.g.,* Harlow et al., Antibodies: A Laboratory Manual, (Cold Spring Harbor Laboratory Press, 2nd ed. 1988).

As will be discussed in more detail below, the term "immunoglobulin" comprises five broad classes of polypeptides that can be distinguished biochemically. All five classes are clearly within the scope of the present invention, the following discussion will generally be directed to the IgG class of immunoglobulin molecules. With regard to IgG, a standard immunoglobulin molecule comprises two identical light chain polypeptides of molecular weight approximately 23,000 Daltons, and two identical heavy chain polypeptides of molecular weight 53,000-70,000. The four chains are typically joined by disulfide bonds in a "Y" configuration wherein the light chains bracket the heavy chains starting at the mouth of the "Y" and continuing through the variable region.

Both the light and heavy chains are divided into regions of structural and functional homology. The terms "constant" and "variable" are used functionally. In this regard, it will be appreciated that the variable domains of both the light (V_{L}) and heavy (V_{H}) chain portions determine antigen recognition and specificity. Conversely, the constant domains of the light chain (C_{L}) and the heavy chain (C_{H}1, C_{H}2 or C_{H}3) confer important biological properties such as secretion, transplacental mobility, Fc receptor binding, complement binding, and the like. By convention the numbering of the constant region domains increases as they become more distal from the antigen binding site or amino-terminus of the antibody. The N-terminal portion is a variable region and at the C-terminal portion is a constant region; the C_{H}3 and C_{L} domains actually comprise the carboxy-terminus of the heavy and light chain, respectively.

Light chains are classified as either kappa or lambda (κ, λ). Each heavy chain class may be bound with either a kappa or lambda light chain. In general, the light and heavy chains are covalently bonded to each other, and the "tail" portions of the two heavy chains are bonded to each other by covalent disulfide linkages or non-covalent linkages when the immunoglobulins are generated either by hybridomas, B cells or genetically engineered host cells. In the heavy chain, the amino acid sequences run from an N-terminus at the forked ends of the Y configuration to the C-terminus at the bottom of each chain. Those skilled in the art will appreciate that heavy chains are classified as gamma, mu, alpha, delta, or epsilon, (γ, µ, α, δ, ε) with some subclasses among them (*e.g*., γ1-γ4). It is the nature of this chain that determines the "class" of the antibody as IgG, IgM, IgA IgG, or IgE, respectively. The immunoglobulin subclasses (isotypes) *e.g.,* IgG₁, IgG₂, IgG₃, IgG₄, IgA₁, etc. are well characterized and are known to confer functional specialization. Modified versions of each of these classes and isotypes are readily discernable to the skilled artisan in view of the instant disclosure and, accordingly, are within the scope of the instant invention.

As indicated above, the variable region allows the antibody to selectively recognize and specifically bind epitopes on antigens. That is, the V_{L} domain and V_{H} domain of an antibody combine to form the variable region that defines a three dimensional antigen binding site. This quaternary antibody structure forms the antigen binding site present at the end of each arm of the Y. More specifically, the antigen binding site is defined by three complementary determining regions (CDRs) on each of the V_{H} and V_{L} chains. In some instances, *e.g.,* certain immunoglobulin molecules derived from camelid species or engineered based on camelid immunoglobulins, a complete immunoglobulin molecule may consist of heavy chains only, with no light chains. *See, e.g.,* Hamers-Casterman et al., Nature 363:446-448 (1993).

In naturally occurring antibodies, the six "complementarity determining regions" or "CDRs" present in each antigen binding domain are short, non-contiguous sequences of amino acids that are specifically positioned to form the antigen binding domain as the antibody assumes its three dimensional configuration in an aqueous environment. The remainder of the amino acids in the antigen binding domains, referred to as "framework" regions, show less intermolecular variability. The framework regions largely adopt a β-sheet conformation and the CDRs form loops which connect, and in some cases form part of, the β-sheet structure. Thus, framework regions act to form a scaffold that provides for positioning the CDRs in correct orientation by inter-chain, non-covalent interactions. The antigen binding domain formed by the positioned CDRs defines a surface complementary to the epitope on the immunoreactive antigen. This complementary surface promotes the non-covalent binding of the antibody to its cognate epitope. The amino acids comprising the CDRs and the framework regions, respectively, can be readily identified for any given heavy or light chain variable region by one of ordinary skill in the art, since they have been precisely defined (*see,* "Sequences of Proteins of Immunological Interest," Kabat, E., et al., U.S. Department of Health and Human Services, (1983); and Chothia and Lesk, J. Mol. Biol., 196:901-917 (1987)).

In camelid species, however, the heavy chain variable region, referred to as V_{H}H, forms the entire CDR. The main differences between camelid V_{H}H variable regions and those derived from conventional antibodies (V_{H}) include (a) more hydrophobic amino acids in the light chain contact surface of V_{H} as compared to the corresponding region in V_{H}H, (b) a longer CDR3 in V_{H}H, and (c) the frequent occurrence of a disulfide bond between CDR1 and CDR3 in V_{H}H.

In one embodiment, an antigen binding molecule comprises at least one heavy or light chain CDR of an antibody molecule. In another embodiment, an antigen binding molecule of the invention comprises at least two CDRs from one or more antibody molecules. In another embodiment, an antigen binding molecule comprises at least three CDRs from one or more antibody molecules. In another embodiment, an antigen binding molecule of the invention comprises at least four CDRs from one or more antibody molecules. In another embodiment, an antigen binding molecule comprises at least five CDRs from one or more antibody molecules. In another embodiment, an antigen binding molecule comprises at least six CDRs from one or more antibody molecules. Exemplary antibody molecules comprising at least one CDR that can be included in the subject antigen binding molecules are known in the art and exemplary molecules are described herein.

Antibodies or immunospecific fragments thereof for use in the invention include, but are not limited to, polyclonal, monoclonal, multispecific, human, humanized, primatized, or chimeric antibodies, single chain antibodies, epitope-binding fragments, *e.g.,* Fab, Fab' and F(ab')2, Fd, Fvs, single-chain Fvs (scFv), single-chain antibodies, disulfide-linked Fvs (sdFv), fragments comprising either a V_{L} or V_{H} domain, fragments produced by a Fab expression library, and anti-idiotypic (anti-Id) antibodies (including, *e.g.,* anti-Id antibodies to binding molecules disclosed herein). ScFv molecules are known in the art and are described, *e.g.,* in US patent 5,892,019. Immunoglobulin or antibody molecules of the invention can be of any type (*e.g*., IgG, IgE, IgM, IgD, IgA, and IgY), class (*e.g*., IgG₁, IgG₂, IgG₃, IgG₄, IgA₁ and IgA₂) or subclass of immunoglobulin molecule.

Antibody fragments, including single-chain antibodies, may comprise the variable region(s) alone or in combination with the entirety or a portion of the following: hinge region, C_{H}1, C_{H}2, and C_{H}3 domains. Also included are antigen-binding fragments also comprising any combination of variable region(s) with a hinge region, C_{H}1, C_{H}2, and C_{H}3 domains. Antibodies or immunospecific fragments thereof for use in the diagnostic and therapeutic methods disclosed herein may be from any animal origin including birds and mammals. In certain embodiments, the antibodies are human, murine, donkey, rabbit, goat, guinea pig, camel, llama, horse, or chicken antibodies. In another embodiment, the variable region may be condricthoid in origin (*e.g*., from sharks). As used herein, "human" antibodies include antibodies having the amino acid sequence of a human immunoglobulin and include antibodies isolated from human immunoglobulin libraries or from animals transgenic for one or more human immunoglobulins and that do not express endogenous immunoglobulins, as described *infra* and, for example in, U.S. Pat. No. 5,939,598 by Kucherlapati et al.

As used herein, the term "heavy chain portion" includes amino acid sequences derived from an immunoglobulin heavy chain. A polypeptide comprising a heavy chain portion comprises at least one of: a C_{H}1 domain, a hinge (*e.g*., upper, middle, and/or lower hinge region) domain, a C_{H}2 domain, a C_{H}3 domain, or a variant or fragment thereof. For example, a binding polypeptide for use in the invention may comprise a polypeptide chain comprising a C_{H}1 domain; a polypeptide chain comprising a C_{H}1 domain, at least a portion of a hinge domain, and a C_{H}2 domain; a polypeptide chain comprising a C_{H}1 domain and a C_{H}3 domain; a polypeptide chain comprising a C_{H}1 domain, at least a portion of a hinge domain, and a C_{H}3 domain, or a polypeptide chain comprising a C_{H}1 domain, at least a portion of a hinge domain, a C_{H}2 domain, and a C_{H}3 domain. In another embodiment, a polypeptide comprises a polypeptide chain comprising a C_{H}3 domain. Further, a binding polypeptide for use in the invention may lack at least a portion of a C_{H}2 domain (*e.g*., all or part of a C_{H}2 domain). As set forth above, it will be understood by one of ordinary skill in the art that these domains (*e.g.,* the heavy chain portions) may be modified such that they vary in amino acid sequence from the naturally occurring immunoglobulin molecule.

In certain LINGO-4 antagonist antibodies or immunospecific fragments thereof for use in the treatment methods disclosed herein, the heavy chain portions of one polypeptide chain of a multimer are identical to those on a second polypeptide chain of the multimer. Alternatively, heavy chain portion-containing monomers for use in the invention are not identical. For example, each monomer may comprise a different target binding site, forming, for example, a bispecific antibody.

The heavy chain portions of a binding polypeptide for use in the diagnostic and treatment methods disclosed herein may be derived from different immunoglobulin molecules. For example, a heavy chain portion of a polypeptide may comprise a C_{H}1 domain derived from an IgG₁ molecule and a hinge region derived from an IgG₃ molecule. In another example, a heavy chain portion can comprise a hinge region derived, in part, from an IgG₁ molecule and, in part, from an IgG₃ molecule. In another example, a heavy chain portion can comprise a chimeric hinge derived, in part, from an IgG₁ molecule and, in part, from an IgG₄ molecule.

As used herein, the term "light chain portion" includes amino acid sequences derived from an immunoglobulin light chain. Typically, the light chain portion comprises at least one of a V_{L} or C_{L} domain.

An isolated nucleic acid molecule encoding a non-natural variant of a polypeptide derived from an immunoglobulin (*e.g.,* an immunoglobulin heavy chain portion or light chain portion) can be created by introducing one or more nucleotide substitutions, additions or deletions into the nucleotide sequence of the immunoglobulin such that one or more amino acid substitutions, additions or deletions are introduced into the encoded protein. Mutations may be introduced by standard techniques, such as site-directed mutagenesis and PCR-mediated mutagenesis. For example, conservative amino acid substitutions are made at one or more non-essential amino acid residues.

Antibodies or immunospecific fragments thereof for use in the treatment methods disclosed herein may also be described or specified in terms of their binding affinity to a polypeptide of the invention. For example, binding affinities include those with a dissociation constant or Kd less than 5 x 10⁻² M, 10⁻² M, 5 x 10⁻³ M, 10⁻³ M, 5 x 10⁻⁴ M, 10⁻⁴ M, 5 x 10⁻⁵ M, 10⁻⁵ M, 5 x 10⁻⁶ M, 10⁻⁶ M, 5 x 10⁻⁷ M, 10⁻⁷ M, 5 x 10⁻⁸ M, 10⁻⁸ M, 5 x 10⁻⁹ M, 10⁻⁹ M, 5 x 10⁻¹⁰ M, 10⁻¹⁰ M, 5 x 10⁻¹¹ M, 10⁻¹¹ M, 5 x 10⁻¹² M, 10⁻¹² M, 5 x 10⁻¹³ M, 10⁻¹³ M, 5 x 10⁻¹⁴ M, 10⁻¹⁴ M_{,} 5 x 10⁻¹⁵ M, or 10⁻¹⁵ M.

Antibodies or immunospecific fragments thereof for use in the treatment methods disclosed herein act as antagonists of LINGO-4 as described herein. For example, an antibody for use in the present invention may function as an antagonist, blocking or inhibiting the suppressive activity of the LINGO-4 polypeptide.

As used herein, the term "chimeric antibody" will be held to mean any antibody wherein the immunoreactive region or site is obtained or derived from a first species and the constant region (which may be intact, partial or modified in accordance with the instant invention) is obtained from a second species. In certain embodiments the target binding region or site will be from a non-human source (*e.g.* mouse or primate) and the constant region is human.

As used herein, the term "engineered antibody" refers to an antibody in which the variable domain in either the heavy and light chain or both is altered by at least partial replacement of one or more CDRs from an antibody of known specificity and, if necessary, by partial framework region replacement and sequence changing. Although the CDRs may be derived from an antibody of the same class or even subclass as the antibody from which the framework regions are derived, it is envisaged that the CDRs will be derived from an antibody of different class and/or an antibody from a different species. An engineered antibody in which one or more "donor" CDRs from a non-human antibody of known specificity is grafted into a human heavy or light chain framework region is referred to herein as a "humanized antibody." It may not be necessary to replace all of the CDRs with the complete CDRs from the donor variable region to transfer the antigen binding capacity of one variable domain to another. Rather, it may only be necessary to transfer those residues that are necessary to maintain the activity of the target binding site. Given the explanations set forth in, *e.g.,* U. S. Pat. Nos. 5,585,089, 5,693,761, 5,693,762, and 6,180,370, it will be well within the competence of those skilled in the art, either by carrying out routine experimentation or by trial and error testing to obtain a functional engineered or humanized antibody.

As used herein, the terms "linked," "fused" or "fusion" are used interchangeably. These terms refer to the joining together of two more elements or components, by whatever means including chemical conjugation or recombinant means. An "in-frame fusion" refers to the joining of two or more open reading frames (ORFs) to form a continuous longer ORF, in a manner that maintains the correct reading frame of the original ORFs. Thus, the resulting recombinant fusion protein is a single protein containing two ore more segments that correspond to polypeptides encoded by the original ORFs (which segments are not normally so joined in nature.) Although the reading frame is thus made continuous throughout the fused segments, the segments may be physically or spatially separated by, for example, in-frame linker sequence.

In the context of polypeptides, a "linear sequence" or a "sequence" is an order of amino acids in a polypeptide in an amino to carboxyl terminal direction in which residues that neighbor each other in the sequence are contiguous in the primary structure of the polypeptide.

The term "expression" as used herein refers to a process by which a gene produces a biochemical, for example, an RNA or polypeptide. The process includes any manifestation of the functional presence of the gene within the cell including, without limitation, gene knockdown as well as both transient expression and stable expression. It includes without limitation transcription of the gene into messenger RNA (mRNA), transfer RNA (tRNA), small hairpin RNA (shRNA), small interfering RNA (siRNA) or any other RNA product and the translation of such mRNA into polypeptide(s). If the final desired product is biochemical, expression includes the creation of that biochemical and any precursors.

By "subject" or "individual" or "animal" or "patient" or "mammal," is meant any subject, particularly a mammalian subject, for whom diagnosis, prognosis, or therapy is desired. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; bears; and so on. In certain embodiments, the mammal is a human subject.

The term "RNA interference" or "RNAi" refers to the silencing or decreasing of gene expression by siRNAs. It is the process of sequence-specific, post-transcriptional gene silencing in animals and plants, initiated by siRNA that is homologous in its duplex region to the sequence of the silenced gene. The gene may be endogenous or exogenous to the organism, present integrated into a chromosome or present in a transfection vector that is not integrated into the genome. The expression of the gene is either completely or partially inhibited. RNAi may also be considered to inhibit the function of a target RNA; the function of the target RNA may be complete or partial.

### LINGO-4

The invention is based on the discovery that LINGO-4 is expressed in oligodendrocyte and negatively regulates oligodendrocyte differentiation and myelination.

Naturally occurring human LINGO-4 is a polypeptide consisting of about 593 amino acids. The polynucleotide encoding the human LINGO-4 mRNA is reported as accession number NM_001004432 in Genbank:

The polypeptide sequence of human LINGO-4 (encoded by nucleotides 191 to 1972 of SEQ ID NO: 1) is reported as accession number NP_001004432 in GenBank:

The polynucleotide encoding the mouse LINGO-4 mRNA is reported as accession number NM_177250 in Genbank:

The polypeptide sequence of mouse LINGO-4 (encoded by nucleotides 199 to 2055 of SEQ ID NO:3) is reported as accession number NP_796224 in GenBank:

Naturally occurring human LINGO-4 polypeptide (also known as DAAT9248, Leucine rich repeat neuronal 6D, LRRN6D, PRO34002, or Q6UY18) is an approximately 64 Kda protein of 593 amino acids (SEQ ID NO: 2). LINGO-4 is a member of the LINGO protein family, which contains at least three other human paralogs, LINGO-1, LINGO-2, and LINGO-3. *See* Mi et al., Nature Neurosci. 7: 221-28 (2004). The human LINGO-4 polypeptide contains a stretch of about twelve (12) leucine-rich repeats (including the N-terminal cap (LRRNT) and C-terminal cap (LRRCT)) (SEQ ID NO: 2). The number of predicted repeats may vary depending upon which protein computer modeling program is used. The LRR domains comprise about 380 amino acid residues of the LINGO-4 protein. LINGO-4 also contains an Ig domain comprising at least about 58 amino acids. There also is a transmembrane region, which is approximately 22 amino acids in length, and an intracellular domain of about 35 amino acids. In addition, the naturally occurring LINGO-4 protein contains a signal sequence at the N-terminus of the protein which is about 28 amino acids in length (FIG. 2). As the person of ordinary skill in the art will appreciate, the lengths of the various domains of LINGO-4 reported here are approximate, and are based on computer predictions, and different computer programs will provide different results. Table 1 lists predicted boundaries of the various LINGO-4 domains, based on the amino acid sequence of SEQ ID NO: 2.

**Table 1**

| **Domain or Region** | **Beginning Residue** | **Ending Residue** |
|---|---|---|
| Signal Sequence | 1 | 28 |
| LRRNT | 30 | 64 |
| LRR | 63 | 82 |
| LRR | 83 | 106 |
| LRR | 107 | 130 |
| LRR | 131 | 154 |
| LRR | 155 | 178 |
| LRR | 179 | 202 |
| LRR | 203 | 226 |
| LRR | 275 | 298 |
| LRR | 299 | 322 |
| LRR | 323 | 346 |
| LRRCT | 358 | 411 |
| Ig | 426 | 491 |
| Transmembrane | 535 | 557 |
| Intracellular | 558 | 593 |

Tissue distribution of LINGO-4 have been studied in humans and mice. Expression of adult mouse and P6 (post-natal day 6) LINGO-4 is localized to nervous-system neurons and brain oligodendrocytes, as determined by northern blot and PCR (*See* Figs. 1, 3, and 4).

### Treatment Methods Using Antagonists of LINGO-4

One instance of the present disclosure provides methods for treating a disease, disorder or injury associated with dysmyelination or demyelination, *e.g.,* multiple sclerosis, in an animal suffering from such disease, the method comprising, consisting essentially of, or consisting of administering to the animal an effective amount of a LINGO-4 antagonist. In certain instances the LINGO-4 antagonist is selected from the group consisting of a soluble LINGO-4 polypeptide, a LINGO-4 antibody, a LINGO-4 antagonist polynucleotide, and a LINGO-4 aptamer.

Additionally, the disclosure is directed to a method for promoting myelination of neurons in a mammal comprising, consisting essentially of, or consisting of administering a therapeutically effective amount of a LINGO-4 antagonist. In certain instances the LINGO-4 antagonist is selected from the group consisting of a soluble LINGO-4 polypeptide, a LINGO-4 antibody, a LINGO-4 antagonist polynucleotide, and a LINGO-4 aptamer.

An additional instance provides methods for treating a disease, disorder or injury associated with oligodendrocyte death or lack of differentiation, *e.g.,* multiple sclerosis, Pelizaeus Merzbacher disease or globoid cell leukodystrophy (Krabbe's disease), in an animal suffering from such disease, the method comprising, consisting essentially of, or consisting of administering to the animal an effective amount of a LINGO-4 antagonist. In certain instances the LINGO-4 antagonist is selected from the group consisting of a soluble LINGO-4 polypeptide, a LINGO-4 antibody, a LINGO-4 antagonist polynucleotide, and a LINGO-4 aptamer.

Another aspect of the disclosure includes a method for promoting proliferation, differentiation and survival of oligodendrocytes in a mammal comprising, consisting essentially of, or consisting of administering a therapeutically effective amount of a LINGO-4 antagonist In certain instances the LINGO-4 antagonist is selected from the group consisting of a soluble LINGO-4 polypeptide, a LINGO-4 antibody , a LINGO-4 antagonist polynucleotide, and a LINGO-4 aptamer.

Further instances include a method for promoting neurite outgrowth or survival of a CNS neuron in the mammal with therapeutically effective amount of a composition comprising, consisting essentially of, or consisting of administering a therapeutically amount of a LINGO-4 antagonist. In certain instance the LINGO-4 antagonist is selected from the group consisting of a soluble LINGO-4 polypeptide, a LINGO-4 antibody, a LINGO-4 antagonist polynucleotide, and a LINGO-4 aptamer.

Another aspect of the disclosure includes a method of treating a CNS disease, disorder or injury in a mammal comprising, consisting essentially of, or consisting of administering to the mammal a therapeutically effective amount of a composition comprising a LINGO-4 antagonist. In certain instances the LINGO-4 antagonist is selected from the group consisting of a soluble LINGO-4 polypeptide, a LINGO-4 antibody, a LINGO-4 antagonist polynucleotide, and a LINGO-4 aptamer.

A LINGO-4 antagonist, *e.g.,* a soluble LINGO-4 polypeptide, a LINGO-4 antibody, a LINGO-4 antagonist polynucleotide, or a LINGO-4 aptamer, to be used in treatment methods disclosed herein, can be prepared and used as a therapeutic agent that stops, reduces, prevents, or inhibits the ability of LINGO-4 to negatively regulate myelination of neurons by oligodendrocytes. Additionally, the LINGO-4 antagonist to be used in treatment methods disclosed herein can be prepared and used as a therapeutic agent that stops, reduces, prevents, or inhibits the ability of LINGO-4 to negatively regulate oligodendrocyte differentiation, proliferation and survival.

Further instances include a method of inducing oligodendrocyte proliferation or survival to treat a disease, disorder or injury involving the destruction of oligodendrocytes or myelin comprising administering to a mammal, at or near the site of the disease, disorder or injury, in an amount sufficient to reduce inhibition of axonal extension and promote myelination.

In methods of the present disclosure, a LINGO-4 antagonist can be administered via direct administration, *e.g.,* of a soluble LINGO-4 polypeptide, LINGO-4 antibody, LINGO-4 antagonist polynucleotide, or a LINGO-4 aptamer to the patient. Alternatively, the LINGO-4 antagonist can be administered via an expression vector which produces the specific LINGO-4 antagonist. In certain instances, a LINGO-4 antagonist is administered in a treatment method that includes: (1) transforming or transfecting an implantable host cell with a nucleic acid, *e.g.,* a vector, that expresses a LINGO-4 antagonist; and (2) implanting the transformed host cell into a mammal, at the site of a disease, disorder or injury. For example, the transformed host cell can be implanted at the site of a chronic lesion of MS. In some instances, the implantable host cell is removed from a mammal, temporarily cultured, transformed or transfected with an isolated nucleic acid encoding a LINGO-4 antagonist, and implanted back into the same mammal from which it was removed. The cell can be, but is not required to be, removed from the same site at which it is implanted. Such instances, sometimes known as *ex vivo* gene therapy, can provide a continuous supply of the LINGO-4 antagonist, localized at the site of action, for a limited period of time.

Diseases or disorders which may be treated or ameliorated by the methods of the present disclosure include diseases, disorders or injuries which relate to dysmyelination or demyelination of mammalian neurons. Specifically, diseases and disorders in which the myelin which surrounds the neuron is either absent, incomplete, not formed properly or is deteriorating. Such diseases include, but are not limited to, multiple sclerosis (MS) including relapsing remitting, secondary progressive and primary progressive forms of MS; progressive multifocal leukoencephalopathy (PML), encephalomyelitis (EPL), central pontine myelolysis (CPM), adrenoleukodystrophy, Alexander's disease, Pelizaeus Merzbacher disease (PMZ), globoid cell leukodystrophy (Krabbe's disease), Wallerian Degeneration, optic neuritis and transvere myelitis.

Diseases or disorders which may be treated or ameliorated by the methods of the present disclosure include diseases, disorders or injuries which relate to the death or lack of proliferation or differentiation ofoligodendrocytes. Such diseases include, but are not limited to, multiple sclerosis (MS), progressive multifocal leukoencephalopathy (PML), encephalomyelitis (EPL), central pontine myelolysis (CPM), adrenoleukodystrophy, Alexander's disease, Pelizaeus Merzbacher disease (PMZ), globoid cell leukodystrophy (Krabbe's disease) and Wallerian Degeneration.

Diseases or disorders which may be treated or ameliorated by the methods of the present disclosure include neurodegenerate disease or disorders. Such diseases include, but are not limited to, amyotrophic lateral sclerosis (ALS), Huntington's disease, Alzheimer's disease and Parkinson's disease.

Examples of additional diseases, disorders or injuries which may be treated or ameliorated by the methods of the present disclosure include, but are not limited, to spinal cord injuries, chronic myelopathy or rediculopathy, traumatic brain injury, motor neuron disease, axonal shearing, contusions, paralysis, post radiation damage or other neurological complications of chemotherapy, stroke, large lacunes, medium to large vessel occlusions, leukoariaosis, acute ischemic optic neuropathy, vitamin E deficiency (isolated deficiency syndrome, AR, Bassen-Kornzweig syndrome), B12, B6 (pyridoxine - pellagra), thiamine, folate, nicotinic acid deficiency, Marchiafava-Bignami syndrome, Metachromatic Leukodystrophy, Trigeminal neuralgia, Bell's palsy, or any neural injury which would require axonal regeneration, remylination or oligodendrocyte survival or differentiation/proliferation.

### Soluble LINGO-4 Polypeptides

Soluble LINGO-4 polypeptides for use in the present invention include fragments, variants, or derivatives thereof of a soluble LINGO-4 polypeptide. Table 1 above describes the various domains of a human LINGO-4 polypeptide. Similar domain structures can be deduced for LINGO-4 polypeptides of other species, *e.g.,* mouse LINGO-4 (SEQ ID NO:4). Soluble LINGO-4 polypetides typically lack the transmembrane domain of the LINGO-4 polypeptide, and optionally lack the cytoplasmic domain of the LINGO-4 polypeptide. For example, certain soluble human LINGO-4 polypeptides lack amino acids 535-557 of SEQ ID NO:2, which comprise the transmembrane domain of human LINGO-4. Additionally, certain soluble LINGO-4 polypeptides comprise the LRR domains and the Ig domain of the LINGO-4 polypeptide.

A variant LINGO-4 polypeptide can also vary in sequence from the corresponding wild-type polypeptide. In particular, certain amino acid substitutions can be introduced into the LINGO-4 sequence without appreciable loss of a LINGO-4 biological activity. In exemplary embodiments, a variant LINGO-4 polypeptide contains one or more amino acid substitutions, and/or comprises an amino acid sequence which is at least 70%, 80%, 85%, 90%, 95%, 98% or 99% identical to a reference amino acid sequence selected from the group consisting of: amino acids 30 to 411 of SEQ ID NO:2, amino acids 30 to 491 of SEQ ID NO:2, and amino acids 30 to 534 of SEQ ID NO:2, or equivalent fragments of SEQ ID NO:4. A variant LINGO-4 polypeptide differing in sequence from any given fragment of SEQ ID NO:2 or SEQ ID NO:4 may include one or more amino acid substitutions (conservative or non-conservative), one or more deletions, and/or one or more insertions. In certain embodiments, the soluble LINGO-4 polypeptide promotes proliferation, differentiation, or survival of oligodendrocytes; promotes, oligodendrocyte-mediated myelination of neurons, or prevents demyelination, e.g., in a mammal.

A soluble LINGO-4 polypeptide can comprise a fragment of at least six, *e.g.,* ten, fifteen, twenty, twenty-five, thirty, forty, fifty, sixty, seventy, one hundred, or more amino acids of SEQ ID NO:2 or SEQ ID NO:4. In addition, a soluble LINGO-4 polypeptide may comprise at least one, *e.g.,* five, ten, fifteen or twenty conservative amino acid substitutions. Corresponding fragments of soluble LINGO-4 polypeptides at least 70%, 75%, 80%, 85%, 90%, or 95% identical to a reference LINGO-4 polypeptide of SEQ ID NO:2 or SEQ ID NO:4 are also contemplated. In certain embodiments, the soluble LINGO-4 polypeptide promotes proliferation, differentiation, or survival of oligodendrocytes; promotes, oligodendrocyte-mediated myelination of neurons, or prevents demyelination, e.g., in a mammal.

By "a LINGO-4 reference amino acid sequence," or "reference amino acid sequence" is meant the specified sequence without the introduction of any amino acid substitutions. As one of ordinary skill in the art would understand, if there are no substitutions, the "isolated polypeptide" of the invention comprises an amino acid sequence which is identical to the reference amino acid sequence.

Conservative substitutions include substitutions within the following groups: valine, alanine and glycine; leucine, valine, and isoleucine; aspartic acid and glutamic acid; asparagine and glutamine; serine, cysteine, and threonine; lysine and arginine; and phenylalanine and tyrosine. The non-polar hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Any substitution of one member of the above-mentioned polar, basic or acidic groups by another member of the same group can be deemed a conservative substitution.

Non-conservative substitutions include those in which (i) a residue having an electropositive side chain (*e.g.,* Arg, His or Lys) is substituted for, or by, an electronegative residue (*e.g.,* Glu or Asp), (ii) a hydrophilic residue (*e.g.,* Ser or Thr) is substituted for, or by, a hydrophobic residue (*e.g.,* Ala, Leu, Ile, Phe or Val), (iii) a cysteine or proline is substituted for, or by, any other residue, or (iv) a residue having a bulky hydrophobic or aromatic side chain (*e.g.,* Val, Ile, Phe or Trp) is substituted for, or by, one having a smaller side chain *(e.g.,* Ala, Ser) or no side chain (*e.g*., Gly).

As known in the art, "sequence identity" between two polypeptides is determined by comparing the amino acid sequence of one polypeptide to the sequence of a second polypeptide. When discussed herein, whether any particular polypeptide is at least about 70%, 75%, 80%, 85%, 90% or 95% identical to another polypeptide can be determined using methods and computer programs/software known in the art such as, but not limited to, the BESTFIT program (Wisconsin Sequence Analysis Package, Version 8 for Unix, Genetics Computer Group, University Research Park, 575 Science Drive, Madison, WI 53711). BESTFIT uses the local homology algorithm of Smith and Waterman, Advances in Applied Mathematics 2:482-489 (1981), to find the best segment of homology between two sequences. When using BESTFIT or any other sequence alignment program to determine whether a particular sequence is, for example, 95% identical to a reference sequence according to the present invention, the parameters are set, of course, such that the percentage of identity is calculated over the full length of the reference polypeptide sequence and that gaps in homology of up to 5% of the total number of amino acids in the reference sequence are allowed.

Additional soluble LINGO-4 polypeptides for use in the present invention include, but are not limited to, a human LINGO-4 polypeptide fragment comprising, consisting essentially of, or consisting of amino acids 30 to 64 of SEQ ID NO:2; amino acids 30 to 82 of SEQ ID NO:2; amino acids 30 to 106 of SEQ ID NO:2; amino acids 30 to 130 of SEQ ID NO:2; amino acids 30 to 154 of SEQ ID NO:2; amino acids 30 to 178 of SEQ ID NO:2; amino acids 30 to 202 of SEQ ID NO:2; amino acids 30 to 226 of SEQ ID NO:2; amino acids 30 to 298 of SEQ ID NO:2; amino acids 30 to 322 of SEQ ID NO:2; amino acids 30 to 346 of SEQ ID NO:2; amino acids 30 to 411 of SEQ ID NO:2; amino acids 30 to 491 of SEQ ID NO:2; amino acids 30 to 534 of SEQ ID NO:2; amino acids amino acids 63 to 82 of SEQ ID NO:2; amino acids 63 to 106 of SEQ ID NO:2; amino acids 63 to 130 of SEQ ID NO:2; amino acids 63 to 154 of SEQ ID NO:2; amino acids 63 to 178 of SEQ ID NO:2; amino acids 63 to 202 of SEQ ID NO:2; amino acids 63 to 226 of SEQ ID NO:2; amino acids 63 to 298 of SEQ ID NO:2; amino acids 63 to 322 of SEQ ID NO:2; amino acids 63 to 346 of SEQ ID NO:2; amino acids 63 to 411 of SEQ ID NO:2; amino acids 63 to 491 of SEQ ID NO:2; amino acids 63 to 534 of SEQ ID NO:2; amino acids 83 to 106 of SEQ ID NO:2; amino acids 83 to 130 of SEQ ID NO:2; amino acids 83 to 154 of SEQ ED NO:2; amino acids 83 to 178 of SEQ ID NO:2; amino acids 83 to 202 of SEQ ID NO:2; amino acids 83 to 226 of SEQ ID NO:2; amino acids 83 to 298 of SEQ ID NO:2; amino acids 83 to 322 of SEQ ID NO:2; amino acids 83 to 346 of SEQ ID NO:2; amino acids 83 to 411 of SEQ ID NO:2; amino acids 83 to 491 of SEQ ID NO:2; amino acids 83 to 534 of SEQ ID NO:2; amino acids 107 to 130 of SEQ ID NO:2; amino acids 107 to 154 of SEQ ID NO:2; amino acids 107 to 178 of SEQ ID NO:2; amino acids 107 to 202 of SEQ ID NO:2; amino acids 107 to 226 of SEQ ID NO:2; amino acids 107 to 298 of SEQ ID NO:2; amino acids 107 to 322 of SEQ ID NO:2; amino acids 107 to 346 of SEQ ID NO:2; amino acids 107 to 411 of SEQ ID NO:2; amino acids 107 to 491 of SEQ ID NO:2; amino acids 107 to 534 of SEQ ID NO:2; amino acids 131 to 154 of SEQ ID NO:2; amino acids 131 to 178 of SEQ ID NO:2; amino acids 131 to 202 of SEQ ID NO:2; amino acids 131 to 226 of SEQ ID NO:2; amino acids 131 to 298 of SEQ ID NO:2; amino acids 131 to 322 of SEQ ID NO:2; amino acids 131 to 346 of SEQ ID NO:2; amino acids 131 to 411 of SEQ ID NO:2; amino acids 131 to 491 of SEQ ID NO:2; amino acids 131 to 534 of SEQ ID NO:2; amino acids 155 to 178 of SEQ ID NO:2; amino acids 155 to 202 of SEQ ID NO:2; amino acids 155 to 226 of SEQ ID NO:2; amino acids 155 to 298 of SEQ ID NO:2; amino acids 155 to 322 of SEQ ID NO:2; amino acids 155 to 346 of SEQ ID NO:2; amino acids 155 to 411 of SEQ ID NO:2; amino acids 155 to 491 of SEQ m NO:2; amino acids 155 to 534 of SEQ ID NO:2; amino acids 179 to 202 of SEQ ID NO:2; amino acids 179 to 226 of SEQ ID NO:2; amino acids 179 to 298 of SEQ ill NO:2; amino acids 179 to 322 of SEQ ID NO:2; amino acids 179 to 346 of SEQ ID NO:2; amino acids 179 to 411 of SEQ ID NO:2; amino acids 179 to 491 of SEQ ID NO:2; amino acids 179 to 534 of SEQ ID NO:2; amino acids 203 to 226 of SEQ ID NO:2; amino acids 203 to 298 of SEQ ID NO:2; amino acids 203 to 322 of SEQ ID NO:2; amino acids 203 to 346 of SEQ ID NO:2; amino acids 203 to 411 of SEQ ID NO:2; amino acids 203 to 491 of SEQ ID NO:2; amino acids 203 to 534 of SEQ ID NO:2; amino acids 275 to 298 of SEQ ID NO:2; amino acids 275 to 322 of SEQ ID NO:2; amino acids 275 to 346 of SEQ ID NO:2; amino acids 275 to 411 of SEQ ID NO:2; amino acids 275 to 491 of SEQ ID NO:2; amino acids 275 to 534 of SEQ ID NO:2; amino acids 299 to 322 of SEQ ID NO:2; amino acids 299 to 346 of SEQ ID NO:2; amino acids 299 to 411 of SEQ ID NO:2; amino acids 299 to 491 of SEQ ID NO:2; amino acids 299 to 534 of SEQ ID NO:2; amino acids 323 to 346 of SEQ ID NO:2; amino acids 323 to 411 of SEQ ID NO:2; amino acids 323 to 491 of SEQ ID NO:2; amino acids 323 to 534 of SEQ ID NO:2; amino acids 358 to 411 of SEQ ID NO:2; amino acids 358 to 491 of SEQ ID NO:2; amino acids 358 to 534 of SEQ ID NO:2; amino acids 426 to 491 of SEQ ill NO:2; amino acids 426 to 534 of SEQ ID NO:2; or fragments, variants, or derivatives of such polypeptides. In certain embodiments of the present invention, the soluble LINGO-4 polypeptide promotes proliferation, differentiation, or survival of oligodendrocytes; promotes, oligodendrocyte-mediated myelination of neurons, or prevents demyelination, e.g., in a mammal.

As would be well understood by a person of ordinary skill in the art, the LINGO-4 fragments such as those listed above may vary in length, for example, by 1, 2, 3, 4, 5, 6, 7, 8, 9, or 10 amino acids at either end (either longer or shorter) based, for example, on alternate predictions of the LINGO-4 domain regions. In addition, any of the fragments listed above may further include a secretory signal peptide at the N-terminus, *e*.*g*., amino acids 1 to 28 of SEQ ID NO:2 or amino acids 1 to 29 of SEQ ID NO:2. Other secretory signal peptides, such as those described elsewhere herein, may also be used. Corresponding fragments of soluble LINGO-4 polypeptides at least 70%, 75%, 80%, 85%, 90%, or 95% identical to SEQ ID NO:2, SEQ ID NO:4, or fragments thereof described herein are also contemplated.

Soluble LINGO-4 polypeptides for use in the present invention may include any combination of two or more soluble LINGO-4 polypeptides. Accordingly, soluble LINGO-4 polypeptide dimers, either homodimers or heterodimers, are contemplated. Two or more soluble LINGO-4 polypeptides as described herein may be directly connected, or may be connected via a suitable peptide linker. Such peptide linkers are described elsewhere herein.

Soluble LINGO-4 polypeptides for use in the present invention may be cyclic. Cyclization of the soluble LINGO-4 polypetides reduces the conformational freedom of linear peptides and results in a more structurally constrained molecule. Many methods of peptide cyclization are known in the art. For example, "backbone to backbone" cyclization by the formation of an amide bond between the N-terminal and the C-terminal amino acid residues of the peptide. The "backbone to backbone" cyclization method includes the formation of disulfide bridges between two ω-thio amino acid residues (*e*.*g*, cysteine, homocysteine). Certain soluble LINGO-4 peptides of the present invention include modifications on the N- and C-terminus of the peptide to form a cyclic LINGO-4 polypeptide. Such modifications include, but are not limited, to cysteine residues, acetylated cysteine residues cystein residues with a NH₂ moiety and biotin. Other methods of peptide cyclization are described in Li & Roller. Curr. Top. Med. Chem. 3:325-341 (2002).

Cyclic LINGO-4 polypeptides for use in the present invention include, but are not limited to, C₁LSPX₁X₂X₃C₂ (SEQ ID NO:41) where X₁ is lysine, arginine, histidine, glutamine, or asparagine, X₂ is lysine, arginine, histidine, glutamine, or asparagine, X₃ is lysine, arginine, histidine, glutamine, or asparagine, C₁ optionally has a moiety to promote cyclization (*e.g.* an acetyl group or biotin) attached and C₂ optionally has a moiety to promote cyclization (*e.g.* an NH₂ moiety) attached.

### Antibodies or Immunospecific Fragments Thereof

LINGO-4 antagonists for use in the present invention also include LINGO-4-specific antibodies or antigen-binding fragments, variants, or derivatives which are antagonists of LINGO-4 activity. For example, binding of certain LINGO-4 antibodies to LINGO-4, as expressed on oligodendrocytes, blocks inhibition of oligodendrocyte growth or differentiation, or blocks demyelination or dysmyelination of CNS neurons.

Certain antagonist antibodies for use in the methods described herein specifically or preferentially binds to a particular LINGO-4 polypeptide fragment or domain, for example, a LINGO-4 polypeptide, fragment, variant, or derivative as described herein. In certain embodiments of the present invention, the LINGO-4 antagonist antibody promotes proliferation, differentiation, or survival of oligodendrocytes; promotes, oligodendrocyte-mediated myelination of neurons, or prevents demyelination, e.g., in a mammal.

In other embodiments, the present disclosure includes an antibody, or antigen-binding fragment, variant, or derivative thereof which specifically or preferentially *binds* to at least one epitope of LINGO-4, where the epitope comprises, consists essentially of, or consists of at least about four to five amino acids of SEQ ID NO:2 or SEQ ID NO:4, at least seven, at least nine, or between at least about 15 to about 30 amino acids of SEQ ID NO:2 or SEQ ID NO:4. The amino acids of a given epitope of SEQ ID NO:2 or SEQ ID NO:4 as described may be, but need not be, contiguous or linear. In certain embodiments, the at least one epitope of LINGO-4 comprises, consists essentially of, or consists of a non-linear epitope formed by the extracellular domain of LINGO-4 as expressed on the surface of a cell or as a soluble fragment, e.g., fused to an IgG Fc region. Thus, in certain embodiments the at least one epitope of LINGO-4 comprises, consists essentially of, or consists of at least 4, at least 5, at least 6, at least 7, at least 8, at least 9, at least 10, at least 15, at least 20, at least 25, between about 15 to about 30, or at least 10, 15, 20,25, 30, 35, 40, 45, 50, 55, 60, 65, 70, 75, 80, 85, 90, 95, or 100 contiguous or non-contiguous amino acids of SEQ ID NO:2, or SEQ ID NO:4. where non-contiguous amino acids form an epitope through protein folding.

In other embodiments, the present disclosure includes an antibody, or antigen-binding fragment, variant, or derivative thereof which specifically or preferentially binds to at least one epitope of LINGO-4, where the epitope comprises, *consists* essentially of, or consists of, in addition to one, two, three, four, five, six or more contiguous or non-contiguous amino acids of SEQ ID NO:2 or SEQ ID NO:4 as described above, and an additional moiety which modifies the protein, *e.g.,* a carbohydrate moiety may be included such that the LINGO-4 antibody binds with higher affinity to modified target protein than it does to an unmodified version of the protein. Alternatively, the LINGO-4 antibody does not bind the unmodified version of the target protein at all.

In *certain* embodiments, an antibody, or antigen-binding fragment, variant, or derivative thereof of the invention binds specifically to at least one epitope of LINGO-4 or fragment or variant described above, *i.e.,* binds to such an epitope more readily than it would bind to an unrelated, or random epitope; binds preferentially to at least one epitope of LINGO-4 or fragment or variant described above, *i.e.,* binds to such an epitope more readily than it would bind to a related, similar, homologous, or analogous epitope; competitively inhibits binding of a reference antibody which itself binds specifically or preferentially to a certain epitope of LINGO-4 or fragment or variant described above; or binds to at least one epitope of LINGO-4 or fragment or variant described above with an affinity characterized by a dissociation constant K_{D} of less than about 5 x 10⁻² M, about 10⁻² M, about 5 x 10⁻³ M, about 10⁻³ M, about 5 x 10⁻⁴ M, about 10⁻⁴ M, about 5 x 10⁻⁵ M, about 10⁻⁵ M, about 5 x 10⁻⁶ M, about 10⁻⁶ M, about 5 x 10⁻⁷ M, about 10⁻⁷ M, about 5 x 10⁻⁸ M, about 10⁻⁸ M, about 5 x 10⁻⁹ M, about 10⁻⁹ M, about 5 x 10⁻¹⁰ M, about 10⁻¹⁰ M, about 5 x 10⁻¹¹ M, about 10⁻¹¹ M, about 5 x 10⁻¹² M, about 10⁻¹² M, about 5 x 10⁻¹³ M, about 10⁻¹³ M, about 5 x 10⁻¹⁴ M, about 10⁻¹⁴ M, about 5 x 10⁻¹⁵ M, or about 10⁻¹⁵ M. In a particular aspect, the antibody or fragment thereof preferentially binds to a human LINGO-4 polypeptide or fragment thereof, relative to a murine LINGO-4 polypeptide or fragment thereof.

As used in the context of antibody binding dissociation constants, the term "about" allows for the degree of variation inherent in the methods utilized for measuring antibody affinity. For example, depending on the level of precision of the instrumentation used, standard error based on the number of samples measured, and rounding error, the term "about 10⁻² M" might include, for example, from 0.05 M to 0.005 M.

In specific embodiments, an antibody, or antigen-binding fragment, variant, or derivative thereof of the invention binds LINGO-4 polypeptides or fragments or *variants* thereof with an off rate (k(off)) of less than or equal to 5 X 10⁻² sec⁻¹, 10⁻² sec⁻¹, 5 X 10⁻³ sec⁻¹ or 10⁻³ sec⁻¹. Alternatively, an antibody, or antigen-binding fragment, variant, or derivative thereof of the disclosure binds LINGO-4 polypeptides or fragments or variants thereof with an off rate (k(off)) of less than or equal to 5 X 10⁻⁴ sec⁻¹, 10⁻⁴ sec⁻¹, 5 X 10⁻⁵ sec⁻¹, or 10⁻⁵ sec⁻¹ 5 X 10⁻⁶ sec⁻¹, 10⁻⁶ sec⁻¹, 5 X 10⁻⁷ sec⁻¹ or 10⁻⁷ sec⁻¹.

In other embodiments, an antibody, or antigen-binding fragment, variant, or derivative thereof of the invention binds LINGO-4 polypeptides or fragments or variants thereof with an on rate (k(on)) of greater than or equal to 10³ M⁻¹ sec⁻¹, 5 X 10³ M⁻¹ sec⁻¹, 10⁴ M⁻¹ sec⁻¹ or 5 X 10⁴ M⁻¹ sec⁻¹. Alternatively, an *antibody*, or antigen-binding fragment, variant, or derivative thereof of the invention binds LINGO-4 polypeptides or fragments or variants thereof with an on rate (k(on)) greater than or equal to 10⁵ M⁻¹ sec⁻¹, 5 X 10⁵ M⁻¹ sec⁻¹, 10⁶ M⁻¹ sec⁻¹, or 5 X 10⁶ M⁻¹ sec⁻¹ or 10⁷ M⁻¹ sec⁻¹.

In one embodiment, a LINGO-4 antagonist for use in the invention is an antibody molecule, or immunospecific fragment thereof. Unless it is specifically noted, as used herein a "fragment thereof" in reference to an antibody *refers* to an immunospecific fragment, *i.e.,* an antigen-specific fragment. In one embodiment, an antibody of the invention is a bispecific binding molecule, binding polypeptide, or antibody, *e.g.,* a bispecific antibody, minibody, domain deleted antibody, or fusion protein having binding specificity for more than one epitope, *e.g.,* more than one antigen or more than one epitope on the same antigen. In one embodiment, a bispecific antibody has at least one binding domain specific for at least one epitope on LINGO-4. A bispecific antibody may be a tetravalent antibody that has two target binding domains specific for an epitope of LINGO-4 and two target binding domains specific for a second target. Thus, a tetravalent bispecific antibody may be bivalent for each specificity.

Certain instances comprise administration of a LINGO-4 antagonist antibody, or immunospecific fragment thereof, in which at least a fraction of one or more of the constant region domains has been deleted or otherwise altered so as to provide desired biochemical characteristics such as reduced effector functions, the ability to non-covalently dimerize, increased ability to localize at the site of a tumor, reduced serum half-life, or increased serum half-life when compared with a whole, unaltered antibody of approximately the same immunogenicity. For example, certain antibodies for use in the treatment methods described herein are domain deleted antibodies which comprise a polypeptide chain similar to an immunoglobulin heavy chain, but which lack at least a portion of one or more heavy chain domains. For instance, in certain antibodies, one entire domain of the constant region of the modified antibody will be deleted, for example, all or part of the C_{H}2 domain will be deleted.

In certain LINGO-4 antagonist antibodies or immunospecific fragments thereof *for* use in the therapeutic methods described herein, the Fc portion may be mutated to decrease effector function using techniques known in the art. For example, modifications of the constant region may be used to modify disulfide linkages or oligosaccharide moieties that allow for enhanced localization due to increased antigen specificity or antibody flexibility. The resulting physiological profile, bioavailability and other biochemical effects of the modifications may easily be measured and quantified using well known immunological techniques without undue experimentation.

Modified forms of antibodies or immunospecific fragments thereof for use in the *diagnostic* and therapeutic methods disclosed herein can be made from whole precursor or parent antibodies using techniques known in the art. Exemplary techniques are discussed in more detail herein.

LINGO-4 antagonist antibodies or immunospecific fragments thereof for use in the diagnostic and treatment methods disclosed herein can be made or manufactured using techniques that are known in the art. In certain embodiments, *antibody* molecules or fragments thereof are "recombinantly produced," *i.e.,* are produced using recombinant DNA technology. Exemplary techniques for making antibody molecules or fragments thereof are discussed in more detail elsewhere herein.

LINGO-4 *antagonist* antibodies or fragments thereof for use in the present invention may be generated by any suitable method known in the art.

Polyclonal antibodies can be produced by various procedures well known in the art. For example, a LINGO-4 immunospecific fragment can be administered to *various* host animals including, but not limited to, rabbits, mice, rats, etc. to induce the production of sera containing polyclonal antibodies specific for the antigen. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and *Corynebacterium parvum.* Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, Cold Spring Harbor Laboratory Press, 2nd ed. (1988); Hammerling et al., in: Monoclonal Antibodies and T-Cell Hybridomas Elsevier, N.Y., 563-681 (1981). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Thus, the term "monoclonal antibody" is not limited to antibodies produced through hybridoma technology. Monoclonal antibodies can be prepared using a wide variety of techniques known in the art including the use of hybridoma and recombinant and phage display technology.

Using art recognized protocols, in one example, antibodies are raised in mammals by multiple subcutaneous or intraperitoneal injections of the relevant antigen (*e.g*., purified LINGO-4 antigens or cells or cellular extracts comprising such antigens) and an adjuvant. This immunization typically elicits an immune *response* that comprises production of antigen-reactive antibodies from activated splenocytes or lymphocytes. While the resulting antibodies may be harvested from the serum of the animal to provide polyclonal preparations, it is often desirable to isolate individual lymphocytes from the spleen, lymph nodes or peripheral blood to provide homogenous preparations of monoclonal antibodies (MAbs). In certain specific embodiments, the lymphocytes are obtained from the spleen.

In this well known process (Kohler et al., Nature 256:495 (1975)) the relatively short-lived, or mortal, lymphocytes from a mammal which has been injected with antigen are fused with an immortal tumor cell line (*e.g.* a myeloma cell line), thus, producing hybrid cells or "hybridomas" which are both immortal and capable of producing the genetically coded antibody of the B cell. The resulting hybrids are segregated into single genetic strains by selection, dilution, and regrowth with each individual strain comprising specific genes for the formation of a single antibody. They produce antibodies which are homogeneous against a desired antigen and, in reference to their pure genetic parentage, are termed "monoclonal."

Typically, hybridoma cells thus prepared are seeded and grown in a suitable culture medium that contains one or more substances that inhibit the growth or survival of the unfused, parental myeloma cells. Those skilled in the art will appreciate that reagents, cell lines and media for the formation, selection and growth of hybridomas are commercially available from a number of sources and standardized protocols are well established. Generally, culture medium in which the hybridoma cells are growing is assayed for production of monoclonal antibodies against the desired antigen. In certain embodiments, the binding specificity of the monoclonal antibodies produced by hybridoma cells is determined by *in vitro* assays such as immunoprecipitation, radioimmunoassay (RIA) or enzyme-linked immunoabsorbent assay (ELISA). After hybridoma cells are identified that produce antibodies of the desired specificity, affinity and/or activity, the clones may be subcloned by limiting dilution procedures and grown by standard methods (Goding, Monoclonal Antibodies: Principles and Practice, Academic Press, pp 59-103 (1986)). It will further be appreciated that the monoclonal antibodies secreted by the subclones may be separated from culture medium, ascites fluid or serum by conventional purification procedures such as, for example, protein-A, hydroxylapatite chromatography, gel electrophoresis, dialysis or affinity chromatography.

Antibody fragments that recognize specific epitopes may be generated by known techniques. For example, Fab and F(ab')2 fragments may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the C_{H}1 domain of the heavy chain.

Those skilled in the art will also appreciate that DNA encoding antibodies or antibody fragments (*e.g*., antigen binding sites) may also be derived from antibody phage libraries. In a particular, such phage can be utilized to display antigen-binding domains expressed from a repertoire or combinatorial antibody library (*e.g*., human or murine). Phage expressing an antigen binding domain that binds the antigen of interest can be selected or identified with antigen, *e.g*., using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Exemplary methods are set forth, for example, in EP 368 684 B1; U.S. patent. 5,969,108, Hoogenboom, H.R. and Chames, Immunol. Today 21:371 (2000); Nagy et al. Nat. Med. 8:801 (2002); Huie et al., Proc. Natl. Acad. Sci. USA 98:2682 (2001); Lui et al., .l. Mol. Biol. 315:1063 (2002). Several publications (*e.g.,* Marks et al., Bio/Technology 10:779-783 (1992)) have described the production of high affinity human antibodies by chain shuffling, as well as combinatorial infection and *in vivo* recombination as a strategy for constructing large phage libraries. In another embodiment, Ribosomal display can be used to replace bacteriophage as the display platform (*see, e.g.,* Hanes et al., Nat. Biotechnol. 18:1287 (2000); Wilson et al., Proc. Natl. Acad. Sci. USA 98:3750 (2001); or Irving et al., J. Immunol. Methods 248:31 (2001)). In yet another embodiment, cell surface libraries can be screened for antibodies (Boder et al., Proc. Natl. Acad. Sci. USA 97:10701 (2000); Daugherty et al., J. Immunol. Methods 243:211 (2000)). Such procedures provide alternatives to traditional hybridoma techniques for the isolation and subsequent cloning of monoclonal antibodies.

In phage display methods, functional antibody domains are displayed on the surface of phage particles which carry the polynucleotide sequences encoding them. In particular, DNA sequences encoding V_{H} and V_{L} regions are amplified from animal cDNA libraries (*e.g*., human or murine cDNA libraries of lymphoid tissues) or synthetic cDNA libraries. In certain embodiments, the DNA encoding the V_{H} and V_{L} regions are joined together by an scFv linker by PCR and cloned into a phagemid vector (*e.g*., p CANTAB 6 or pComb 3 HSS). The vector is electroporated in *E. coli* and the *E. coli* is infected with *helper* phage. Phage used in these methods are typically filamentous phage including fd and M13 and the V_{H} or V_{L} regions are usually recombinantly fused to either the phage gene III or gene VIII. Phage expressing an antigen binding domain that binds to an antigen of interest (*i.e.,* a LINGO-4 polypeptide or a fragment thereof) can be selected or identified with antigen, e.g., using labeled antigen or antigen bound or captured to a solid surface or bead.

Additional examples of phage display methods that can be used to make the antibodies include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187:9-18 (1997); Burton et al., Advances in Immunology 57:191-280 (1994); PCT Application No. PCT/GB91/01134; PCT publications WO 90/02809; WO 91/10737; WO 92/01047; WO 92/18619; WO 93/11236; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria. For example, techniques to recombinantly produce Fab, Fab' and F(ab')2 fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988).

In another embodiment, DNA encoding desired monoclonal antibodies may be readily isolated and sequenced using conventional procedures (*e.g.,* by using oligonucleotide probes that are capable of binding specifically to genes encoding the heavy and light chains of murine antibodies). In certain embodiments, isolated and subcloned hybridoma cells serve as a source of such DNA. Once isolated, the DNA may be placed into expression vectors, which are then transfected into prokaryotic or eukaryotic host cells such as *E. coli* cells, simian COS cells, Chinese Hamster Ovary (CHO) cells or myeloma cells that do not otherwise produce immunoglobulins. More particularly, the isolated DNA (which may be synthetic as described herein) may be used to clone constant and variable region sequences for the manufacture antibodies as described in Newman et al., U.S. Pat. No. 5,658,570, filed January 25, 1995. Essentially, this entails extraction of RNA from the selected cells, conversion to cDNA, and amplification by PCR using Ig specific primers. Suitable primers for this purpose are also described in U.S. Pat. No. 5,658,570. As will be discussed in more detail below, transformed cells expressing the desired antibody may be grown up in relatively large quantities to provide clinical and commercial supplies of the immunoglobulin.

In a specific embodiments, the amino acid sequence of the heavy and/or light chain variable domains may be inspected to identify the sequences of the complementarity determining regions (CDRs) by methods that are well known in the art, *e.g.,* by comparison to known amino acid sequences of other heavy and light chain variable regions to determine the regions of sequence hypervariability. Using routine recombinant DNA techniques, one or more of the CDRs may be inserted within framework regions, *e.g.,* into human framework regions to humanize a non-human antibody. The framework regions may be naturally occurring or consensus framework regions, *e.g.,* human framework regions (see, *e.g.,* Chothia et al., J. Mol. Biol. 278:457-479 (1998) for a listing of human framework regions). In certain embodiments, the polynucleotide generated by the combination of the framework regions and CDRs encodes an antibody that specifically binds to at least one epitope of a desired polypeptide, *e.g.,* LINGO-4. In further embodiments, one or more amino acid substitutions may be made within the framework regions, for example, to improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present invention and within the skill of the art.

In certain embodiments, a LINGO-4 antagonist antibody or immunospecific fragment thereof for use in the treatment methods disclosed herein will not elicit a deleterious immune response in the animal to be treated, *e.g.,* in a human. In one embodiment, LINGO-4 antagonist antibodies or immunospecific fragments thereof for use in the treatment methods disclosed herein can be modified to reduce their immunogenicity using art-recognized techniques. For example, antibodies can be humanized, primatized, deimmunized, or chimeric antibodies can be made. These types of antibodies are derived from a non-human antibody, typically a murine or primate antibody, that retains or substantially retains the antigen-binding properties of the parent antibody, but which is less immunogenic in humans. This may be achieved by various methods, including (a) grafting the entire non-human variable domains onto human constant regions to generate chimeric antibodies; (b) grafting at least a part of one or more of the non-human complementarity determining regions (CDRs) into a human framework and constant regions with or without retention of critical framework residues; or (c) transplanting the entire non-human variable domains, but "cloaking" them with a human-like section by replacement of surface residues. Such methods are disclosed in Morrison et al., Proc. Natl. Acad. Sci. 81:6851-6855 (1984); Morrison et al., Adv. Immunol. 44:65-92 (1988); Verhoeyen et al., Science 239:1534-1536 (1988); Padlan, Molec. Immun. 28:489-498 (1991); Padlan, Molec. Immun. 31:169-217 (1994), and U.S. Pat. Nos. 5,585,089, 5,693,761, 5,693,762, and 6,190,370.

De-immunization can also be used to decrease the immunogenicity of an antibody. As used herein, the term "de-immunization" includes alteration of an antibody to modify T cell epitopes (see, *e.g.,* WO9852976A1, WO0034317A2). For example, V_{H} and V_{L} sequences from the starting antibody are analyzed and a human T cell epitope "map" from each V region showing the location of epitopes in relation to complementarity-determining regions (CDRs) and other key residues within the sequence. Individual T cell epitopes from the T cell epitope map are analyzed in order to identify alternative amino acid substitutions with a low risk of altering activity of the final antibody. A range of alternative V_{H} and V_{L} sequences are designed comprising combinations of amino acid substitutions and these sequences are subsequently incorporated into a range of binding polypeptides, *e.g.,* LINGO-4 antagonist antibodies or immunospecific fragments thereof for use in the diagnostic and treatment methods disclosed herein, which are then tested for function. Typically, between 12 and 24 variant antibodies are generated and tested. Complete heavy and light chain genes comprising modified V and human C regions are then cloned into expression vectors and the subsequent plasmids introduced into cell lines for the production of whole antibody. The antibodies are then compared in appropriate biochemical and biological assays, and the optimal variant is identified.

A chimeric antibody is a molecule in which different portions of the antibody are derived from different animal species, such as antibodies having a variable region derived from a murine monoclonal antibody and a human immunoglobulin constant region. Methods for producing chimeric antibodies are known in the art. *See, e.g.,* Morrison, Science 229:1202 (1985); Oi et al., BioTechniques 4:214 (1986); Gillies et al., J. Immunol. Methods 125:191-202 (1989); Takeda et al., Nature 314:452-454 (1985), Neuberger et al., Nature 312:604-608 (1984); U.S. Pat. Nos. 5,807,715; 4,816,567; and 4,816397. Humanized antibodies are antibody molecules from non-human species antibody that binds the desired antigen having one or more complementarity determining regions (CDRs) from the non-human species and framework regions from a human immunoglobulin molecule. Often, framework residues in the human framework regions will be substituted with the corresponding residue from the CDR donor antibody to alter, e.g., improve, antigen binding. These framework substitutions are identified by methods well known in the art, *e.g.,* by modeling of the interactions of the CDR and framework residues to identify framework residues important for antigen binding and sequence comparison to identify unusual framework residues at particular positions. (See, *e.g.,* Queen et al., U.S. Pat. No. 5,585,089; Riechmann et al., Nature 332:323 (1988). Antibodies can be humanized using a variety of techniques known in the art including, for example, CDR-grafting (EP 239,400; PCT publication WO 91/09967; U.S. Pat. Nos. 5,225,539; 5,530,101; and 5,585,089), veneering or resurfacing (EP 592,106; EP 519,596; Padlan, Molecular Immunology 28(4/5):489-498 (1991); Studnicka et al., Protein Engineering 7(6):805-814 (1994); Roguska. et al., PNAS 91:969-973 (1994)), and chain shuffling (U.S. Pat. No. 5,565,332).

Yet another highly efficient means for generating recombinant antibodies is disclosed by Newman, Biotechnology 10: 1455-1460 (1992). Specifically, this technique results in the generation of primatized antibodies that contain monkey variable domains and human constant sequences. Moreover, this technique is also described in commonly assigned U.S. Pat. Nos. 5,658,570, 5,693,780 and 5,756,096.

Completely human antibodies are particularly desirable for therapeutic treatment of human patients. Human antibodies can be made by a variety of methods known in the art including phage display methods described above using antibody libraries derived from human immunoglobulin sequences. See also, U.S. Pat. Nos. 4,444,887 and 4,716,111; and PCT publications WO 98/46645, WO 98/50433, WO 98/24893, WO 98/16654, WO 96/34096, WO 96/33735, and WO 91/10741.

Human antibodies can also be produced using transgenic mice which are incapable of expressing functional endogenous immunoglobulins, but which can express human immunoglobulin genes that are incapable of endogenous immunoglobulin production (see *e.g.,* U.S. Pat. Nos. 6,075,181, 5,939,598, 5,591,669 and 5,589,369). For example, it has been described that the homozygous deletion of the antibody heavy-chain joining region in chimeric and germ-line mutant mice results in complete inhibition of endogenous antibody production. The human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring that express human antibodies. The transgenic mice are immunized in the normal fashion with a selected antigen, *e.g.,* all or a portion of a desired target polypeptide. Monoclonal antibodies directed against the antigen can be obtained from the immunized, transgenic mice using conventional hybridoma technology. The human immunoglobulin transgenes harbored by the transgenic mice rearrange during B-cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar Int. Rev. Immunol. 13:65-93 (1995). For a detailed discussion of this technology for producing human antibodies and human monoclonal antibodies and protocols for producing such antibodies, see, *e.g.,* PCT publications WO 98/24893; WO 96/34096; WO 96/33735; U.S. Pat. Nos. 5,413,923; 5,625,126; 5,633,425; 5,569,825; 5,661,016; 5,545,806; 5,814,318; and 5,939,598. In addition, companies such as Abgenix, Inc. (Freemont, Calif.) and GenPharm (San Jose, Calif.) can be engaged to provide human antibodies directed against a selected antigen using technology similar to that described above.

Another means of generating human antibodies using SCID mice is disclosed in U.S. Pat. No. 5,811,524. It will be appreciated that the genetic material associated with these human antibodies may also be isolated and manipulated as described herein.

Completely human antibodies which recognize a selected epitope can be generated using a technique referred to as "guided selection." In this approach a selected non-human monoclonal antibody, *e.g.,* a mouse antibody, is used to guide the selection of a completely human antibody recognizing the same epitope. (Jespers et al., Bio/Technology 12:899-903 (1988)). *See also,* U.S. Patent No. 5,565,332.

Alternatively, techniques described for the production of single chain antibodies (U.S. Pat. No. 4,694,778; Bird, Science 242:423-442 (1988); Huston et al., Proc. Natl. Acad. Sci. USA 85:5879-5883 (1988); and Ward et al., Nature 334:544-554 (1989)) can be used. Single chain antibodies are formed by linking the heavy and light chain fragments of the Fv region via an amino acid bridge, resulting in a single chain antibody. Techniques for the assembly of functional Fv fragments in *E. coli* may also be used (Skerra et al., Science 242:1038-1041 (1988)). Examples of techniques which can be used to produce single-chain Fvs and antibodies include those described in U.S. Pat. Nos. 4,946,778 and 5,258,498; Huston et al., Methods in Enzymology 203:46-88 (1991); and Shu et al., PNAS 90:7995-7999 (1993).

In another embodiment, lymphocytes can be selected by micromanipulation and the variable genes isolated. For example, peripheral blood mononuclear cells can be isolated from an immunized mammal and cultured for about 7 days *in vitro*. The cultures can be screened for specific IgGs that meet the screening criteria. Cells from positive wells can be isolated. Individual Ig-producing B cells can be isolated by FACS or by identifying them in a complement-mediated hemolytic plaque assay. Ig-producing B cells can be micromanipulated into a tube and the V_{H} and V_{L} genes can be amplified using, *e.g.,* RT-PCR. The V_{H} and V_{L} genes can be cloned into an antibody expression vector and transfected into cells (*e.g.,* eukaryotic or prokaryotic cells) for expression.

Alternatively, antibody-producing cell lines may be selected and cultured using techniques well known to the skilled artisan. Such techniques are described in a variety of laboratory manuals and primary publications. In this respect, techniques suitable for use in the invention as described below are described in Current Protocols in Immunology, Coligan et al., Eds., Green Publishing Associates and Wiley-Interscience, John Wiley and Sons, New York (1991).

Antibodies for use in the therapeutic methods disclosed herein can be produced by any method known in the art for the synthesis of antibodies, in particular, by chemical synthesis or by recombinant expression techniques as described herein.

It will further be appreciated that the scope of this invention further encompasses all alleles, variants and mutations of antigen binding DNA sequences.

In one embodiment, cDNAs that encode the light and the heavy chains of the antibody may be made, either simultaneously or separately, using reverse transcriptase and DNA polymerase in accordance with well known methods. PCR may be initiated by consensus constant region primers or by more specific primers based on the published heavy and light chain DNA and amino acid sequences. As discussed above, PCR also may be used to isolate DNA clones encoding the antibody light and heavy chains. In this case the libraries may be screened by consensus primers or larger homologous probes, such as mouse constant region probes.

DNA, typically plasmid DNA, may be isolated from the cells using techniques known in the art, restriction mapped and sequenced in accordance with standard, well known techniques set forth in detail, *e.g.,* in the foregoing references relating to recombinant DNA techniques. Of course, the DNA may be synthetic according to the present invention at any point during the isolation process or subsequent analysis.

Recombinant expression of an antibody, or fragment, derivative or analog thereof, *e.g.,* a heavy or light chain of an antibody which is a LINGO-4 antagonist, requires construction of an expression vector containing a polynucleotide that encodes the antibody. Once a polynucleotide encoding an antibody molecule or a heavy or light chain of an antibody, or portion thereof (*e.g.,* containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule of the invention, or a heavy or light chain thereof, or a heavy or light chain variable domain, operably linked to a promoter. Such vectors may include the nucleotide sequence encoding the constant region of the antibody molecule (see, *e.g.,* PCT Publication WO 86/05807; PCT Publication WO 89/01036; and U.S. Pat. No. 5,122,464) and the variable domain of the antibody may be cloned into such a vector for expression of the entire heavy or light chain.

The expression vector is transferred to a host cell by conventional techniques and the transfected cells are then cultured by conventional techniques to produce an antibody for use in the methods described herein. Thus, the disclosure includes host cells containing a polynucleotide encoding an antibody of the invention, or a heavy or light chain thereof, operably linked to a heterologous promoter. In certain embodiments for the expression of double-chained antibodies, vectors encoding both the heavy and light chains may be co-expressed in the host cell for expression of the entire immunoglobulin molecule, as detailed below.

A variety of host-expression vector systems may be utilized to express antibody molecules for use in the methods described elsewhere herein.

The host cell may be co-transfected with two expression vectors, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain is advantageously placed before the heavy chain to avoid an excess of toxic free heavy chain (Proudfoot, Nature 322:52 (1986); Kohler, Proc. Natl. Acad. Sci. USA 77:2197 (1980)). The coding sequences for the heavy and light chains may comprise cDNA or genomic DNA.

Once an antibody molecule has been recombinantly expressed, it may be purified by any method known in the art for purification of an immunoglobulin molecule, for example, by chromatography (*e.g.,* ion exchange, affinity, particularly by affinity for the specific antigen after Protein A, and sizing column chromatography), centrifugation, differential solubility, or by any other standard technique for the purification of proteins. Alternatively, a method for increasing the affinity of antibodies of the invention is disclosed in US 2002 0123057 A1.

In one embodiment, a binding molecule or antigen binding molecule for use in the invention comprises a synthetic constant region wherein one or more domains are partially or entirely deleted ("domain-deleted antibodies"). In certain embodiments compatible modified antibodies will comprise domain deleted constructs or variants wherein the entire C_{H}2 domain has been removed (ΔC_{H}2 constructs). For other embodiments a short connecting peptide may be substituted for the deleted domain to provide flexibility and freedom of movement for the variable region. Those skilled in the art will appreciate that such constructs may be desirable under certain circumstances due to the regulatory properties of the C_{H}2 domain on the catabolic rate of the antibody.

In certain embodiments, modified antibodies for use in the methods disclosed herein are minibodies. Minibodies can be made using methods described in the art (see, *e.g.,* see *e.g.,* US patent 5,837,821 or WO 94/09817A1).

In another embodiment, modified antibodies for use in the methods disclosed herein are C_{H}2 domain deleted antibodies which are known in the art. Domain deleted constructs can be derived using a vector (*e.g.,* from Biogen IDEC Incorporated) encoding an IgG₁ human constant domain (see, *e.g.,* WO 02/060955A2 and WO02/096948A2). This exemplary vector was engineered to delete the C_{H}2 domain and provide a synthetic vector expressing a domain deleted IgG₁ constant region.

In one embodiment, a LINGO-4 antagonist antibody or fragment thereof for use in the treatment methods disclosed herein comprises an immunoglobulin heavy chain having deletion or substitution of a few or even a single amino acid as long as it permits association between the monomeric subunits. For example, the mutation of a single amino acid in selected areas of the C_{H}2 domain may be enough to substantially reduce Fc binding and thereby increase tumor localization. Similarly, it may be desirable to simply delete that part of one or more constant region domains that control the effector function (*e.g.* complement binding) to be modulated. Such partial deletions of the constant regions may improve selected characteristics of the antibody (serum half-life) while leaving other desirable functions associated with the subject constant region domain intact. Moreover, as alluded to above, the constant regions of the disclosed antibodies may be synthetic through the mutation or substitution of one or more amino acids that enhances the profile of the resulting construct. In this respect it may be possible to disrupt the activity provided by a conserved binding site (*e.g.* Fc binding) while substantially maintaining the configuration and immunogenic profile of the modified antibody. Yet other embodiments comprise the addition of one or more amino acids to the constant region to enhance desirable characteristics such as effector function or provide for more cytotoxin or carbohydrate attachment. In such embodiments it may be desirable to insert or replicate specific sequences derived from selected constant region domains.

Also disclosed is the use of antibodies that comprise, consist essentially of, or consist of, variants (including derivatives) of antibody molecules (*e.g.,* the V_{H} regions and/or V_{L} regions) described herein, which antibodies or fragments thereof immunospecifically bind to a LINGO-4 polypeptide. Standard techniques known to those of skill in the art can be used to introduce mutations in the nucleotide sequence encoding a binding molecule, including, but not limited to, site-directed mutagenesis and PCR-mediated mutagenesis which result in amino acid substitutions. In various embodiments, the variants (including derivatives) encode less than 50 amino acid substitutions, less than 40 amino acid substitutions, less than 30 amino acid substitutions, less than 25 amino acid substitutions, less than 20 amino acid substitutions, less than 15 amino acid substitutions, less than 10 amino acid substitutions, less than 5 amino acid substitutions, less than 4 amino acid substitutions, less than 3 amino acid substitutions, or less than 2 amino acid substitutions relative to the reference V_{H} region, V_{H}CDR1, V_{H}CDR2, V_{H}CDR3, V_{L} region, V_{L}CDR1, V_{L}CDR2, or V_{L}CDR3. A "conservative amino acid substitution" is one in which the amino acid residue is replaced with an amino acid residue having a side chain with a similar charge. Families of amino acid residues having side chains with similar charges have been defined in the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g.,* alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity.

For example, it is possible to introduce mutations only in framework regions or only in CDR regions of an antibody molecule. Introduced mutations may be silent or neutral missense mutations, *i.e.,* have no, or little, effect on an antibody's ability to bind antigen. These types of mutations may be useful to optimize codon usage, or improve a hybridoma's antibody production. Alternatively, non-neutral missense mutations may alter an antibody's ability to bind antigen. The location of most silent and neutral missense mutations is likely to be in the framework regions, while the location of most non-neutral missense mutations is likely to be in CDR, though this is not an absolute requirement. One of skill in the art would be able to design and test mutant molecules with desired properties such as no alteration in antigen binding activity or alteration in binding activity (*e.g.,* improvements in antigen binding activity or change in antibody specificity). Following mutagenesis, the encoded protein may routinely be expressed and the functional and/or biological activity of the encoded protein can be determined using techniques described herein or by routinely modifying techniques known in the art.

### Fusion Polypeptides and Antibodies

LINGO-4 polypeptides and antibodies for use in the treatment methods disclosed herein may further be recombinantly fused to a heterologous polypeptide at the N- or C-terminus. For example, LINGO-4 antagonist polypeptides or antibodies may be recombinantly fused or conjugated to molecules useful as labels in detection assays and effector molecules such as heterologous polypeptides, drugs, radionuclides, or toxins. *See, e.g.,* PCT publications WO 92/08495; WO 91/14438; WO 89/12624; U.S. Patent No. 5,314,995; and EP 396,387.

LINGO-4 antagonist polypeptides and antibodies for use in the treatment methods disclosed herein can be composed of amino acids joined to each other by peptide bonds or modified peptide bonds, *i.e.,* peptide isosteres, and may contain amino acids other than the 20 gene-encoded amino acids.

The present disclosure provides for fusion proteins comprising, consisting essentially of, or consisting of a LINGO-4 antagonist polypeptide or antibody fusion that inhibits LINGO-4 function. In certain embodiments, the heterologous polypeptide to which the LINGO-4 antagonist polypeptide or antibody is fused is useful for function or is useful to target the LINGO-4 antagonist polypeptide or antibody. In certain embodiments of the invention a soluble LINGO-4 antagonist polypeptide, *e.g.,* a LINGO-4 polypeptide comprising the LRR domains, Ig domain, or the entire extracellular domain (corresponding to amino acids 34 to 532 of SEQ ID NO: 2), or any other LINGO-4 polypeptide fragment, variant or derivative described herein, is fused to a heterologous polypeptide moiety to form a LINGO-4 antagonist fusion polypeptide. LINGO-4 antagonist fusion proteins and antibodies can be used to accomplish various objectives, *e.g.,* increased serum half-life, improved bioavailability, *in vivo* targeting to a specific organ or tissue type, improved recombinant expression efficiency, improved host cell secretion, ease of purification, and higher avidity. Depending on the objective(s) to be achieved, the heterologous moiety can be inert or biologically active. Also, it can be chosen to be stably fused to the LINGO-4 antagonist polypeptide or antibody or to be cleavable, *in vitro* or *in vivo.* Heterologous moieties to accomplish these other objectives are known in the art.

As an alternative to expression of a LINGO-4 antagonist fusion polypeptide or antibody, a chosen heterologous moiety can be preformed and chemically conjugated to the LINGO-4 antagonist polypeptide or antibody. In most cases, a chosen heterologous moiety will function similarly, whether fused or conjugated to the LINGO-4 antagonist polypeptide or antibody. Therefore, in the following discussion of heterologous amino acid sequences, unless otherwise noted, it is to be understood that the heterologous sequence can be joined to the LINGO-4 antagonist polypeptide or antibody in the form of a fusion protein or as a chemical conjugate.

Pharmacologically active polypeptides such as LINGO-4 antagonist polypeptides or antibodies often exhibit rapid *in vivo* clearance, necessitating large doses to achieve therapeutically effective concentrations in the body. In addition, polypeptides smaller than about 60 kDa potentially undergo glomerular filtration, which sometimes leads to nephrotoxicity. Fusion or conjugation of relatively small polypeptides such as LINGO-4 antagonist polypeptides or antibodies can be employed to reduce or avoid the risk of such nephrotoxicity. Various heterologous amino acid sequences, *i.e.,* polypeptide moieties or "carriers," for increasing the *in vivo* stability, *i.e.,* serum half-life, of therapeutic polypeptides are known.

Due to its long half-life, wide *in vivo* distribution, and lack of enzymatic or immunological function, essentially full-length human serum albumin (HSA), or an HSA fragment, is commonly used as a heterologous moiety. Through application of methods and materials such as those taught in Yeh et al., Proc. Natl. Acad. Sci. USA 89:1904-08 (1992) and Syed et al., Blood 89:3243-52 (1997), HSA can be used to form a LINGO-4 antagonist fusion polypeptide or antibody or polypeptide/antibody conjugate that displays pharmacological activity by virtue of the LINGO-4 moiety while displaying significantly increased *in vivo* stability, *e.g.,* 10-fold to 100-fold higher. The C-terminus of the HSA can be fused to the N-terminus of the soluble LINGO-4 moiety. Since HSA is a naturally secreted protein, the HSA signal sequence can be exploited to obtain secretion of the soluble LINGO-4 fusion protein into the cell culture medium when the fusion protein is produced in a eukaryotic, *e.g.,* mammalian, expression system.

In certain embodiments, LINGO-4 antagonist polypeptides or antibodies for use in the present invention further comprise a targeting moiety. Targeting moieties include a protein or a peptide which directs localization to a certain part of the body, for example, to the brain or compartments therein. In certain embodiments, LINGO-4 antagonist polypeptides or antibody for use in the present invention are attached or fused to a brain targeting moiety. The brain targeting moieties are attached covalently (*e.g.,* direct, translational fusion, or by chemical linkage either directly or through a spacer molecule, which can be optionally cleavable) or non-covalently attached (*e.g.,* through reversible interactions such as avidin, biotin, protein A, IgG, etc.). In other embodiments, a LINGO-4 antagonist polypeptide or antibody for use in the present invention is attached to one more brain targeting moieties. In additional embodiments, the brain targeting moiety is attached to a plurality of LINGO-4 antagonist polypeptides or antibodies for use in the present invention.

A brain targeting moiety associated with a LINGO-4 antagonist polypeptide or antibody enhances brain delivery of such a LINGO-4 antagonist polypeptide or antibody. A number of polypeptides have been described which, when fused to a protein or therapeutic agent, delivers the protein or therapeutic agent through the blood brain barrier (BBB). Nonlimiting examples include the single domain antibody FC5 (Abulrob et al. (2005) J. Neurochem. 95, 1201-1214); mAB 83-14, a monoclonal antibody to the human insulin receptor (Pardridge et al. (1995) Pharmacol. Res. 12, 807-816); the B2, B6 and B8 peptides binding to the human transferrin receptor (hTfR) (Xia et al. (2000) J. Virol. 74, 11359-11366); the OX26 monoclonal antibody to the transferrin receptor (Pardridge et al. (1991) J. Pharmacol. Exp. Ther. 259, 66-70); and SEQ ID NOs: 1-18 of U.S. Patent No. 6,306,365.

Enhanced brain delivery of a LINGO-4 antagonist composition is determined by a number of means well established in the art. For example, administering to an animal a radioactively, enzymatically or fluorescently labeled LINGO-4 antagonist polypeptide or antibody linked to a brain targeting moiety; determining brain localization; and comparing localization with an equivalent radioactively labeled LINGO-4 antagonist polypeptide or antibody that is not associated with a brain targeting moiety. Other means of determining enhanced targeting are described in the above references.

The signal sequence is a polynucleotide that encodes an amino acid sequence that initiates transport of a protein across the membrane of the endoplasmic reticulum. Signal sequences useful for constructing an immunofusin include antibody light chain signal sequences, *e.g.,* antibody 14.18 (Gillies et al., J. Immunol. Meth. 125:191-202 (1989)), antibody heavy chain signal sequences, *e.g.,* the MOPC141 antibody heavy chain signal sequence (Sakano et al., Nature 286:5774 (1980)). Alternatively, other signal sequences can be used. *See, e.g.,* Watson, Nucl. Acids Res. 12:5145 (1984). The signal peptide is usually cleaved in the lumen of the endoplasmic reticulum by signal peptidases. This results in the secretion of an immunofusin protein containing the Fc region and the soluble LINGO-4 moiety.

In some embodiments, the DNA sequence may encode a proteolytic cleavage site between the secretion cassette and the soluble LINGO-4 moiety. Such a cleavage site may provide, *e.g.,* for the proteolytic cleavage of the encoded fusion protein, thus separating the Fc domain from the target protein. Useful proteolytic cleavage sites include amino acid sequences recognized by proteolytic enzymes such as trypsin, plasmin, thrombin, factor Xa, or enterokinase K.

The secretion cassette can be incorporated into a replicable expression vector. Useful vectors include linear nucleic acids, plasmids, phagemids, cosmids and the like. An exemplary expression vector is pdC, in which the transcription of the immunofusin DNA is placed under the control of the enhancer and promoter of the human cytomegalovirus. *See, e.g.,* Lo et al., Biochim. Biophys. Acta 1088:712 (1991); and Lo et al., Protein Engineering 11:495-500 (1998). An appropriate host cell can be transformed or transfected with a DNA that encodes a soluble LINGO-4 polypeptide and used for the expression and secretion of the soluble LINGO-4 polypeptide. Host cells that are typically used include immortal hybridoma cells, myeloma cells, 293 cells, Chinese hamster ovary (CHO) cells, Hela cells, and COS cells.

In one embodiment, a soluble LINGO-4 polypeptide is fused to a hinge and Fc region, *i.e*., the C-terminal portion of an Ig heavy chain constant region. Potential advantages of a LINGO-4-Fc fusion include solubility, *in vivo* stability, and multivalency, *e.g.,* dimerization. The Fc region used can be an IgA, IgD, or IgG Fc region (hinge- C_{H}2- C_{H}3). Alternatively, it can be an IgE or IgM Fc region (hinge- C_{H}2- C_{H}3-C_{H}4). An IgG Fc region is generally used, *e.g.,* an IgG₁ Fc region or IgG₄ Fc region. In one embodiment, a sequence beginning in the hinge region just upstream of the papain cleavage site which defines IgG Fc chemically (*i.e.* residue 216, taking the first residue of heavy chain constant region to be 114 according to the Kabat system), or analogous sites of other immunoglobulins is used in the fusion. The precise site at which the fusion is made is not critical; particular sites are well known and may be selected in order to optimize the biological activity, secretion, or binding characteristics of the molecule. Materials and methods for constructing and expressing DNA encoding Fc fusions are known in the art and can be applied to obtain soluble LINGO-4 fusions without undue experimentation. Some embodiments of the invention employ a LINGO-4 fusion protein such as those described in Capon et al., U.S. Patent Nos. 5,428,130 and 5,565,335.

In some embodiments, fully intact, wild-type Fc regions display effector functions that may be unnecessary and undesired in an Fc fusion protein used in the present invention. Therefore, certain binding sites may be deleted from the Fc region during the construction of the secretion cassette. For example, since coexpression with the light chain is unnecessary, the binding site for the heavy chain binding protein, Bip (Hendershot et al., Immunol. Today 8:111-14 (1987)), is deleted from the C_{H}2 domain of the Fc region of IgE, such that this site does not interfere with the efficient secretion of the immunofusin. Transmembrane domain sequences, such as those present in IgM, also are generally deleted.

In certain embodiments, the IgG₁ Fc region is used. Alternatively, the Fc region of the other subclasses of immunoglobulin gamma (gamma-2, gamma-3 and gamma-4) can be used in the secretion cassette. The IgG₁ Fc region of immunoglobulin gamma-1 includes at least part of the hinge region, the C_{H}2 region, and the C_{H}3 region. In some embodiments, the Fc region of immunoglobulin gamma-1 is a C_{H}2-deleted-Fc, which includes part of the hinge region and the C_{H}3 region, but not the C_{H}2 region. A C_{H}2-deleted-Fc has been described by Gillies et al., Hum. Antibod. Hybridomas 1:47 (1990). In some embodiments, the Fc region of one of IgA, IgD, IgE, or IgM, is used.

LINGO-4-Fc fusion proteins can be constructed in several different configurations. In one configuration the C-terminus of the soluble LINGO-4 moiety is fused directly to the N-terminus of the Fc hinge moiety. In a slightly different configuration, a short polypeptide, *e.g.,* 2-10 amino acids, is incorporated into the fusion between the N-terminus of the soluble LINGO-4 moiety and the C-terminus of the Fc moiety. Such a linker provides conformational flexibility, which may improve biological activity in some circumstances. If a sufficient portion of the hinge region is retained in the Fc moiety, the LINGO-4-Fc fusion will dimerize, thus forming a divalent molecule. A homogeneous population of monomeric Fc fusions will yield monospecific, bivalent dimers. A mixture of two monomeric Fc fusions each having a different specificity will yield bispecific, bivalent dimers.

Soluble LINGO-4 polypeptides can be fused to heterologous peptides to facilitate purification or identification of the soluble LINGO-4 moiety. For example, a histidine tag can be fused to a soluble LINGO-4 polypeptide to facilitate purification using commercially available chromatography media.

A "linker" sequence is a series of one or more amino acids separating two polypeptide coding regions in a fusion protein. A typical linker comprises at least 5 amino acids. Additional linkers comprise at least 10 or at least 15 amino acids. In certain embodiments, the amino acids of a peptide linker are selected so that the linker is hydrophilic. The linker (Gly-Gly-Gly-Gly-Ser)₃ (G₄S)₃ (SEQ ID NO:5) is a preferred linker that is widely applicable to many antibodies as it provides sufficient flexibility. Other linkers include (Gly-Gly-Gly-Gly-Ser)₂ (G₄S)₂ (SEQ ID NO:6), Glu Ser Gly Arg Ser Gly Gly Gly Gly Ser Gly Gly Gly Gly Ser (SEQ ID NO:7), Glu Gly Lys Ser Ser Gly Ser Gly Ser Glu Ser Lys Ser Thr (SEQ ID NO:8), Glu Gly Lys Ser Ser Gly Ser Gly Ser Glu Ser Lys Ser Thr Gln (SEQ ID NO:9), Glu Gly Lys Ser Ser Gly Ser Gly Ser Glu Ser Lys Val Asp (SEQ ID NO: 10), Gly Ser Thr Ser Gly Ser Gly Lys Ser Ser Glu Gly Lys Gly (SEQ ID NO:11), Lys Glu Ser Gly Ser Val Ser Ser Glu Gln Leu Ala Gln Phe Arg Ser Leu Asp (SEQ ID NO:12), and Glu Ser Gly Ser Val Ser Ser Glu Glu Leu Ala Phe Arg Ser Leu Asp (SEQ ID NO:13). Examples of shorter linkers include fragments of the above linkers, and examples of longer linkers include combinations of the linkers above, combinations of fragments of the linkers above, and combinations of the linkers above with fragments of the linkers above.

LINGO-4 polypeptides can be fused to a polypeptide tag. The term "polypeptide tag," as used herein, is intended to mean any sequence of amino acids that can be attached to, connected to, or linked to a LINGO-4 polypeptide and that can be used to identify, purify, concentrate or isolate the LINGO-4 polypeptide. The attachment of the polypeptide tag to the LINGO-4 polypeptide may occur, e.g., by constructing a nucleic acid molecule that comprises: (a) a nucleic acid sequence that encodes the polypeptide tag, and (b) a nucleic acid sequence that encodes an LINGO-4 polypeptide. Exemplary polypeptide tags include, e.g., amino acid sequences that are capable of being post-translationally modified, e.g., amino acid sequences that are biotinylated. Other exemplary polypeptide tags include, e.g., amino acid sequences that are capable of being recognized and/or bound by an antibody (or fragment thereof) or other specific binding reagent. Polypeptide tags that are capable of being recognized by an antibody (or fragment thereof) or other specific binding reagent include, e.g., those that are known in the art as "epitope tags." An epitope tag may be a natural or an artificial epitope tag. Natural and artificial epitope tags are known in the art, including, e.g., artificial epitopes such as FLAG, Strep, or poly-histidine peptides. FLAG peptides include the sequence Asp-Tyr-Lys-Asp-Asp-Asp-Asp-Lys (SEQ ID NO:14) or Asp-Tyr-Lys-Asp-Glu-Asp-Asp-Lys (SEQ ID NO:15) (Einhauer, A. and Jungbauer, A., J. Biochem. Biophys. Methods 49:1-3:455-465 (2001)). The Strep epitope has the sequence Ala-Trp-Arg-His-Pro-Gln-Phe-Gly-Gly (SEQ ID NO:16). The VSV-G epitope can also be used and has the sequence Tyr-Thr-Asp-Ile-Glu-Met-Asn-Arg-Leu-Gly-Lys (SEQ ID NO:17). Another artificial epitope is a poly-His sequence having six histidine residues (His-His-His-His-His-His (SEQ ID NO:18). Naturally-occurring epitopes include the influenza virus hemagglutinin (HA) sequence Tyr-Pro-Tyr-Asp-Val-Pro-Asp-Tyr-Ala-Ile-Glu-Gly-Arg (SEQ ID NO:19) recognized by the monoclonal antibody 12CA5 (Murray et al., Anal. Biochem. 229:170-179 (1995)) and the eleven amino acid sequence from human c-myc (Myc) recognized by the monoclonal antibody 9E10 (Glu-Gln-Lys-Leu-Leu-Ser-Glu-Glu-Asp-Leu-Asn (SEQ ID NO:20) (Manstein et al., Gene 162:129-134 (1995)). Another useful epitope is the tripeptide Glu-Glu-Phe which is recognized by the monoclonal antibody YL 1/2. (Stammers et al. FEBS Lett. 283:298-302(1991)).

In certain embodiments, the LINGO-4 polypeptide and the polypeptide tag may be connected via a linking amino acid sequence. As used herein, a "linking amino acid sequence" may be an amino acid sequence that is capable of being recognized and/or cleaved by one or more proteases. Amino acid sequences that can be recognized and/or cleaved by one or more proteases are known in the art. Exemplary amino acid sequences are those that are recognized by the following proteases: factor VIIa, factor IXa, factor Xa, APC, t-PA, u-PA, trypsin, chymotrypsin, enterokinase, pepsin, cathepsin B,H,L,S,D, cathepsin G, renin, angiotensin converting enzyme, matrix metalloproteases (collagenases, stromelysins, gelatinases), macrophage elastase, Cir, and Cis. The amino acid sequences that are recognized by the aforementioned proteases are known in the art. Exemplary sequences recognized by certain proteases can be found, e.g., in U.S. Patent No. 5,811,252.

In some embodiments of the invention, a soluble LINGO-4 fusion construct is used to enhance the production of a soluble LINGO-4 moiety in bacteria. In such constructs a bacterial protein normally expressed and/or secreted at a high level is employed as the N-terminal fusion partner of a soluble LINGO-4 polypeptide. *See, e.g.,* Smith et al., Gene 67:31 (1988); Hopp et al., Biotechnology 6:1204 (1988); La Vallie et al., Biotechnology 11:187 (1993).

By fusing a soluble LINGO-4 moiety at the amino and carboxy termini of a suitable fusion partner, bivalent or tetravalent forms of a soluble LINGO-4 polypeptide can be obtained. For example, a soluble LINGO-4 moiety can be fused to the amino and carboxy termini of an Ig moiety to produce a bivalent monomeric polypeptide containing two soluble LINGO-4 moieties. Upon dimerization of two of these monomers, by virtue of the Ig moiety, a tetravalent form of a soluble LINGO-4 protein is obtained. Such multivalent forms can be used to achieve increased binding affinity for the target. Multivalent forms of soluble LINGO-4 also can be obtained by placing soluble LINGO-4 moieties in tandem to form concatamers, which can be employed alone or fused to a fusion partner such as Ig or HSA.

### LINGO-4 Conjugates

LINGO-4 antagonist polypeptides and antibodies for use in the treatment methods disclosed herein include derivatives that are modified, *i.e.,* by the covalent attachment of any type of molecule such that covalent attachment does not prevent the LINGO-4 antagonist polypeptide or antibody from inhibiting the biological function of LINGO-4. For example, but not by way of limitation, the LINGO-4 antagonist polypeptides and antibodies of the present invention may be modified *e.g.,* by glycosylation, acetylation, pegylation, phosphylation, phosphorylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

LINGO-4 antagonist polypeptides and antibodies may be modified by natural processes, such as posttranslational processing, or by chemical modification techniques which are well known in the art. Such modifications are well described in basic texts and in more detailed monographs, as well as in a voluminous research literature. Modifications can occur anywhere in the LINGO-4 antagonist polypeptide or antibody, including the peptide backbone, the amino acid side-chains and the amino or carboxyl termini, or on moieties such as carbohydrates. It will be appreciated that the same type of modification may be present in the same or varying degrees at several sites in a given LINGO-4 antagonist polypeptide or antibody. Also, a given LINGO-4 antagonist polypeptide or antibody may contain many types of modifications. LINGO-4 antagonist polypeptides or antibodies may be branched, for example, as a result of ubiquitination, and they may be cyclic, with or without branching. Cyclic, branched, and branched cyclic LINGO-4 antagonist polypeptides and antibodies may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (*See,* for instance, Proteins - Structure And Molecular Properties, T. E. Creighton, W. H. Freeman and Company, New York 2nd Ed., (1993); Posttranslational Covalent Modification Of Proteins, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992)).

Any of a number of cross-linkers that contain a corresponding amino-reactive group and thiol-reactive group can be used to link LINGO-4 antagonist polypeptides to a heterologous fusion partner. Examples of suitable linkers include amine reactive cross-linkers that insert a thiol-reactive maleimide, *e.g*., SMCC, AMAS, BMPS, MBS, EMCS, SMPB, SMPH, KMUS, and GMBS. Other suitable linkers insert a thiol-reactive haloacetate group, *e.g.,* SBAP, SIA, SIAB. Linkers that provide a protected or non-protected thiol for reaction with sulfhydryl groups to product a reducible linkage include SPDP, SMPT, SATA, and SATP. Such reagents are commercially available (*e.g.,* Pierce Chemicals).

Conjugation does not have to involve the N-terminus of a soluble LINGO-4 polypeptide or the thiol moiety on serum albumin. For example, soluble LINGO-4-albumin fusions can be obtained using genetic engineering techniques, wherein the soluble LINGO-4 moiety is fused to the serum albumin gene at its N-terminus, C-terminus, or both.

Some embodiments of the invention involve a soluble LINGO-4 polypeptide or LINGO-4 antibody wherein one or more polymers are conjugated (covalently linked) to the LINGO-4 polypeptide or antibody. Examples of polymers suitable for such conjugation include polypeptides (discussed above), sugar polymers and polyalkylene glycol chains. Typically, but not necessarily, a polymer is conjugated to the soluble LINGO-4 polypeptide or LINGO-4 antibody for the purpose of improving one or more of the following: solubility, stability, or bioavailability.

The class of polymer generally used for conjugation to a LINGO-4 antagonist polypeptide or antibody is a polyalkylene glycol. Polyethylene glycol (PEG) is most frequently used. PEG moieties, *e.g.,* 1, 2, 3, 4 or 5 PEG polymers, can be conjugated to each LINGO-4 antagonist polypeptide or antibody to increase serum half life, as compared to the LINGO-4 antagonist polypeptide or antibody alone. PEG moieties are non-antigenic and essentially biologically inert. PEG moieties used in the practice of the invention may be branched or unbranched.

The number of PEG moieties attached to the LINGO-4 antagonist polypeptide or antibody and the molecular weight of the individual PEG chains can vary. In general, the higher the molecular weight of the polymer, the fewer polymer chains attached to the polypeptide. Usually, the total polymer mass attached to the LINGO-4 antagonist polypeptide or antibody is from 20 kDa to 40 kDa. Thus, if one polymer chain is attached, the molecular weight of the chain is generally 20-40 kDa. If two chains are attached, the molecular weight of each chain is generally 10-20 kDa. If three chains are attached, the molecular weight is generally 7-14 kDa.

The polymer, *e.g*., PEG, can be linked to the LINGO-4 antagonist polypeptide or antibody through any suitable, exposed reactive group on the polypeptide. The exposed reactive group(s) can be, *e.g*., an N-terminal amino group or the epsilon amino group of an internal lysine residue, or both. An activated polymer can react and covalently link at any free amino group on the LINGO-4 antagonist polypeptide or antibody. Free carboxylic groups, suitably activated carbonyl groups, hydroxyl, guanidyl, imidazole, oxidized carbohydrate moieties and mercapto groups of the LINGO-4 antagonist polypeptide or antibody (if available) also can be used as reactive groups for polymer attachment.

In a conjugation reaction, from about 1.0 to about 10 moles of activated polymer per mole of polypeptide, depending on polypeptide concentration, is typically employed. Usually, the ratio chosen represents a balance between maximizing the reaction while minimizing side reactions (often non-specific) that can impair the desired pharmacological activity of the LINGO-4 antagonist polypeptide or antibody. In certain embodiments, at least 50% of the biological activity (as demonstrated, *e.g*., in any of the assays described herein or known in the art) of the LINGO-4 antagonist polypeptide or antibody is retained. In further embodiments, nearly 100% is retained.

The polymer can be conjugated to the LINGO-4 antagonist polypeptide or antibody using conventional chemistry. For example, a polyalkylene glycol moiety can be coupled to a lysine epsilon amino group of the LINGO-4 antagonist polypeptide or antibody. Linkage to the lysine side chain can be performed with an N-hydroxylsuccinimide (NHS) active ester such as PEG succinimidyl succinate (SS-PEG) and succinimidyl propionate (SPA-PEG). Suitable polyalkylene glycol moieties include, *e.g*., carboxymethyl-NHS and norleucine-NHS, SC. These reagents are commercially available. Additional amine-reactive PEG linkers can be substituted for the succinimidyl moiety. These include, *e.g*., isothiocyanates, nitrophenylcarbonates (PNP), epoxides, benzotriazole carbonates, SC-PEG, tresylate, aldehyde, epoxide, carbonylimidazole and PNP carbonate. Conditions are usually optimized to maximize the selectivity and extent of reaction. Such optimization of reaction conditions is within ordinary skill in the art.

PEGylation can be carried out by any of the PEGylation reactions known in the art. *See, e.g.,* Focus on Growth Factors 3:4-10 (1992), and European patent applications EP0154316 and EP0401384. PEGylation may be carried out using an acylation reaction or an alkylation reaction with a reactive polyethylene glycol molecule (or an analogous reactive water-soluble polymer).

PEGylation by acylation generally involves reacting an active ester derivative of polyethylene glycol. Any reactive PEG molecule can be employed in the PEGylation. PEG esterified to N-hydroxysuccinimide (NHS) is a frequently used activated PEG ester. As used herein, "acylation" includes without limitation the following types of linkages between the therapeutic protein and a water-soluble polymer such as PEG: amide, carbamate, urethane, and the like. *See, e.g.,* Bioconjugate Chem. 5:133-140, 1994. Reaction parameters are generally selected to avoid temperature, solvent, and pH conditions that would damage or inactivate the soluble LINGO-4 polypeptide.

Generally, the connecting linkage is an amide and typically at least 95% of the resulting product is mono-, di- or tri-PEGylated. However, some species with higher degrees of PEGylation may be formed in amounts depending on the specific reaction conditions used. Optionally, purified PEGylated species are separated from the mixture, particularly unreacted species, by conventional purification methods, including, *e.g*., dialysis, salting-out, ultrafiltration, ion-exchange chromatography, gel filtration chromatography, hydrophobic exchange chromatography, and electrophoresis.

PEGylation by alkylation generally involves reacting a terminal aldehyde derivative of PEG with LINGO-4 antagonist polypeptide or antibody in the presence of a reducing agent. In addition, one can manipulate the reaction conditions to favor PEGylation substantially only at the N-terminal amino group of a LINGO-4 antagonist polypeptide or antibody, *i.e.* a mono-PEGylated protein. In either case of mono-PEGylation or poly-PEGylation, the PEG groups are typically attached to the protein via a - C_{H}2-NH- group. With particular reference to the - C_{H}2-group, this type of linkage is known as an "alkyl" linkage.

Derivatization via reductive alkylation to produce an N-terminally targeted mono-PEGylated product exploits differential reactivity of different types of primary amino groups (lysine versus the N-terminal) available for derivatization. The reaction is performed at a pH that allows one to take advantage of the pKa differences between the epsilon-amino groups of the lysine residues and that of the N-terminal amino group of the protein. By such selective derivatization, attachment of a water-soluble polymer that contains a reactive group, such as an aldehyde, to a protein is controlled: the conjugation with the polymer takes place predominantly at the N-terminus of the protein and no significant modification of other reactive groups, such as the lysine side chain amino groups, occurs.

The polymer molecules used in both the acylation and alkylation approaches are selected from among water-soluble polymers. The polymer selected is typically modified to have a single reactive group, such as an active ester for acylation or an aldehyde for alkylation, so that the degree of polymerization may be controlled as provided for in the present methods. An exemplary reactive PEG aldehyde is polyethylene glycol propionaldehyde, which is water stable, or mono C1-C10 alkoxy or aryloxy derivatives thereof (*see, e.g*., Harris et al., U.S. Pat. No. 5,252,714). The polymer may be branched or unbranched. For the acylation reactions, the polymer(s) selected typically have a single reactive ester group. For reductive alkylation, the polymer(s) selected typically have a single reactive aldehyde group. Generally, the water-soluble polymer will not be selected from naturally occurring glycosyl residues, because these are usually made more conveniently by mammalian recombinant expression systems.

Methods for preparing a PEGylated soluble LINGO-4 polypeptide or antibody generally includes the steps of (a) reacting a LINGO-4 antagonist polypeptide or antibody with polyethylene glycol (such as a reactive ester or aldehyde derivative of PEG) under conditions whereby the molecule becomes attached to one or more PEG groups, and (b) obtaining the reaction product(s). In general, the optimal reaction conditions for the acylation reactions will be determined case-by-case based on known parameters and the desired result. For example, a larger the ratio of PEG to protein, generally leads to a greater the percentage of poly-PEGylated product.

Reductive alkylation to produce a substantially homogeneous population of mono-polymer/soluble LINGO-4 polypeptide or LINGO-4 antibody generally includes the steps of: (a) reacting a soluble LINGO-4 protein or polypeptide with a reactive PEG molecule under reductive alkylation conditions, at a pH suitable to permit selective modification of the N-terminal amino group of the polypeptide or antibody; and (b) obtaining the reaction product(s).

For a substantially homogeneous population of mono-polymer/soluble LINGO-4 polypeptide or LINGO-4 antibody, the reductive alkylation reaction conditions are those that permit the selective attachment of the water-soluble polymer moiety to the N-terminus of the polypeptide or antibody. Such reaction conditions generally provide for pKa differences between the lysine side chain amino groups and the N-terminal amino group. For purposes of the present invention, the pH is generally in the range of 3-9, typically 3-6.

Soluble LINGO-4 polypeptides or antibodies can include a tag, *e.g*., a moiety that can be subsequently released by proteolysis. Thus, the lysine moiety can be selectively modified by first reacting a His-tag modified with a low-molecular-weight linker such as Traut's reagent (Pierce) which will react with both the lysine and N-terminus, and then releasing the His tag. The polypeptide will then contain a free SH group that can be selectively modified with a PEG containing a thiol-reactive head group such as a maleimide group, a vinylsulfone group, a haloacetate group, or a free or protected SH.

Traut's reagent can be replaced with any linker that will set up a specific site for PEG attachment. For example, Traut's reagent can be replaced with SPDP, SMPT, SATA, or SATP (Pierce). Similarly one could react the protein with an amine-reactive linker that inserts a maleimide (for example SMCC, AMAS, BMPS, MBS, EMCS, SMPB, SMPH, KMUS, or GMBS), a haloacetate group (SBAP, SIA, SIAB), or a vinylsulfone group and react the resulting product with a PEG that contains a free SH.

In some embodiments, the polyalkylene glycol moiety is coupled to a cysteine group of the LINGO-4 antagonist polypeptide or antibody. Coupling can be effected using, *e.g*., a maleimide group, a vinylsulfone group, a haloacetate group, or a thiol group.

Optionally, the soluble LINGO-4 polypeptide or antibody is conjugated to the polyethylene-glycol moiety through a labile bond. The labile bond can be cleaved in, *e.g*., biochemical hydrolysis, proteolysis, or sulfhydryl cleavage. For example, the bond can be cleaved under *in vivo* (physiological) conditions.

The reactions may take place by any suitable method used for reacting biologically active materials with inert polymers, generally at about pH 5-8, *e.g*., pH 5, 6, 7, or 8, if the reactive groups are on the alpha amino group at the N-terminus. Generally the process involves preparing an activated polymer and thereafter reacting the protein with the activated polymer to produce the soluble protein suitable for formulation.

### LINGO-4 Polynucleotide Antagonists

Specific instances comprise a method of treating a demyelination or dysmyelination disorder, comprising administering an effective amount of a LINGO-4 polynucleotide antagonist which comprises a nucleic acid molecule which specifically binds to a polynucleotide which encodes LINGO-4. The LINGO-4 polynucleotide antagonist prevents expression of LINGO-4 (knockdown). In certain embodiments of the present invention, the LINGO-4 polynucleotide antagonist promotes proliferation, differentiation, or survival of oligodendrocytes; promotes, oligodendrocyte-mediated myelination of neurons, or prevents demyelination, e.g., in a mammal. LINGO-4 polynucleotide antagonists include, but are not limited to antisense molecules, ribozymes, siRNA, shRNA and RNAi. Typically, such binding molecules are separately administered to the animal (*see,* for example, O'Connor, J. Neurochem. 56:560 (1991), but such binding molecules may also be expressed *in vivo* from polynucleotides taken up by a host cell and expressed *in vivo. See also* Oligodeoxynucleotides as Antisense Inhibitors of Gene Expression, CRC Press, Boca Raton, FL (1988).

RNAi refers to the expression of an RNA which interferes with the expression of the targeted mRNA. Specifically, the RNAi silences a targeted gene via interacting with the specific mRNA (*e.g*. LINGO-4) through a siRNA (short interfering RNA). The ds RNA complex is then targeted for degradation by the cell. Additional RNAi molecules include Short hairpin RNA (shRNA); also short interfering hairpin. The shRNA molecule contains sense and antisense sequences from a target gene connected by a loop. The shRNA is transported from the nucleus into the cytoplasm, it is degraded along with the mRNA. Pol III or U6 promoters can be used to express RNAs for RNAi.

RNAi is mediated by double stranded RNA (dsRNA) molecules that have sequence-specific homology to their "target" mRNAs (Caplen et al., Proc Natl Acad Sci USA 98:9742-9747, 2001). Biochemical studies in *Drosophila* cell-free lysates indicates that the mediators of RNA-dependent gene silencing are 21-25 nucleotide "small interfering" RNA duplexes (siRNAs). Accordingly, siRNA molecules are advantageously used in the present invention. The siRNAs are derived from the processing of dsRNA by an RNase known as DICER (Bernstein et al., Nature 409:363-366, 2001). It appears that siRNA duplex products are recruited into a multi-protein siRNA complex termed RISC (RNA Induced Silencing Complex). Without wishing to be bound by any particular theory, it is believed that a RISC is guided to a target mRNA, where the siRNA duplex interacts sequence-specifically to mediate cleavage in a catalytic fashion (Bernstein et al., Nature 409:363-366, 2001; Boutla et al., Curr Biol 11:1776-1780, 2001).

RNAi has been used to analyze gene function and to identify essential genes in mammalian cells (Elbashir et al., Methods 26:199-213, 2002; Harborth et al., J Cell Sci 114:4557-4565, 2001), including by way of non-limiting example neurons (Krichevsky et al., Proc Natl Acad Sci USA 99:11926-11929, 2002). RNAi is also being evaluated for therapeutic modalities, such as inhibiting or blocking the infection, replication and/or growth of viruses, including without limitation poliovirus (Gitlin et al., Nature 418:379-380, 2002) and HIV (Capodici et al., J Immunol 169:5196-5201, 2002), and reducing expression of oncogenes (*e.g.,* the bcr-abl gene; Scherr et al., Blood Sep 26 epub ahead of print, 2002). RNAi has been used to modulate gene expression in mammalian (mouse) and amphibian (*Xenopus*) embryos (respectively, Calegari et al., Proc Natl Acad Sci USA 99:14236-14240, 2002; and Zhou, et al., Nucleic Acids Res 30:1664-1669, 2002), and in postnatal mice (Lewis et al., Nat Gene 32:107-108, 2002), and to reduce trangsene expression in adult transgenic mice (McCaffrey et al., Nature 418:38-39, 2002). Methods have been described for determining the efficacy and specificity of siRNAs in cell culture and *in vivo* (see, *e.g.,* Bertrand et al., Biochem Biophys Res Commun 296:1000-1004, 2002; Lassus et al., Sci STKE 2002(147):PL13, 2002; and Leirdal et al., Biochem Biophys Res Commun 295:744-748, 2002).

Molecules that mediate RNAi, including without limitation siRNA, can be produced *in vitro* by chemical synthesis (Hohjoh, FEBS Lett 521:195-199, 2002), hydrolysis of dsRNA (Yang et al., Proc Natl Acad Sci USA 99:9942-9947, 2002), by *in vitro* transcription with T7 RNA polymerase (Donzeet et al., Nucleic Acids Res 30:e46, 2002; Yu et al., Proc Natl Acad Sci USA 99:6047-6052, 2002), and by hydrolysis of double-stranded RNA using a nuclease such as *E. coli* RNase III (Yang et al., Proc Natl Acad Sci USA 99:9942-9947, 2002).

siRNA molecules may also be formed by annealing two oligonucleotides to each other, typically have the following general structure, which includes both double-stranded and single-stranded portions:

Wherein N, X and Y are nucleotides; X hydrogen bonds to Y; ":" signifies a hydrogen bond between two bases; ***x*** is a natural integer having a value between 1 and about 100; and ***m*** and ***n*** are whole integers having, independently, values between 0 and about 100. In some embodiments, N, X and Y are independently A, G, C and T or U. Non-naturally occurring bases and nucleotides can be present, particularly in the case of synthetic siRNA (*i.e.,* the product of annealing two oligonucleotides). The double-stranded central section is called the "core" and has base pairs (bp) as units of measurement; the single-stranded portions are overhangs, having nucleotides (nt) as units of measurement. The overhangs shown are 3' overhangs, but molecules with 5' overhangs are also within the scope of the invention. Also within the scope of the invention are siRNA molecules with no overhangs (*i.e., **m*** = 0 and ***n** =* 0), and those having an overhang on one side of the core but not the other (*e.g., **m*** = 0 and ***n*** ≥ 1, or vice-versa).

Initially, RNAi technology did not appear to be readily applicable to mammalian systems. This is because, in mammals, dsRNA activates dsRNA-activated protein kinase (PKR) resulting in an apoptotic cascade and cell death (Der et al, Proc. Natl. Acad. Sci. USA 94:3279-3283, 1997). In addition, it has long been known that dsRNA activates the interferon cascade in mammalian cells, which can also lead to altered cell physiology (Colby et al, Annu. Rev. Microbiol. 25:333, 1971; Kleinschmidt et al., Annu. Rev. Biochem. 41:517, 1972; Lampson et al., Proc. Natl. Acad. Sci. USA 58L782, 1967; Lomniczi et al., J. Gen. Virol. 8:55, 1970; and Younger et al., J. Bacteriol. 92:862, 1966). However, dsRNA-mediated activation of the PKR and interferon cascades requires dsRNA longer than about 30 base pairs. In contrast, dsRNA less than 30 base pairs in length has been demonstrated to cause RNAi in mammalian cells (Caplen et al., Proc. Natl. Acad. Sci. USA 98:9742-9747, 2001). Thus, it is expected that undesirable, non-specific effects associated with longer dsRNA molecules can be avoided by preparing short RNA that is substantially free from longer dsRNAs.

References regarding siRNA: Bernstein et al., Nature 409:363-366, 2001; Boutla et al., Curr Biol 11:1776-1780, 2001; Cullen, Nat Immunol. 3:597-599, 2002; Caplen et al., Proc Natl Acad Sci USA 98:9742-9747, 2001; Hamilton et al., Science 286:950-952, 1999; Nagase et al., DNA Res. 6:63-70, 1999; Napoli et al., Plant Cell 2:279-289, 1990; Nicholson et al., Mamm. Genome 13:67-73, 2002; Parrish et al., Mol Cell 6:1077-1087, 2000; Romano et al., Mol Microbiol 6:3343-3353, 1992; Tabara et al., Cell 99:123-132, 1999; and Tuschl, Chembiochem. 2:239-245, 2001.

Paddison et al. (Genes & Dev. 16:948-958, 2002) have used small RNA molecules folded into hairpins as a means to effect RNAi. Accordingly, such short hairpin RNA (shRNA) molecules are also advantageously used in the invention. The length of the stem and loop of functional shRNAs varies; stem lengths can range anywhere from about 25 to about 30 nt, and loop size can range between 4 to about 25 nt without affecting silencing activity. While not wishing to be bound by any particular theory, it is believed that these shRNAs resemble the dsRNA products of the DICER RNase and, in any event, have the same capacity for inhibiting expression of a specific gene.

In some embodiments, the shRNA is expressed from a lentiviral vector (*e.g*., pLL3.7).

Antisense technology can be used to control gene expression through antisense DNA or RNA, or through triple-helix formation. Antisense techniques are discussed for example, in Okano, J. Neurochem. 56:560 (1991); Oligodeoxynucleotides as Antisense Inhibitors of Gene Expression, CRC Press, Boca Raton, FL (1988). Triple helix formation is discussed in, for instance, Lee et al., Nucleic Acids Research 6:3073 (1979); Cooney et al., Science 241:456 (1988); and Dervan et al., Science 251:1300 (1991). The methods are based on binding of a polynucleotide to a complementary DNA or RNA.

For example, the 5' coding portion of a polynucleotide that encodes LINGO-4 may be used to design an antisense RNA oligonucleotide of from about 10 to 40 base pairs in length. A DNA oligonucleotide is designed to be complementary to a region of the gene involved in transcription thereby preventing transcription and the production of the target protein. The antisense RNA oligonucleotide hybridizes to the mRNA *in vivo* and blocks translation of the mRNA molecule into the target polypeptide.

In one embodiment, antisense nucleic acids specific for the LINGO-4 gene are produced intracellularly by transcription from an exogenous sequence. For example, a vector or a portion thereof, is transcribed, producing an antisense nucleic acid (RNA). Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in vertebrate cells. Expression of the antisense molecule can be by any promoter known in the art to act in vertebrate, *e.g*., human cells, such as those described elsewhere herein.

Absolute complementarity of an antisense molecule is not required. A sequence complementary to at least a portion of an RNA encoding LINGO-4, means a sequence having sufficient complementarity to be able to hybridize with the RNA, forming a stable duplex; or triplex. The ability to hybridize will depend on both the degree of complementarity and the length of the antisense nucleic acid. Generally, the larger the hybridizing nucleic acid, the more base mismatches it may contain and still form a stable duplex (or triplex as the case may be). One skilled in the art can ascertain a tolerable degree of mismatch by use of standard procedures to determine the melting point of the hybridized complex.

Oligonucleotides that are complementary to the 5' end of a messenger RNA, *e.g*., the 5' untranslated sequence up to and including the AUG initiation codon, should work most efficiently at inhibiting translation. However, sequences complementary to the 3' untranslated sequences of mRNAs have been shown to be effective at inhibiting translation of mRNAs as well. See generally, Wagner, R., Nature 372:333-335 (1994). Thus, oligonucleotides complementary to either the 5'- or 3'- non- translated, non-coding regions could be used in an antisense approach to inhibit translation of LINGO-4. Oligonucleotides complementary to the 5' untranslated region of the mRNA should include the complement of the AUG start codon. Antisense oligonucleotides complementary to mRNA coding regions are less efficient inhibitors of translation but could be used in accordance with the invention. Antisense nucleic acids are typically at least six nucleotides in length, for example, oligonucleotides ranging from 6 to about 50 nucleotides in length. In specific aspects the oligonucleotide is at least 10 nucleotides, at least 17 nucleotides, at least 25 nucleotides or at least 50 nucleotides.

Polynucleotides for use the therapeutic methods disclosed herein can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (*e.g*., for targeting host cell receptors *in vivo*), or agents facilitating transport across the cell membrane (see, *e.g.,* Letsinger et al., Proc. Natl. Acad. Sci. U.S.A. 86:6553-6556 (1989); Lemaitre et al., Proc. Natl. Acad. Sci. 84:648-652 (1987)); PCT Publication No. WO88/09810, published December 15, 1988) or the blood-brain barrier (see, *e.g.,* PCT Publication No. WO89/10134, published April 25, 1988), hybridization-triggered cleavage agents. (*See, e.g.,* Krol et al., BioTechniques 6:958-976 (1988)) or intercalating agents. (*See, e.g.,* Zon, Pharm. Res. 5:539-549(1988)). To this end, the oligonucleotide may be conjugated to another molecule, *e.g*., a peptide, hybridization triggered cross-linking agent, transport agent, hybridization-triggered cleavage agent, etc.

An antisense oligonucleotide for use in the therapeutic methods disclosed herein may comprise at least one modified base moiety which is selected from the group including, but not limited to, 5fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N-6-isopentenyladenine, 1-methylguanine, 1-methylinosine, 2,2-dimethylguanine, 2-methyladenine, 2-methylguanine, 3-methylcytosine, 5-methylcytosine, N-6-adenine, 7-methylguanine, 5-methylaminomethyluracil, 5-methoxyaminomethyl-2-thiouracil, beta-D-mannosylqueosine, 5'methoxycarboxymethyluracil, 5-methoxyuracil, 2-methylthio-N-6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3(3-amino-3-N2-carboxypropyl) uracil, (acp3)w, and 2,6-diaminopurine.

An antisense oligonucleotide for use in the therapeutic methods disclosed herein may also comprise at least one modified sugar moiety selected from the group including, but not limited to, arabinose, 2-fluoroarabinose, xylulose, and hexose.

In yet another embodiment, an antisense oligonucleotide for use in the therapeutic methods disclosed herein comprises at least one modified phosphate backbone selected from the group including, but not limited to, a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, an antisense oligonucleotide for use in the therapeutic methods disclosed herein is an α-anomeric oligonucleotide. An α-anomeric oligonucleotide forms specific double-stranded hybrids with complementary RNA in which, contrary to the usual situation, the strands run parallel to each other (Gautier et al., Nucl. Acids Res. 15:6625-6641(1987)). The oligonucleotide is a 2'-0-methylribonucleotide (Inoue et al., Nucl. Acids Res. 15:6131-6148(1987)), or a chimeric RNA-DNA analogue (Inoue et al., FEBS Lett. 215:327-330(1987)).

Polynucleotides of the invention may be synthesized by standard methods known in the art, *e.g*. by use of an automated DNA synthesizer (such as are commercially available from Biosearch, Applied Biosystems, etc.). As examples, phosphorothioate oligonucleotides may be synthesized by the method of Stein et al., Nucl. Acids Res. 16:3209 (1988), methylphosphonate oligonucleotides can be prepared by use of controlled pore glass polymer supports (Sarin et al., Proc. Natl. Acad. Sci. U.S.A. 85:7448-7451(1988)), etc.

Polynucleotide compositions for use in the therapeutic methods disclosed herein further include catalytic RNA, or a ribozyme (*See*, *e.g*., PCT International Publication WO 90/11364, published October 4, 1990; Sarver et al., Science 247:1222-1225 (1990). Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, Nature 334:585-591 (1988). In certain embodiments, the ribozyme is engineered so that the cleavage recognition site is located near the 5' end of the target mRNA; *i.e*., to increase efficiency and minimize the intracellular accumulation of non-functional mRNA transcripts.

As in the antisense approach, ribozymes for use in the diagnostic and therapeutic methods disclosed herein can be composed of modified oligonucleotides (*e.g*. for improved stability, targeting, etc.) and may be delivered to cells which express LINGO-4 *in vivo.* DNA constructs encoding the ribozyme may be introduced into the cell in the same manner as described above for the introduction of antisense encoding DNA. One method of delivery involves using a DNA construct "encoding" the ribozyme under the control of a strong constitutive or inducible promoter, such as, for example, pol III or pol II promoter, so that transfected cells will produce sufficient quantities of the ribozyme to destroy endogenous LINGO-4 messages and inhibit translation. Since ribozymes unlike antisense molecules, are catalytic, a lower intracellular concentration is required for efficiency.

### LINGO-4 Aptamer Antagonists

In another embodiment, the LINGO-4 antagonist for use in the present invention is an aptamer. An aptamer can be a nucleotide or a polypeptide which has a unique sequence, has the property of binding specifically to a desired target (e.g., a polypeptide), and is a specific ligand of a given target. Nucleotide aptamers of the invention include double stranded DNA and single stranded RNA molecules that bind to LINGO-4. In certain embodiments of the present invention, the LINGO-4 aptamer antagonist promotes proliferation, differentiation, or survival of oligodendrocytes; promotes, oligodendrocyte-mediated myelination of neurons, or prevents demyelination, e.g., in a mammal.

Nucleic acid aptamers are selected using methods known in the art, for example via the Systematic Evolution of Ligands by Exponential Enrichment (SELEX) process. SELEX is a method for the in vitro evolution of nucleic acid molecules with highly specific binding to target molecules as described in e.g. U.S. Pat. Nos. 5,475,096, 5,580,737, 5,567,588, 5,707,796, 5,763,177, 6, 011,577, and 6,699,843. Another screening method to identify aptamers is described in U.S. Pat. No. 5,270,163). The SELEX process is based on the capacity of nucleic acids for forming a variety of two- and three- dimensional structures, as well as the chemical versatility available within the nucleotide monomers to act as ligands (form specific binding pairs) with virtually any chemical compound, whether monomeric or polymeric, including other nucleic acid molecules and polypeptides. Molecules of any size or composition can serve as targets.

The SELEX method involves selection from a mixture of candidate oligonucleotides and step-wise iterations of binding, partitioning and amplification, using the same general selection scheme, to achieve desired binding affinity and selectivity. Starting from a mixture of nucleic acids, preferably comprising a segment of randomized sequence, the SELEX method includes steps of contacting the mixture with the target under conditions favorable for binding; partitioning unbound nucleic acids from those nucleic acids which have bound specifically to target molecules; dissociating the nucleic acid-target complexes; amplifying the nucleic acids dissociated from the nucleic acid-target complexes to yield a ligand enriched mixture of nucleic acids. The steps of binding, partitioning, dissociating and amplifying are repeated through as many cycles as desired to yield highly specific high affinity nucleic acid ligands to the target molecule.

Nucleotide aptamers may be used, for example, as diagnostic tools or as specific inhibitors to dissect intracellular signaling and transport pathways (James (2001) Curr. Opin. Pharmacol. 1:540-546). The high affinity and specificity of nucleotide aptamers makes them good candidates for drug discovery. For example, aptamer antagonists to the toxin ricin have been isolated and have IC50 values in the nanomolar range (Hesselberth JR et al. (2000) J Biol Chem 275:4937-4942). Nucleotide aptamers may also be used against infectious disease, malignancy and viral surface proteins to reduce cellular infectivity.

Nucleotide aptamers for use in the present invention may be modified (e.g., by modifying the backbone or bases or conjugated to peptides) as described herein for other polynucleotides.

Using the protein structure of LINGO-4, screening for aptamers that act on LINGO-4 using the SELEX process would allow for the identification of aptamers that inhibit LINGO-4-mediated processes.

Polypeptide aptamers for use in the present invention are random peptides selected for their ability to bind to and thereby block the action of LINGO-4. Polypeptide aptamers may include a short variable peptide domain attached at both ends to a protein scaffold. This double structural constraint greatly increases the binding affinity of the peptide aptamer to levels comparable to an antibody's (nanomolar range). See, e.g., Hoppe-Seyler F et al. (2000) J Mol Med 78(8):426-430. The length of the short variable peptide is typically about 10 to 20 amino acids, and the scaffold may be any protein which has good solubility and compacity properties. One non-limiting example of a scaffold protein is the bacterial protein Thioredoxin-A. See, e.g., Cohen BA et al. (1998) PNAS 95(24): 14272-14277.

Polypeptide aptamers are peptides or small polypeptides that act as dominant inhibitors of protein function. Peptide aptamers specifically bind to target proteins, blocking their functional ability (Kolonin et al. (1998) Proc. Natl. Acad. Sci. 95: 14,266-14,271). Peptide aptamers that bind with high affinity and specificity to a target protein can be isolated by a variety of techniques known in the art. Peptide aptamers can be isolated from random peptide libraries by yeast two-hybrid screens (Xu, C.W., et al. (1997) Proc. Natl. Acad. Sci. 94:12,473-12,478) or by ribosome display (Hanes et al. (1997) Proc. Natl. Acad. Sci. 94:4937-4942). They can also be isolated from phage libraries (Hoogenboom, H.R., et al. (1998) Immunotechnology 4:1-20) or chemically generated peptide libraries. Additionally, polypeptide aptamers may be selected using the selection of Ligand Regulated Peptide Aptamers (LiRPAs). See, e.g., Binkowski BF et al., (2005) Chem & Biol 12(7): 847-855. Although the difficult means by which peptide aptamers are synthesized makes their use more complex than polynucleotide aptamers, they have unlimited chemical diversity. Polynucleotide aptamers are limited because they utilize only the four nucleotide bases, while peptide aptamers would have a much-expanded repertoire (i.e., 20 amino acids).

Peptide aptamers for use in the present invention may be modified (e.g., conjugated to polymers or fused to proteins) as described for other polypeptides elsewhere herein.

### Vectors and Host Cells

Host-expression systems represent vehicles by which the coding sequences of interest may be produced and subsequently purified, but also represent cells which may, when transformed or transfected with the appropriate nucleotide coding sequences, express a LINGO-4 antagonist polypeptide or antibody of the invention *in situ.* These include but are not limited to microorganisms such as bacteria (*e.g*., *E*. *coli*, *B*. *subtilis*) transformed with recombinant bacteriophage DNA, plasmid DNA or cosmid DNA expression vectors containing antibody coding sequences; yeast (*e*.*g*., *Saccharomyces*, *Pichia*) transformed with recombinant yeast expression vectors containing antibody coding sequences; insect cell systems infected with recombinant virus expression vectors (*e.g*., baculovirus) containing antibody coding sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing antibody coding sequences; or mammalian cell systems (*e.g.,* COS, CHO, BLK, 293, 3T3 cells) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter). Bacterial cells such as *Escherichia coli*, or eukaryotic cells, *e.g.,* for the expression of whole recombinant antibody molecules, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., Gene 45:101 (1986); Cockett et al., Bio/Technology 8:2 (1990)).

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited, to the *E. coli* expression vector pUR278 (Ruther et al., EMBO J. 2:1791 (1983)), in which the antibody coding sequence may be ligated individually into the vector in frame with the lacZ coding region so that a fusion protein is produced; pIN vectors (Inouye & Inouye, Nucleic Acids Res. 13:3101-3109 (1985); Van Heeke & Schuster, J. Biol. Chem. 24:5503-5509 (1989)); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is typically used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, *e.g.,* the late promoter and tripartite leader sequence. This chimeric gene may then be inserted in the adenovirus genome by *in vitro* or *in vivo* recombination. Insertion in a non- essential region of the viral genome (*e.g*., region E1 or E3) will result in a recombinant virus that is viable and capable of expressing the antibody molecule in infected hosts. (*e.g*., see Logan & Shenk, Proc. Natl. Acad. Sci. USA 81:355-359 (1984)). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (*see* Bittner et al., Methods in Enzymol. 153:51-544 (1987)).

In addition, a host cell strain may be chosen which modulates the expression of the inserted sequences, or modifies and processes the gene product in the specific fashion desired. Such modifications *(*e*.g*., glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, HeLa, COS, MDCK, 293, 3T3, WI38, and in particular, breast cancer cell lines such as, for example, BT483, Hs578T, HTB2, BT20 and T47D, and normal mammary gland cell line such as, for example, CRL7030 and Hs578Bst.

For long-term, high-yield production of recombinant proteins, stable expression is typically used. For example, cell lines which stably express the antibody molecule may be engineered. Rather than using expression vectors which contain viral origins of replication, host cells can be transformed with DNA controlled by appropriate expression control elements (*e.g*., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which stably express the antibody molecule.

A number of selection systems may be used, including but not limited to the herpes simplex virus thymidine kinase (Wigler et al., Cell 11:223 (1977)), hypoxanthine-guanine phosphoribosyltransferase (Szybalska & Szybalski, Proc. Natl. Acad. Sci. USA 48:202 (1992)), and adenine phosphoribosyltransferase (Lowy et al., Cell 22:817 1980) genes can be employed in tk-, hgprt- or aprt-cells, respectively. Also, antimetabolite resistance can be used as the basis of selection for the following genes: dhfr, which confers resistance to methotrexate (Wigler et al., Natl. Acad. Sci. USA 77:357 (1980); O'Hare et al., Proc. Natl. Acad. Sci. USA 78:1527 (1981)); gpt, which confers resistance to mycophenolic acid (Mulligan & Berg, Proc. Natl. Acad. Sci. USA 78:2072 (1981)); neo, which confers resistance to the aminoglycoside G-418 Clinical Pharmacy 12:488-505; Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); TIB TECH 11(5):155-215 (May, 1993); and hygro, which confers resistance to hygromycin (Santerre et al., Gene 30:147 (1984). Methods commonly known in the art of recombinant DNA technology which can be used are described in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Prolocols in Human Genetics, John Wiley & Sons, NY (1994); Colberre-Garapin et al., J. Mol. Bioi. 150:1 (1981).

The expression levels of a LINGO-4 polypeptide or antibody can be increased by vector amplification (for a review, *see* Bebbington and Hentschel, *The use of vectors based on gene amplification for the expression of cloned genes in mammalian cells in* DNA cloning, Academic Press, New York, Vol. 3. (1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., Mol. Cell. Biol. 3:257 (1983)).

Vectors comprising nucleic acids encoding LINGO-4 antagonists, *e.g*., soluble LINGO-4 polypeptides, LINGO-4 antibodies, LINGO-4 antagonist polynucleotides, or LINGO-4 aptamers, may be used to produce antagonists for use in the invention. The choice of vector and expression control sequences to which such nucleic acids are operably linked depends on the functional properties desired, *e.g*., protein expression, and the host cell to be transformed.

Expression control elements useful for regulating the expression of an operably linked coding sequence are known in the art. Examples include, but are not limited to, inducible promoters, constitutive promoters, secretion signals, and other regulatory elements. When an inducible promoter is used, it can be controlled, *e.g*., by a change in nutrient status, or a change in temperature, in the host cell medium.

The vector can include a prokaryotic replicon, *i.e*., a DNA sequence having the ability to direct autonomous replication and maintenance of the recombinant DNA molecule extra-chromosomally in a bacterial host cell. Such replicons are well known in the art. In addition, vectors that include a prokaryotic replicon may also include a gene whose expression confers a detectable marker such as a drug resistance. Examples of bacterial drug-resistance genes are those that confer resistance to ampicillin or tetracycline.

Vectors that include a prokaryotic replicon can also include a prokaryotic or bacteriophage promoter for directing expression of the coding gene sequences in a bacterial host cell. Promoter sequences compatible with bacterial hosts are typically provided in plasmid vectors containing convenient restriction sites for insertion of a DNA segment to be expressed. Examples of such plasmid vectors are pUC8, pUC9, pBR322 and pBR329 (BioRad), pPL and pKK223 (Pharmacia). Any suitable prokaryotic host can be used to express a recombinant DNA molecule encoding a protein used in the invention.

For the purposes of this invention, numerous expression vector systems may be employed. For example, one class of vector utilizes DNA elements which are derived from animal viruses such as bovine papilloma virus, polyoma virus, adenovirus, vaccinia virus, baculovirus, retroviruses (RSV, MMTV or MOMLV) or SV40 virus. Others involve the use of polycistronic systems with internal ribosome binding sites. Additionally, cells which have integrated the DNA into their chromosomes may be selected by introducing one or more markers which allow selection of transfected host cells. The marker may provide for prototrophy to an auxotrophic host, biocide resistance (*e.g*., antibiotics) or resistance to heavy metals such as copper. The selectable marker gene can either be directly linked to the DNA sequences to be expressed, or introduced into the same cell by cotransformation. The neomycin phosphotransferase (neo) gene is an example of a selectable marker gene (Southern et al., J. Mol. Anal. Genet. 1:327-341 (1982)). Additional elements may also be needed for optimal synthesis of mRNA. These elements may include signal sequences, splice signals, as well as transcriptional promoters, enhancers, and termination signals.

In one embodiment, a proprietary expression vector of Biogen IDEC, Inc., referred to as NEOSPLA (U.S. patent 6,159,730) may be used. This vector contains the cytomegalovirus promoter/enhancer, the mouse beta globin major promoter, the SV40 origin of replication, the bovine growth hormone polyadenylation sequence, neomycin phosphotransferase exon 1 and exon 2, the dihydrofolate reductase gene and leader sequence. This vector has been found to result in very high level expression upon transfection in CHO cells, followed by selection in G418 containing medium and methotrexate amplification. Of course, any expression vector which is capable of eliciting expression in eukaryotic cells may be used in the present invention. Examples of suitable vectors include, but are not limited to plasmids pcDNA3, pHCMV/Zeo, pCR3.1, pEFl/His, pIND/GS, pRc/HCMV2, pSV40/Zeo2, pTRACER-HCMV, pUB6N5-His, pVAX1, and pZeoSV2 (available from Invitrogen, San Diego, CA), and plasmid pCI (available from Promega, Madison, WI). Additional eukaryotic cell expression vectors are known in the art and are commercially available. Typically, such vectors contain convenient restriction sites for insertion of the desired DNA segment. Exemplary vectors include pSVL and pKSV-10 (Pharmacia), pBPV-1, pml2d (International Biotechnologies), pTDT1 (ATCC 31255), retroviral expression vector pMIG and pLL3.7, adenovirus shuttle vector pDC315, and AAV vectors. Other exemplary vector systems are disclosed *e.g*., in U.S. Patent 6,413,777.

In general, screening large numbers of transformed cells for those which express suitably high levels of the antagonist is routine experimentation which can be carried out, for example, by robotic systems.

Frequently used regulatory sequences for mammalian host cell expression include viral elements that direct high levels of protein expression in mammalian cells, such as promoters and enhancers derived from retroviral LTRs, cytomegalovirus (CMV) (such as the CMV promoter/enhancer), Simian Virus 40 (SV40) (such as the SV40 promoter/enhancer), adenovirus, (*e.g*., the adenovirus major late promoter (AdmlP)), polyoma and strong mammalian promoters such as native immunoglobulin and actin promoters. For further description of viral regulatory elements, and sequences thereof, *see e*.*g*., Stinski, U.S. Pat. No. 5,168,062; Bell, U.S. Pat. No. 4,510,245; and Schaffner, U.S. Pat. No. 4,968,615.

The recombinant expression vectors may carry sequences that regulate replication of the vector in host cells (*e.g*., origins of replication) and selectable marker genes. The selectable marker gene facilitates selection of host cells into which the vector has been introduced (*see*, *e.g*., Axel, U.S. Pat. Nos. 4,399,216; 4,634,665 and 5,179,017). For example, typically the selectable marker gene confers resistance to a drug, such as G418, hygromycin or methotrexate, on a host cell into which the vector has been introduced. Frequently used selectable marker genes include the dihydrofolate reductase (DHFR) gene (for use in dhfr- host cells with methotrexate selection/amplification) and the neo gene (for G418 selection).

Vectors encoding LINGO-4 antagonists can be used for transformation of a suitable host cell. Transformation can be by any suitable method. Methods for introduction of exogenous DNA into mammalian cells are well known in the art and include dextran-mediated transfection, calcium phosphate precipitation, polybrene-mediated transfection, protoplast fusion, electroporation, encapsulation of the polynucleotide(s) in liposomes, and direct microinjection of the DNA into nuclei. In addition, nucleic acid molecules may be introduced into mammalian cells by viral vectors.

Host cells for expression of a LINGO-4 antagonist for use in the invention may be prokaryotic or eukaryotic. Exemplary eukaryotic host cells include, but are not limited to, yeast and mammalian cells, *e.g*., Chinese hamster ovary (CHO) cells (ATCC Accession No. CCL61), NIH Swiss mouse embryo cells NIH-3T3 (ATCC Accession No. CRL1658), and baby hamster kidney cells (BHK). Other useful eukaryotic host cells include insect cells and plant cells. Exemplary prokaryotic host cells are *E. coli* and *Streptomyces*.

Transformation of host cells can be accomplished by conventional methods suited to the vector and host cell employed. For transformation of prokaryotic host cells, electroporation and salt treatment methods can be employed (Cohen et al., Proc. Natl. Acad. Sci. USA 69:2110-14 (1972)). For transformation of vertebrate cells, electroporation, cationic lipid or salt treatment methods can be employed. *See*, *e.g*., Graham et al., Virology 52:456-467 (1973); Wigler et al., Proc. Natl. Acad. Sci. USA 76:1373-76 (1979).

In certain embodiments, the host cell line used for protein expression is of mammalian origin; those skilled in the art are credited with ability to determine particular host cell lines which are best suited for the desired gene product to be expressed therein. Exemplary host cell lines include, but are not limited to NSO, SP2 cells, baby hamster kidney (BHK) cells, monkey kidney cells (COS), human hepatocellular carcinoma cells (*e.g*., Hep G2), A549 cells DG44 and DUXB11 (Chinese Hamster Ovary lines, DHFR minus), HELA (human cervical carcinoma), CVI (monkey kidney line), COS (a derivative of CVI with SV40 T antigen), R1610 (Chinese hamster fibroblast) BALBC/3T3 (mouse fibroblast), HAK (hamster kidney line), SP2/O (mouse myeloma), P3x63-Ag3.653 (mouse myeloma), BFA-1c1BPT (bovine endothelial cells), RAJI (human lymphocyte) and 293 (human kidney). Host cell lines are typically available from commercial services, the American Tissue Culture Collection or from published literature.

Expression of polypeptides from production cell lines can be enhanced using known techniques. For example, the glutamine synthetase (GS) system is commonly used for enhancing expression under certain conditions. *See*, *e.g*., European Patent Nos. 0 216 846, 0 256 055, and 0 323 997 and European Patent Application No. 89303964.4.

### Gene Therapy

A LINGO-4 antagonist can be produced *in vivo* in a mammal, *e.g.,* a human patient, using a gene-therapy approach to treatment of a nervous-system disease, disorder or injury in which promoting survival, proliferation and differentiation of oligodendrocytes or promoting myelination of neurons would be therapeutically beneficial. This involves administration of a suitable LINGO-4 antagonist-encoding nucleic acid operably linked to suitable expression control sequences. Generally, these sequences are incorporated into a viral vector. Suitable viral vectors for such gene therapy include an adenoviral vector, an alphavirus vector, an enterovirus vector, a pestivirus vector, a lentiviral vector, a baculoviral vector, a herpesvirus vector, an Epstein Barr viral vector, a papovaviral vector, a poxvirus vector, a vaccinia viral vector, adeno-associated viral vector and a herpes simplex viral vector. The viral vector can be a replication-defective viral vector. Adenoviral vectors that have a deletion in its E1 gene or E3 gene are typically used. When an adenoviral vector is used, the vector usually does not have a selectable marker gene.

### Pharmaceutical Compositions

The LINGO-4 antagonists used in the invention may be formulated into pharmaceutical compositions for administration to mammals, including humans. The pharmaceutical compositions used in this invention comprise pharmaceutically acceptable carriers, including, *e.g*., ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The compositions used in -the present invention may be administered by any suitable method, *e.g*., parenterally, intraventricularly, orally, by inhalation spray, topically, rectaliy, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. As described previously, LINGO-4 antagonists used in -the invention act in the nervous system to promote survival, proliferation and differentiation of oligodendrocytes and myelination of neurons. Accordingly, in certain methods of the disclosure, the LINGO-4 antagonists are administered in such a way that they cross the blood-brain barrier. This crossing can result from the physico-chemical properties inherent in the LINGO-4 antagonist molecule itself, from other components in a pharmaceutical formulation, or from the use of a mechanical device such as a needle, cannula or surgical instruments to breach the blood-brain barrier. Where the LINGO-4 antagonist is a molecule that does not inherently cross the blood-brain barrier, *e.g*., a fusion to a moiety that facilitates the crossing, suitable routes of administration are, *e.g*., intrathecal or intracranial, *e.g*., directly into a chronic lesion of MS. Where the LINGO-4 antagonist is a molecule that inherently crosses the blood-brain barrier, the route of administration may be by one or more of the various routes described below.

Sterile injectable forms of the compositions used in this invention may be aqueous or oleaginous suspension. These suspensions may be formulated according to techniques known in the art using suitable dispersing or wetting agents and suspending agents. The sterile, injectable preparation may also be a sterile, injectable solution or suspension in a non-toxic parenterally acceptable diluent or solvent, for example as a suspension in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Parenteral formulations may be a single bolus dose, an infusion or a loading bolus dose followed with a maintenance dose. These compositions may be administered at specific fixed or variable intervals, *e.g*., once a day, or on an "as needed" basis.

Certain pharmaceutical compositions used in this invention may be orally administered in an acceptable dosage form including, *e.g*., capsules, tablets, aqueous suspensions or solutions. Certain pharmaceutical compositions also may be administered by nasal aerosol or inhalation. Such compositions may be prepared as solutions in saline, employing benzyl alcohol or other suitable preservatives, absorption promoters to enhance bioavailability, and/or other conventional solubilizing or dispersing agents.

The amount of a LINGO-4 antagonist that may be combined with the carrier materials to produce a single dosage form will vary depending upon the host treated, the type of antagonist used and the particular mode of administration. The composition may be administered as a single dose, multiple doses or over an established period of time in an infusion. Dosage regimens also may be adjusted to provide the optimum desired response (*e.g*., a therapeutic or prophylactic response).

The invention uses a "therapeutically effective amount" or a "prophylactically effective amount" of a LINGO-4 antagonist. Such a therapeutically or prophylactically effective amount may vary according to factors such as the disease state, age, sex, and weight of the individual. A therapeutically or prophylactically effective amount is also one in which any toxic or detrimental effects are outweighed by the therapeutically beneficial effects.

A specific dosage and treatment regimen for any particular patient will depend upon a variety of factors, including the particular LINGO-4 antagonist used, the patient's age, body weight, general health, sex, and diet, and the time of administration, rate of excretion, drug combination, and the severity of the particular disease being treated. Judgment of such factors by medical caregivers is within the ordinary skill in the art. The amount will also depend on the individual patient to be treated, the route of administration, the type of formulation, the characteristics of the compound used, the severity of the disease, and the desired effect. The amount used can be determined by pharmacological and pharmacokinetic principles well known in the art.

The LINGO-4 antagonists are generally administered directly to the nervous system, intracerebroventricularly, or intrathecally, *e.g*. into a chronic lesion of MS. Compositions for administration according to the invention can be formulated so that a dosage of 0.001 - 10 mg/kg body weight per day of the LINGO-4 antagonist is administered. In some embodiments of the invention, the dosage is 0.01 - 1.0 mg/kg body weight per day. In some embodiments, the dosage is 0.001 - 0.5 mg/kg body weight per day.

For treatment with a LINGO-4 antagonist antibody, the dosage can range, *e.g*., from about 0.0001 to 100 mg/kg, and more usually 0.01 to 5 mg/kg (*e.g*., 0.02 mg/kg, 0.25 mg/kg, 0.5 mg/kg, 0.75 mg/kg, 1mg/kg, 2 mg/kg, etc.), of the host body weight. For example dosages can be 1 mg/kg body weight or 10 mg/kg body weight or within the range of 1-10 mg/kg, for example, at least 1 mg/kg. Doses intermediate in the above ranges are also intended to be within the scope of the invention. Subjects can be administered such doses daily, on alternative days, weekly or according to any other schedule determined by empirical analysis. An exemplary treatment entails administration in multiple dosages over a prolonged period, for example, of at least six months. Additional exemplary treatment regimes entail administration once per every two weeks or once a month or once every 3 to 6 months. Exemplary dosage schedules include 1-10 mg/kg or 15 mg/kg on consecutive days, 30 mg/kg on alternate days or 60 mg/kg weekly. In some methods, two or more monoclonal antibodies with different binding specificities are administered simultaneously, in which case the dosage of each antibody administered falls within the ranges indicated.

In certain embodiments, a subject can be treated with a nucleic acid molecule encoding a LINGO-4 antagonist polynucleotide. Doses for nucleic acids range from about 10 ng to 1 g, 100 ng to 100 mg, 1 µg to 10 mg, or 30-300 µg DNA per patient. Doses for infectious viral vectors vary from 10-100, or more, virions per dose.

Supplementary active compounds also can be incorporated into the compositions used in the invention. For example, a soluble LINGO-4 polypeptide or a fusion protein may be coformulated with and/or coadministered with one or more additional therapeutic agents.

Encompassed is any suitable delivery method for a LINGO-4 antagonist to a selected target tissue, including bolus injection of an aqueous solution or implantation of a controlled-release system. Use of a controlled-release implant reduces the need for repeat injections.

The LINGO-4 antagonists used in the invention may be directly infused into the brain. Various implants for direct brain infusion of compounds are known and are effective in the delivery of therapeutic compounds to human patients suffering from neurological disorders. These include chronic infusion into the brain using a pump, stereotactically implanted, temporary interstitial catheters, permanent intracranial catheter implants, and surgically implanted biodegradable implants. *See*, *e*.*g*., Gill *et al*., *supra*; Scharfen et al., "High Activity Iodine-125 Interstitial Implant For Gliomas," Int. J. Radiation Oncology Biol. Phys. 24(4):583-591 (1992); Gaspar et al., "Permanent 125I Implants for Recurrent Malignant Gliomas," Int. J. Radiation Oncology Biol. Phys. 43(5):977-982 (1999); chapter 66, pages 577-580, Bellezza et al., "Stereotactic Interstitial Brachytherapy," in Gildenberg et al., Textbook of Stereotactic and Functional Neurosurgery, McGraw-Hill (1998); and Brem et al., "The Safety of Interstitial Chemotherapy with BCNU-Loaded Polymer Followed by Radiation Therapy in the Treatment of Newly Diagnosed Malignant Gliomas: Phase I Trial," J. Neuro-Oncology 26:111-23 (1995).

The compositions may also comprise a LINGO-4 antagonist dispersed in a biocompatible carrier material that functions as a suitable delivery or support system for the compounds. Suitable examples of sustained release carriers include semipermeable polymer matrices in the form of shaped articles such as suppositories or capsules. Implantable or microcapsular sustained release matrices include polylactides (U.S. Patent No. 3,773,319; EP 58,481), copolymers of L-glutamic acid and gamma-ethyl-L-glutamate (Sidman et al., Biopolymers 22:547-56 (1985)); poly(2-hydroxyethyl-methacrylate), ethylene vinyl acetate (Langer et al., J Biomed. Mater. Res. 15:167-277 (1981); Langer, Chem. Tech. 12:98-105 (1982)) or poly-D-(-)-3hydroxybutyric acid (EP 133,988).

In some instances, a LINGO-4 antagonist is administered to a patient by direct infusion into an appropriate region of the brain. *See*, *e.g*., Gill et al., "Direct brain infusion of glial cell line-derived neurotrophic factor in Parkinson disease," Nature Med. 9: 589-95 (2003). Alternative techniques are available and may be applied to administer a LINGO-4 antagonist according to the invention. For example, stereotactic placement of a catheter or implant can be accomplished using the Riechert-Mundinger unit and the ZD (Zamorano-Dujovny) multipurpose localizing unit. A contrast-enhanced computerized tomography (CT) scan, injecting 120 ml of omnipaque, 350 mg iodine/ml, with 2 mm slice thickness can allow three-dimensional multiplanar treatment planning (STP, Fischer, Freiburg, Germany). This equipment permits planning on the basis of magnetic resonance imaging studies, merging the CT and MRI target information for clear target confirmation.

The Leksell stereotactic system (Downs Surgical, Inc., Decatur, GA) modified for use with a GE CT scanner (General Electric Company, Milwaukee, WI) as well as the Brown-Roberts-Wells (BRW) stereotactic system (Radionics, Burlington, MA) can be used for this purpose. Thus, on the morning of the implant, the annular base ring of the BRW stereotactic frame can be attached to the patient's skull. Serial CT sections can be obtained at 3 mm intervals though the (target tissue) region with a graphite rod localizer frame clamped to the base plate. A computerized treatment planning program can be run on a VAX 11/780 computer (Digital Equipment Corporation, Maynard, Mass.) using CT coordinates of the graphite rod images to map between CT space and BRW space.

The methods of treatment of demyelination or dysmyelination disorders as described herein are typically tested *in vitro*, and then *in vivo* in an acceptable animal model, for the desired therapeutic or prophylactic activity, prior to use in humans. Suitable animal models, including transgenic animals, are will known to those of ordinary skill in the art. For example, *in vitro* assays to demonstrate the differentiation and survival effect of the LINGO-4 antagonists are described herein. The effect of the LINGO-4 antagonists on myelination of axons can be tested *in vitro* as described in the Examples. Finally, *in vivo* tests can be performed by creating transgenic mice which express the LINGO-4 antagonist or by administering the LINGO-4 antagonist to mice or rats in models as described herein.

### Examples

### Example 1

### LINGO-4 is Highly Expressed in the Central Nervous System

Oligodendrocytes mature through several developmental stages from A2B5 progenitor cells (which express A2B5), differentiating into pre-myelinating oligodendrocytes (which express O1 and 04) and finally into mature myelinating oligodendrocytes (which express O1, 04 and MBP). Thus, by monitoring the presence and absence of the A2B5, O1, 04 and MBP markers it is possible to determine a given cell's developmental stage and to evaluate the role of LINGO-4-Fc in oligodendrocyte biology. For a general review of oligodendrocyte biology, see, *e.g*., Baumann and Pham-Dinh, Physiol. Rev. 81: 871-927 (2001).

Expression of LINGO-4 in mouse tissues was evaluated by quantitative PCR (Q-PCR) assay by the following method. Tissue mRNA from a Mouse Total RNA Master Panel (Clonetech) was subjected to Taqman RT-PCR (performed as described in Mi et al., Nature Neuroscience 7: 221-228 (2004)) to quantify LINGO-4 mRNA levels, using forward primer 5'-GAGCCTGGTTGGCCTCAA-3' (SEQ ID NO:21), reverse primer 5'-GCAGTGCTTGGAAGGGTACT-3' (SEQ ID NO:22) and FAB-labeled probe 5'-CAGCCTGGCTATCACC-3' (SEQ ID NO:23). The primers and FAB-labeled probe were designed using Primer Express v1.0 (Applied Biosystems).

Relative LINGO-4 expression levels were determined by first normalizing LINGO-4 mRNA levels in the tissues to actin mRNA levels in the same tissues. Then, relative LINGO-4 mRNA levels were determined by comparing normalized LINGO-4 mRNA levels in each tissue to the normalized expression level in the eye, which was assigned a value of 1. The relative LINGO-4 expression levels are shown in Figure 1. LINGO-4 was expressed to the greatest extent in brain and spinal cord. Expression levels in heart, uterus, spleen, stomach, kidney, eye, salivary gland, liver, and lymph node were detectable, but were less relative to brain and spinal cord.

### Example 2

### Human LINGO-4 is Homologous to Human LINGO-1

The amino acid sequences of the four human LINGO paralogs were aligned and compared. *See* Fig. 2. The similarity and identity percentages between LINGO-1 and the various other human paralogs is shown below in Table 2. While hLINGO-1 and hLINGO-2 are the most closely related, hLINGO-1 and hLINGO-4 share significant similarity and identity.

**Table 2: Comparison of the human LINGO-1 amino acid sequence to other LINGO paralogs**

| hLINGO-1 vs | Percent Similarity | Percent Identity |
|---|---|---|
| hLINGO-2 | 70.4% | 60.7% |
| hLINGO-3 | 66.4% | 55.4% |
| hLINGO-4 | 52.1% | 44.3% |

### Example 3

### LINGO-4 is Specifically Expressed in the Spinal Cord

Various mouse tissues were examined for the expression of LINGO-4 in comparison with LINGO-1, LINGO-2 and LINGO-3 by quantitative PCR (Q-PCR), using the methods as described in Example 1. LINGO-1, LINGO-2, LINGO-3 and LINGO-4 mRNA levels were first normalized to actin mRNA. In these experiments, relative expression of LINGO-1, LINGO-2, LINGO-3 and LINGO-4 mRNA were determined by comparing to the mRNA level in Mouse Universal Reference Total RNA (Clonetech), which was assigned a value of 1.

LINGO expression levels were assayed using the following primer pairs:
LINGO-4: same primers as described in Example 1.
LINGO-1: 5' PCR Primer - 5' - CTTTCCCCTTCGACATCAAGAC- 3' (SEQ ID NO:24);
   3' PCR Primer - 5' - CAGCAGCACCAGGCAGAA- 3' (SEQ ID NO:25); and
   FAM-labeled probe 5'-ATCGCCACCACCATGGGCTTCAT-3' (SEQ ID NO:26).
LINGO-2: 5' PCR Primer - 5' - ACCTTGTATACCTGACCCACCTTAA- 3' (SEQ ID NO:27);
   3' PCR Primer - 5' - AGAGAACATGCCAGCTTCAATAGTG- 3' (SEQ ID NO:28); and
   FAM-labeled probe 5'-CCTCTCCTACAATCCC-3' (SEQ ID NO:29).
LINGO-3: 5' PCR Primer - 5' - CGCGGCTCCTTCAGAGA- 3' (SEQ ID NO:30);
   3' PCR Primer - 5' - GGCTCCTGCTAGGTGCA- 3' (SEQ ID NO:31); and
   FAM-labeled probe 5'-CTGGTGCGCCTGCGTG-3' (SEQ ID NO:32).

Of the 11 tissue types tested, LINGO-4 showed highest level of expression in the spinal cord in adult and P6 mouse tissues. Results of the relative expression of LINGO-1, LINGO-2, LINGO-3, and LINGO-4 are shown in Fig. 3 (adult tissues) and Fig. 4 (P6 tissues).

### Example 4

### Preparation of LINGO-4 Expression Constructs

### Construction of LINGO-4 FL and DN Lentivirus Vectors

We constructed lentiviral vectors that express wild-type and a dominant-negative form of LINGO-4 generally according to the methods described in Mi et al. Nat Neurosci. 8:745-51 (2005). Briefly, DNA sequence encoding human full length LINGO-4 (FL-LINGO-4), amino acid residues 1-593 ( of SEQ ID NO:2) was amplified from human brain cDNA (Clontech) by PCR using the following primers:
5' PCR Primer:
   5'- TTTTTGCGGCCGCCACCATGGATGCAGCCACAGCTCCAAAGCAAGCC-3' (SEQ ID NO:33)
3' PCR Primer:
   5'- TTTTTGCGGCCGCTCAGAAGAGCTTGGCAGTGACCCGGTTACCCCCAG-3' (SEQ ID NO:34).

The FL-LINGO-4 PCR product was cloned into pCR4bluntTOPO vector (Invitrogen). The resulting FL-LINGO-4 clone was designated pJST1011 and the DNA sequence of the insert and flanking vector confirmed.

The FL-LINGO-4 clone pJST1011 was subjected to site directed mutagenesis in order to insert an HA epitope tag after the predicted signal sequence (amino acid 1- 29) and prior to the predicted extracellular domain of LINGO-4 (amino acids 30-535) using the forward PCR primer-5'-CTCCTCCTACCTGGAGGGAGCGGTGGCTACCCTTACGACGTCCCTGATTACGCTAGC TGCCCTGCTGTGTGTGACTGCACCTCCCAGC-3' (SEQ ID NO:35) and the reverse PCR primer-5'-GCTGGGAGGTGCAGTCACACACAGCAGGGCAGCTAGCGTAATCAGGGACGTCGTA AGGGTAGCCACCGCTCCCTCCAGGTAGGAGGAG-3' (SEQ ID NO:36). The resultant plasmid encoding HA-FL-LINGO-4 was designated pJST1037 and its DNA sequence confirmed. The HA-FL-LINGO-4 coding sequence of pJST1037 was isolated as a NotI fragment and sub-cloned to similarly digested lentiviral vector HRST-IRESeGFP to yield pJST1040.

A gene encoding HA tagged dominant negative LINGO-4 protein, HA-DN-LINGO-4, encoding amino acids 1-571 of HA tagged full length LINGO-4 (HA-FL-LINGO-4) was amplified by PCR from pJST1037 using the following primers:
5' PCR Primer - 5' - AGGAAACAGCTATGACCATG - 3' (SEQ ID NO:37); and
3' PCR Primer -
   5' - TTTTTGCGGCCGCTCAACCTTTGCCCTTGCTCCAAAGGGCAATCAGG- 3' (SEQ ID NO:38).

The resultant PCR fragment was digested with NotI and cloned into the similarly digested lentiviral vector HRST-IRESeGFP. The resultant plasmid was designated pJST1043 and the DNA sequence of the HA-DN-LINGO-4 insert and flanking vector sequence was confirmed.

The lentiviral vectors encoding HA-FL-LINGO-4 (pJST1040) and HA-DN-LINGO-4 (pJST1043) were transfected into 293 cells to produce lentivirus as described by Rubinson et al., "A lentivirus-based system to functionally silence genes in primary mammalian cells, stem cells and transgenic mice by RNA interference," Nat. Genet. 33: 401-06 (2003).

### Construction and purification of LINGO-4-Fc fusion protein

A construct was made fusing the extra-cellular portion of human LINGO-4 (residues 1-535) to the hinge and Fc region of human IgG1 to study the biological function of LINGO-4. A partial coding sequence for human LINGO-4 extracellular domain was obtained by PCR using the human full length LINGO-4 clone pJST1011 as a template with the forward primer 5' - AGGAAACAGCTATGACCATG- 3' (SEQ ID NO:39) and reverse primer 5' - AAAAAGGTCGACCATGGCCACACCTCTGCTATCCAG - 3' (SEQ ID NO:40).

The PCR product encoding the LINGO-4 extracellular domain was digested with NotI (5') and SalI (3') and cloned with the SalI (5') to BamHI (3') DNA cassette encoding IgG1 hinge and Fc into the vector pNE001 (Biogen Idec) to yield pJST1064. The DNA sequence of the insert in pJST1064 was determined and confirmed to encode LINGO-4 signal sequence and extracellular domain (amino acids 1 to 535) in-frame with the hinge and Fc region of human IgG1. The pJST1064 *Not*I fragment encompassing the LINGO-4-Fc fragment was subcloned into the single *Not*I cloning site of the CHO expression vector, PV90 (Biogen Idec). The resulting plasmid was confirmed by DNA sequencing and designated pJST1084.

Stable cell lines expressing the LINGO-4-Fc fusion protein were generated by electroporation of CHO host cells DG44 with plasmid pJST1084. Transfected CHO cells were cultured in alpha minus MEM in the presence of 10% dialyzed serum and 4 mM glutamine to select for nucleoside-independent growth. Fourteen days post-transfection, cells were fed fresh media. To screen for cells expressing LINGO-4-Fc, CHO cells were labeled with phycoerythrin (PE)-labeled goat anti-human IgG (Jackson Labs) and subjected to high speed flow cytometry sorting in a FACS Mo-Flo (Cytomation). The cells that expressed the highest levels of LINGO-4-Fc were selected. These cells were expanded in culture for 7 days, then re-labeled and resorted. Cells expressing the highest levels of LINGO-4-Fc were isolated as individual clones in 96-well plates. These clones were grown for two weeks and then fed fresh media one day prior to FACS analysis to check for expression levels. Clones that expressed the highest levels of LINGO-4-Fc were expanded, and frozen cell banks were established. The cell lines were adapted to grow in suspension culture in the serum-free media BCM16. The titer of LINGO-4-Fc produced by these clones was determined by growing cell lines at 37°C for 4-5 passages, then growing the cells to 50% maximal cell density and culturing them for 10-15 days at 28°C until the viable cell density dropped to 75%. At this time, the culture media was harvested, cleared of cells and debris by centrifugation, and the culture supernatants were titered for LINGO-4-Fc levels by Western blot analysis using an anti-human Ig antibody (Jackson Lab) as the probe.

LINGO-4-Fc fusion protein was purified from the clarified culture medium as follows: 9 ml of 1M HEPES pH 7.5 was added to 900 ml of conditioned medium. The medium was batch loaded for 3 hr at 4°C onto 3 ml of Protein A Sepharose (Amersham Bioscience). The resin was collected in a 1.5 cm (I.D.) column, and washed four times with 3 ml PBS, two times with 4 ml of PBS containing 800 mM NaCl, and then again with 3 ml of PBS. The LINGO-4-Fc was eluted from the column with 25 mM NaH₂PO₄, pH 2.8 and 100 mM NaCl in 1.5 ml fractions and neutralized by adding 75 µl of 0.5 M NaH₂PO₄, pH 8.6. Peak protein-containing fractions were identified by absorbance at 280 nm, pooled, and subjected to further purification on a 1 mL Protein A column. Prior to loading, NaCl was added to 600 mM and HEPES, pH 7.5 to 50 mM. The column was washed twice with 600 µl of 10 mM HEPES pH 7.5 and 1 M NaCl , and then with 1 ml PBS. LINGO-4-Fc was eluted from the column with 25 mM NaH₂PO₄, pH 2.8 and 100 mM NaCl, collecting 0.5 mL fractions, and neutralized by adding 25 µl of 0.5 M NaH₂PO₄, pH 8.6. Peak protein-containing fractions were identified by absorbance at 280 nm and pooled. The purified LINGO-4-Fc protein was aliquoted and stored at -70°C.

### Example 5

### Dominant-negative LINGO-4 Promotes Oligodendrocyte Differentiation

Enriched populations of oligodendrocytes were grown from female Long Evans P2 rats as described by Conn, Meth. Neurosci. 2:1-4 (Academic Press; 1990) with modifications as follows. Briefly, the forebrain was dissected and placed in Hank's buffered salt solution (HBSS; Invitrogen). The tissue was cut into 1-mm fragments and was incubated at 37°C for 15 min in 0.01 % trypsin and 10 µg/ml DNase. Dissociated cells were plated on poly-L-lysine-coated T75 tissue culture flasks and were grown at 37°C for 10 d in DMEM medium with 20% fetal bovine serum (Invitrogen). Oligodendrocyte precursors (A2B5+) were collected by shaking the flask overnight at 200 rpm at 37°C, resulting in a 95% pure population. Cultures were maintained in high-glucose Dulbecco's modified Eagle's medium (DMEM) with FGF/PDGF (10 ng/ml; Peprotech) for 1 week. Removal of FGF/PDGF allowed A2B5+ cells to differentiate into O4+ premyelinating oligodendrocytes after 3-7 d, and to differentiate into O4+ and MBP+ mature oligodendrocytes after 7-10 d. Oligodendrocytes were infected with lentivirus at 2 MOI per cell and overexpression of FL-LINGO-4 and DN-LINGO-4 was confirmed by western blot (data not shown).

The differentiation of A2B5 oligodendrocytes was measured by western blot, using an antibody to the oligodendrocyte differentiation marker myelin basic protein (MBP). Uninfected oligodendrocytes treated with anti-LINGO-1 monoclonal antibody 1A7, which has been described in International PCT Publication WO 2007/008547, U.S. Published Application No. 2006/0009388 and Mi et al., Nature Medicine 13, 1228-1233(2007). Irrelevant Mouse IgG MOPC21 (available from Protos Immunoresearch (San Francisco, CA)) were used as positive and negative controls, respectively. The LINGO-4 lentiviruses expressed an HA tag, which was used as an expression level control for the lentivirus-infected cells. As shown in Fig. 5, overexpression of DN-LINGO-4 promoted oligodendrocyte differentiation, as indicated by an increase in expression of myelin basic protein (MBP). In contrast, overexpression of full-length LINGO-4 had the opposite effect and inhibited differentiation, as was evident by a reduction in MBP expression. This study indicates that expression of a dominant negative LINGO-4 protein in oligodendrocytes promotes oligodendrocyte differentiation.

In a similar experiment, uninfected A2B5 oligodendrocytes were treated with hLINGO-4 Fc protein. Oligodendrocyte differentiation was measured by western blot, using an antibody to the oligodendrocyte differentiation marker myelin basic protein (MBP) as well as an antibody to another oligodendrocyte differentiation marker myelin-oligodendrocyte glycoprotein (MOG). LINGO-4 FL and LINGO-4 DN lentivirus-infected oligodendrocytes, as described above, as well as LINGO-1 FL and LINGO-1 DN lentivirus-infected oligodendrocytes (described in U.S. Published Application No. 2007/0059793) were used as controls. As shown in Fig. 6, treatment with h LINGO-4 Fc, as well as overexpression of LINGO-1 DN or LINGO-4 DN, promoted oligodendrocyte differentiation, as indicated by an increase in expression of MBP and MOG. In contrast, overexpression of full-length LINGO-1 or full-length LINGO-4 had the opposite effect and inhibited differentiation, as was evident by a lack of MBP or MOG expression. This study indicates that treatment with exogenous LINGO-4-Fc protein promotes oligodendrocyte differentiation.

### Example 6

### LINGO-4-Fc promotes Oligodendrocyte Myelination in Co-culture

The role of LINGO-4 in myelination was examined *in vitro* by infecting co-cultures of dorsal root ganglion (DRG) neurons and oligodendrocytes with LINGO-4 FL and LINGO-4 DN and testing for myelination by western blot analysis. For these studies, it was necessary to first generate primary cultures of DRG neurons and of oligodendrocytes.

Female Long Evans rat E14-E17 embryonic dorsal root ganglia were cultured as described by Plant et al., J. Neurosci. 22:6083-91 (2002). Dissected DRGs were plated on poly-L-lysine-coated cover slips (100 µg/ml) for 2 weeks in the presence of fluorodeoxyuridine for days 2-6 and days 8-11 in NLA medium containing 1 x B27, 100 ng/ml NGF (Invitrogen).

A2B5⁺ oligodendrocytes were prepared as described in Mi et al., Nature Neuroscience 7: 221-228 (2004), and were harvested by trypsinization.

For coculture studies, A2B5⁺ oligodendrocytes infected with LINGO-4 FL or LINGO-4 DN lentiviruses prepared as described in Example 4 were added to DRG neuron drop cultures. Control cocultures were also prepared in the presence of 1A7 or MOPC21 antibodies as positive and negative controls, respectively. The culture medium (Neurobasal medium supplemented with B27 and 100 ng/ml NGF) was changed every 3 d for two weeks. Western blot analysis demonstrated that expression of MBP, the major protein component of myelin, was increased in LINGO-4-Fc-treated cultures (Fig. 7). Expression of DN-LINGO-4 or treatment with 1A7 resulted in DRG myelination, as demonstrated by expression of MBP. In contrast, overexpression of FL-LINGO-4 blocked expression of MBP. Expression of FL-LINGO-4 and DN-LINGO-4 proteins in cultures was confirmed by western blotting (data not shown). These studies further indicate that expression of FL LINGO-4 inhibits myelination and that expression of DN LINGO-4 can reverse the inhibition.

### Example 7

### LINGO-4-Fc promotes oligodendrocyte survival and myelination in vivo

Adult wild-type C57B1/6 male mice are fed cuprizone (0.2% milled with ground mouse chow by weight) for 6 weeks to induce demyelination within the corpus callosum. LINGO-4-Fc is stereotactically injected into the demyelinating corpus callosum at 2, 2.5, and 3 weeks of cuprizone feeding. Control mice are stereotactically injected at the same intervals with sterilized media containing no LINGO-4-Fc. After 6 weeks of cuprizone feeding, the mice are returned to a normal diet for 2, 4 and 6 weeks (ground mouse chow only) to allow remyelination.

The cuprizone-treated mice are anesthetized with ketamine (80 mg/kg body weight) and xylazine (10 mg/kg body weight) and positioned in an immobilization apparatus designed for stereotactic surgery (David Kopf Instruments). The scalp is opened and the sterile compounds injected (1 µM in 1 ml of HBSS) unilaterally into the acutely demyelinated corpus callosum of the wild-type recipient mice with a 10 ml Hamilton syringe using stereotactic coordinates of 0.7 mm posterior and 0.3 mm lateral to bregma at a depth of 1.7 mm (Messier et al., Pharmacol. Biochem. Behav. 63(2): 313-18 (1999)). Additionally, control recipient mice are stereotactically injected with HBSS containing no compounds. The opening in the skull is filled with Gelfoam, and the area is swabbed with penicillin and streptomycin (Gibco) and the wound will be sutured. Post injection, mice are sacrificed every week of the experiment and their brains are removed and processed for molecular, biochemical and histological analysis.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the scope of the appended claims.

## Claims

1. A composition comprising a LINGO-4 antagonist for use in the treatment of a disease, disorder, or injury involving demyelination or dysmyelination selected from the group consisting of multiple sclerosis (MS), progressive multifocal leukoencephalopathy (PML), encephalomyelitis (EPL), central pontine myelinolysis (CPM), adrenoleukodystrophy, Alexander's disease, Pelizaeus Merzbacher disease (PMZ), Wallerian Degeneration, optic neuritis, transverse myelitis, amylotrophic lateral sclerosis (ALS), Huntington's disease, Alzheimer's disease, Parkinson's disease, spinal cord injury, traumatic brain injury, post radiation injury, neurologic complications of chemotherapy, stroke, acute ischemic optic neuropathy, isolated vitamin E deficiency syndrome, Bassen-Kornzweig syndrome, Marchiafava-Bignami syndrome, metachromatic leukodystrophy, trigeminal neuralgia, and Bell's palsy, wherein the LINGO-4 antagonist is selected from the group consisting of:
(i) a soluble LINGO-4 polypeptide, optionally fused to a non-LINGO-4 moiety;
(ii) a LINGO-4 antibody or an antigen-binding fragment thereof;
(iii) a LINGO-4 antagonist polynucleotide selected from the group consisting of
(a) an antisense polynucleotide;
(b) a ribozyme;
(c) a small interfering RNA (siRNA); and
(d) a small-hairpin RNA (shRNA);
(iv) a LINGO-4 aptamer; and
(v) a combination of two or more of said LINGO-4 antagonists;
wherein the non-LINGO-4 moiety is selected from the group consisting of immunoglobulin, serum albumin, a targeting polypeptide, a reporter polypeptide, a purification-facilitating polypeptide, polyalkylene glycol, a sugar polymer, and a combination of two or more of said non-LINGO-4 moieties.

2. The composition for use according to claim 1, wherein said soluble LINGO-4 polypeptide comprises a LINGO-4 region selected from the group consisting of:
(i) a LINGO-4 Ig domain,
(ii) a LINGO-4 LRR domain, and
(iii) a combination of said LINGO-4 domains.

3. The composition for use according to claim 1 or claim 2, wherein said soluble LINGO-4 polypeptide lacks a LINGO-4 region selected from the group consisting of:
(i) a LINGO-4 transmembrane domain,
(ii) a LINGO-4 intracellular domain, and
(iii) a combination of said LINGO-4 domains.

4. The composition for use according to any one of claims 1-3, wherein said LINGO-4 polypeptide comprises an amino acid sequence at least 90% identical or at least 95% identical to a reference amino acid sequence selected from the group consisting of: amino acids 30 to 411 of SEQ ID NO:2; amino acids 30 to 491 of SEQ ID NO:2; and amino acids 30 to 534 of SEQ ID NO:2.

5. The composition for use according to any one of claims 1-3, wherein said LINGO-4 polypeptide comprises an amino acid sequence identical to a reference amino acid sequence selected from the group consisting of: amino acids 30 to 64 of SEQ ID NO:2; amino acids 30 to 82 of SEQ ID NO:2; amino acids 30 to 106 of SEQ ID NO:2; amino acids 30 to 130 of SEQ ID NO:2; amino acids 30 to 154 of SEQ ID NO:2; amino acids 30 to 178 of SEQ ID NO:2; amino acids 30 to 202 of SEQ ID NO:2; amino acids 30 to 226 of SEQ ID NO:2; amino acids 30 to 298 of SEQ ID NO:2; amino acids 30 to 322 of SEQ ID NO:2; amino acids 30 to 346 of SEQ ID NO:2; amino acids 30 to 411 of SEQ ID NO:2; amino acids 30 to 491 of SEQ ID NO:2; amino acids 30 to 534 of SEQ ID NO:2; amino acids amino acids 63 to 82 of SEQ ID NO:2; amino acids 63 to 106 of SEQ ID NO:2; amino acids 63 to 130 of SEQ ID NO:2; amino acids 63 to 154 of SEQ ID NO:2; amino acids 63 to 178 of SEQ ID NO:2; amino acids 63 to 202 of SEQ ID NO:2; amino acids 63 to 226 of SEQ ID NO:2; amino acids 63 to 298 of SEQ ID NO:2; amino acids 63 to 322 of SEQ ID NO:2; amino acids 63 to 346 of SEQ ID NO:2; amino acids 63 to 411 of SEQ ID NO:2; amino acids 63 to 491 of SEQ ID NO:2; amino acids 63 to 534 of SEQ ID NO:2; amino acids 83 to 106 of SEQ ID NO:2; amino acids 83 to 130 of SEQ ID NO:2; amino acids 83 to 154 of SEQ ID NO:2; amino acids 83 to 178 of SEQ ID NO:2; amino acids 83 to 202 of SEQ ID NO:2; amino acids 83 to 226 of SEQ ID NO:2; amino acids 83 to 298 of SEQ ID NO:2; amino acids 83 to 322 of SEQ ID NO:2; amino acids 83 to 346 of SEQ ID NO:2; amino acids 83 to 411 of SEQ ID NO:2; amino acids 83 to 491 of SEQ ID NO:2; amino acids 83 to 534 of SEQ ID NO:2; amino acids 107 to 130 of SEQ ID NO:2; amino acids 107 to 154 of SEQ ID NO:2; amino acids 107 to 178 of SEQ ID NO:2; amino acids 107 to 202 of SEQ ID NO:2; amino acids 107 to 226 of SEQ ID NO:2; amino acids 107 to 298 of SEQ ID NO:2; amino acids 107 to 322 of SEQ ID NO:2; amino acids 107 to 346 of SEQ ID NO:2; amino acids 107 to 411 of SEQ ID NO:2; amino acids 107 to 491 of SEQ ID NO:2; amino acids 107 to 534 of SEQ ID NO:2; amino acids 131 to 154 of SEQ ID NO:2; amino acids 131 to 178 of SEQ ID NO:2; amino acids 131 to 202 of SEQ ID NO:2; amino acids 131 to 226 of SEQ ID NO:2; amino acids 131 to 298 of SEQ ID NO:2; amino acids 131 to 322 of SEQ ID NO:2; amino acids 131 to 346 of SEQ ID NO:2; amino acids 131 to 411 of SEQ ID NO:2; amino acids 131 to 491 of SEQ ID NO:2; amino acids 131 to 534 of SEQ ID NO:2; amino acids 155 to 178 of SEQ ID NO:2; amino acids 155 to 202 of SEQ ID NO:2; amino acids 155 to 226 of SEQ ID NO:2; amino acids 155 to 298 of SEQ ID NO:2; amino acids 155 to 322 of SEQ ID NO:2; amino acids 155 to 346 of SEQ ID NO:2; amino acids 155 to 411 of SEQ ID NO:2; amino acids 155 to 491 of SEQ ID NO:2; amino acids 155 to 534 of SEQ ID NO:2; amino acids 179 to 202 of SEQ ID NO:2; amino acids 179 to 226 of SEQ ID NO:2; amino acids 179 to 298 of SEQ ID NO:2; amino acids 179 to 322 of SEQ ID NO:2; amino acids 179 to 346 of SEQ ID NO:2; amino acids 179 to 411 of SEQ ID NO:2; amino acids 179 to 491 of SEQ ID NO:2; amino acids 179 to 534 of SEQ ID NO:2; amino acids 203 to 226 of SEQ ID NO:2; amino acids 203 to 298 of SEQ ID NO:2; amino acids 203 to 322 of SEQ ID NO:2; amino acids 203 to 346 of SEQ ID NO:2; amino acids 203 to 411 of SEQ ID NO:2; amino acids 203 to 491 of SEQ ID NO:2; amino acids 203 to 534 of SEQ ID NO:2; amino acids 275 to 298 of SEQ ID NO:2; amino acids 275 to 322 of SEQ ID NO:2; amino acids 275 to 346 of SEQ ID NO:2; amino acids 275 to 411 of SEQ ID NO:2; amino acids 275 to 491 of SEQ ID NO:2; amino acids 275 to 534 of SEQ ID NO:2; amino acids 299 to 322 of SEQ ID NO:2; amino acids 299 to 346 of SEQ ID NO:2; amino acids 299 to 411 of SEQ ID NO:2; amino acids 299 to 491 of SEQ ID NO:2; amino acids 299 to 534 of SEQ ID NO:2; amino acids 323 to 346 of SEQ ID NO:2; amino acids 323 to 411 of SEQ ID NO:2; amino acids 323 to 491 of SEQ ID NO:2; amino acids 323 to 534 of SEQ ID NO:2; amino acids 358 to 411 of SEQ ID NO:2; amino acids 358 to 491 of SEQ ID NO:2; amino acids 358 to 534 of SEQ ID NO:2; amino acids 426 to 491 of SEQ ID NO:2; amino acids 426 to 534 of SEQ ID NO:2; and a combination of at least two of any of said polypeptides.

6. The composition for use according to any one of claims 1 to 5, wherein said soluble LINGO-4 polypeptide is a cyclic peptide.

7. The composition for use according to claim 6, wherein said cyclic polypeptide further comprises a biotin molecule or an acetylated cysteine linked to the N-terminus and a cysteine molecule linked to the C-terminus; wherein said biotin molecule or said acetylated cysteine molecule and said cysteine molecule are joined to each other to form said cyclic molecule.

8. A polynucleotide that encodes the soluble LINGO-4 polypeptide, the LINGO-4 antibody or fragment thereof, or the LINGO-4 aptamer defined in claim 1, for use in the treatment of a disease, disorder, or injury involving demyelination or dysmyelination selected from the group consisting of multiple sclerosis (MS), progressive multifocal leukoencephalopathy (PML), encephalomyelitis (EPL), central pontine myelinolysis (CPM), adrenoleukodystrophy, Alexander's disease, Pelizaeus Merzbacher disease (PMZ), Wallerian Degeneration, optic neuritis, transverse myelitis, amylotrophic lateral sclerosis (ALS), Huntington's disease, Alzheimer's disease, Parkinson's disease, spinal cord injury, traumatic brain injury, post radiation injury, neurologic complications of chemotherapy, stroke, acute ischemic optic neuropathy, isolated vitamin E deficiency syndrome, Bassen-Kornzweig syndrome, Marchiafava-Bignami syndrome, metachromatic leukodystrophy, trigeminal neuralgia, and Bell's palsy, where the polynucleotide is operably linked to an expression control sequence, allowing expression of said LINGO-4 antagonist.

9. An expression vector comprising the polynucleotide defined in claim 8, for use in the treatment of a disease, disorder, or injury involving demyelination or dysmyelination selected from the group consisting of multiple sclerosis (MS), progressive multifocal leukoencephalopathy (PML), encephalomyelitis (EPL), central pontine myelinolysis (CPM), adrenoleukodystrophy, Alexander's disease, Pelizaeus Merzbacher disease (PMZ), Wallerian Degeneration, optic neuritis, transverse myelitis, amylotrophic lateral sclerosis (ALS), Huntington's disease, Alzheimer's disease, Parkinson's disease, spinal cord injury, traumatic brain injury, post radiation injury, neurologic complications of chemotherapy, stroke, acute ischemic optic neuropathy, isolated vitamin E deficiency syndrome, Bassen-Kornzweig syndrome, Marchiafava-Bignami syndrome, metachromatic leukodystrophy, trigeminal neuralgia, and Bell's palsy, where the polynucleotide is operably linked to an expression control sequence.

10. The expression vector for use according to claim 9, wherein the expression vector is a viral vector selected from the group consisting of an adenoviral vector, an alphavirus vector, an enterovirus vector, a pestivirus vector, a lentivirus vector, a baculovirus vector, a herpesvirus vector, a papovavirus vector, and a poxvirus vector.

11. The composition for use according to any one of claims 1 to 7, wherein the composition is formulated for administration by bolus injection or chronic infusion, optionally directly into the central nervous system.

12. An *in vitro* method of promoting differentiation or survival of oligodendrocytes comprising contacting oligodendrocytes with a composition comprising a LINGO-4 antagonist selected from the group consisting of:
(i) a soluble LINGO-4 polypeptide, optionally fused to a non-LINGO-4 moiety;
(ii) a LINGO-4 antibody or an antigen-binding fragment thereof;
(iii) a LINGO-4 antagonist polynucleotide selected from the group consisting of
(a) an antisense polynucleotide;
(b) a ribozyme;
(c) a small interfering RNA (siRNA); and
(d) a small-hairpin RNA (shRNA);
(iv) a LINGO-4 aptamer; and
(v) a combination of two or more of said LINGO-4 antagonists;
wherein the non-LINGO-4 moiety is selected from the group consisting of immunoglobulin, serum albumin, a targeting polypeptide, a reporter polypeptide, a purification-facilitating polypeptide, polyalkylene glycol, a sugar polymer, and a combination of two or more of said non-LINGO-4 moieties.

13. An *in vitro* method of promoting myelination of neurons or of preventing demyelination of neurons comprising contacting a mixture of neurons and oligodendrocytes with a composition comprising a LINGO-4 antagonist selected from the group consisting of:
(i) a soluble LINGO-4 polypeptide, optionally fused to a non-LINGO-4 moiety;
(ii) a LINGO-4 antibody or an antigen-binding fragment thereof;
(iii) a LINGO-4 antagonist polynucleotide selected from the group consisting of
(a) an antisense polynucleotide;
(b) a ribozyme;
(c) a small interfering RNA (siRNA); and
(d) a small-hairpin RNA (shRNA);
(iv) a LINGO-4 aptamer; and
(v) a combination of two or more of said LINGO-4 antagonists;
wherein the non-LINGO-4 moiety is selected from the group consisting of immunoglobulin, serum albumin, a targeting polypeptide, a reporter polypeptide, a purification-facilitating polypeptide, polyalkylene glycol, a sugar polymer, and a combination of two or more of said non-LINGO-4 moieties.

## Patentansprüche

1. Zusammensetzung, die einen LINGO-4 Antagonisten zur Verwendung bei der Behandlung einer Krankheit, Störung oder Verletzung umfasst, die eine Demyelination oder Dysmyelination beinhaltet, ausgewählt aus der Gruppe bestehend aus multipler Sklerose (MS), progressiver multifokaler Leukenzephalopathie (PML), Enzephalomyelitis (EPL), zentraler pontiner Myelinolyse (CPM), Adrenoleukodystrophie, Alexander-Krankheit, Pelizaeus-Merzbacher-Krankheit, Wallerscher Degeneration, optischer Neuritis, transversaler Myelitis, amylotropher Lateralsklerose (ALS), Huntington-Krankheit, Alzheimer-Krankheit, Parkinson-Krankheit, Rückenmarksverletzung, traumatischer Hirnverletzung, Verletzung nach Bestrahlung, neurologischen Komplikationen von Chemotherapie, Schlaganfall, akuter ischämischer optischer Neuropathie, isoliertem Vitamin-E-Mangelsyndrom, Bassen-Kornzweig-Syndrom, Marchiafava-Bignami-Syndrom, metachromatischer Leukodystrophie, Trigeminus-Neuralgie und Bellscher Parese, wobei der LINGO-4 Antagonist ausgewählt ist aus der Gruppe bestehend aus:
(i) einem löslichen LINGO-4-Polypeptid, optional an einen Non-LINGO-4-Anteil fusioniert;
(ii) einem LINGO-4-Antikörper oder einem Antigen-Bindungsfragment davon;
(iii) einem LINGO-4-Antagonisten-Polynukleotid, ausgewählt aus der Gruppe bestehend aus
(a) einem Antisense-Polynukleotid;
(b) einem Ribozym;
(c) einer kleinen eingreifenden RNA (siRNA); und
(d) einer kleinen Haarnadel-RNA (shRNA);
(iv) einem LINGO-4-Aptamer; und
(v) einer Kombination aus zwei oder mehr der genannten LINGO-4 Antagonisten;
wobei der Non-LINGO-4-Anteil ausgewählt ist aus der Gruppe bestehend aus Immunglobulin, Serumalbumin, einem Targetierungspolypeptid, einem Reporter-Polypeptid, einem Reinigungserleichterungspolypeptid, Polyalkylenglykol, einem Zuckerpolymer und einer Kombination von zwei oder mehr der genannten Non-LINGO-4-Anteile.

2. Zusammensetzung zur Verwendung nach Anspruch 1, wobei das genannte lösliche LINGO-4-Polypeptid eine LINGO-4-Region umfasst, die ausgewählt ist aus der Gruppe bestehend aus:
(i) einer LINGO-4 Ig Domäne,
(ii) einer LINGO-4 LRR Domäne, und
(iii) einer Kombination der genannten LINGO-4 Domänen.

3. Zusammensetzung zur Verwendung nach Anspruch 1 oder Anspruch 2, wobei dem genannten löslichen LINGO-4 Polypeptid eine LINGO-4 Region fehlt, die ausgewählt ist aus der Gruppe bestehend aus:
(i) einer LINGO-4 Transmembrandomäne,
(ii) einer LINGO-4 Intrazellulärdomäne, und
(iii) einer Kombination der genannten LINGO-4 Domänen.

4. Zusammensetzung zur Verwendung nach einem der Ansprüche 1 - 3, wobei das genannte LINGO-4 Polypeptid eine Aminosäuresequenz umfasst, die wenigstens 90 % identisch oder wenigstens 95 % identisch mit einer Referenz-Aminosäuresequenz ist, die ausgewählt ist aus der Gruppe bestehend aus Aminosäuren 30 bis 411 von SEQ ID NO: 2; Aminosäuren 30 bis 491 von SEQ ID NO: 2; und Aminosäuren 30 bis 534 von SEQ ID NO: 2.

5. Zusammensetzung zur Verwendung nach einem der Ansprüche 1 - 3, wobei das genannte LINGO-4 Polypeptid eine Aminosäuresequenz umfasst, die mit einer Referenz-Aminosäuresequenz identisch ist, die ausgewählt ist aus der Gruppe bestehend aus: Aminosäuren 30 bis 64 von SEQ ID NO: 2; Aminosäuren 30 bis 82 von SEQ ID NO: 2; Aminosäuren 30 bis 106 von SEQ ID NO: 2; Aminosäuren 30 bis 130 von SEQ ID NO: 2; Aminosäuren 30 bis 154 von SEQ ID NO: 2; Aminosäuren 30 bis 178 von SEQ ID NO: 2; Aminosäuren 30 bis 202 von SEQ ID NO: 2; Aminosäuren 30 bis 226 von SEQ ID NO: 2; Aminosäuren 30 bis 298 von SEQ ID NO: 2; Aminosäuren 30 bis 322 von SEQ ID NO: 2; Aminosäuren 30 bis 346 von SEQ ID NO: 2; Aminosäuren 30 bis 411 von SEQ ID NO: 2; Aminosäuren 30 bis 491 von SEQ ID NO: 2; Aminosäuren 30 bis 534 von SEQ ID NO: 2; Aminosäuren Aminosäuren 63 bis 82 von SEQ ID NO: 2; Aminosäuren 63 bis 106 von SEQ ID NO: 2; Aminosäuren 63 bis 130 von SEQ ID NO: 2; Aminosäuren 63 bis 154 von SEQ ID NO: 2; Aminosäuren 63 bis 178 von SEQ ID NO: 2; Aminosäuren 63 bis 202 von SEQ ID NO: 2; Aminosäuren 63 bis 226 von SEQ ID NO: 2; Aminosäuren 63 bis 298 von SEQ ID NO: 2; Aminosäuren 63 bis 322 von SEQ ID NO: 2; Aminosäuren 63 bis 346 von SEQ ID NO: 2; Aminosäuren 63 bis 411 von SEQ ID NO: 2; Aminosäuren 63 bis 491 von SEQ ID NO: 2; Aminosäuren 63 bis 534 von SEQ ID NO: 2; Aminosäuren 83 bis 106 von SEQ ID NO: 2; Aminosäuren 83 bis 130 von SEQ ID NO: 2; Aminosäuren 83 bis 154 von SEQ ID NO: 2; Aminosäuren 83 bis 178 von SEQ ID NO: 2; Aminosäuren 83 bis 202 von SEQ ID NO: 2; Aminosäuren 83 bis 226 von SEQ ID NO: 2; Aminosäuren 83 bis 298 von SEQ ID NO: 2; Aminosäuren 83 bis 322 von SEQ ID NO: 2; Aminosäuren 83 bis 346 von SEQ ID NO: 2; Aminosäuren 83 bis 411 von SEQ ID NO: 2; Aminosäuren 83 bis 491 von SEQ ID NO: 2; Aminosäuren 83 bis 534 von SEQ ID NO: 2; Aminosäuren 107 bis 130 von SEQ ID NO: 2; Aminosäuren 107 bis 154 von SEQ ID NO: 2; Aminosäuren 107 bis 178 von SEQ ID NO: 2; Aminosäuren 107 bis 202 von SEQ ID NO: 2; Aminosäuren 107 bis 226 von SEQ ID NO: 2; Aminosäuren 107 bis 298 von SEQ ID NO: 2; Aminosäuren 107 bis 322 von SEQ ID NO: 2; Aminosäuren 107 bis 346 von SEQ ID NO: 2; Aminosäuren 107 bis 411 von SEQ ID NO: 2; Aminosäuren 107 bis 491 von SEQ ID NO: 2; Aminosäuren 107 bis 534 von SEQ ID NO: 2; Aminosäuren 131 bis 154 von SEQ ID NO: 2; Aminosäuren 131 bis 178 von SEQ ID NO: 2; Aminosäuren 131 bis 202 von SEQ ID NO: 2; Aminosäuren 131 bis 226 von SEQ ID NO: 2; Aminosäuren 131 bis 298 von SEQ ID NO: 2; Aminosäuren 131 bis 322 von SEQ ID NO: 2; Aminosäuren 131 bis 346 von SEQ ID NO: 2; Aminosäuren 131 bis 411 von SEQ ID NO: 2; Aminosäuren 131 bis 491 von SEQ ID NO: 2; Aminosäuren 131 bis 534 von SEQ ID NO: 2; Aminosäuren 155 bis 178 von SEQ ID NO: 2; Aminosäuren 155 bis 202 von SEQ ID NO: 2; Aminosäuren 155 bis 226 von SEQ ID NO: 2; Aminosäuren 155 bis 298 von SEQ ID NO: 2; Aminosäuren 155 bis 322 von SEQ ID NO: 2; Aminosäuren 155 bis 346 von SEQ ID NO: 2; Aminosäuren 155 bis 411 von SEQ ID NO: 2; Aminosäuren 155 bis 491 von SEQ ID NO: 2; Aminosäuren 155 bis 534 von SEQ ID NO: 2; Aminosäuren 179 bis 202 von SEQ ID NO: 2; Aminosäuren 179 bis 226 von SEQ ID NO: 2; Aminosäuren 179 bis 298 von SEQ ID NO: 2; Aminosäuren 179 bis 322 von SEQ ID NO: 2; Aminosäuren 179 bis 346 von SEQ ID NO: 2; Aminosäuren 179 bis 411 von SEQ ID NO: 2; Aminosäuren 179 bis 491 von SEQ ID NO: 2; Aminosäuren 179 bis 534 von SEQ ID NO: 2; Aminosäuren 203 bis 226 von SEQ ID NO: 2; Aminosäuren 203 bis 298 von SEQ ID NO: 2; Aminosäuren 203 bis 322 von SEQ ID NO: 2; Aminosäuren 203 bis 346 von SEQ ID NO: 2; Aminosäuren 203 bis 411 von SEQ ID NO: 2; Aminosäuren 203 bis 491 von SEQ ID NO: 2; Aminosäuren 203 bis 534 von SEQ ID NO: 2; Aminosäuren 275 bis 298 von SEQ ID NO: 2; Aminosäuren 275 bis 322 von SEQ ID NO: 2; Aminosäuren 275 bis 346 von SEQ ID NO: 2; Aminosäuren 275 bis 411 von SEQ ID NO: 2; Aminosäuren 275 bis 491 von SEQ ID NO: 2; Aminosäuren 275 bis 534 von SEQ ID NO: 2; Aminosäuren 299 bis 322 von SEQ ID NO: 2; Aminosäuren 299 bis 346 von SEQ ID NO: 2; Aminosäuren 299 bis 411 von SEQ ID NO: 2; Aminosäuren 299 bis 491 von SEQ ID NO: 2; Aminosäuren 299 bis 534 von SEQ ID NO: 2; Aminosäuren 323 bis 346 von SEQ ID NO: 2; Aminosäuren 323 bis 411 von SEQ ID NO: 2; Aminosäuren 323 bis 491 von SEQ ID NO: 2; Aminosäuren 323 bis 534 von SEQ ID NO: 2; Aminosäuren 358 bis 411 von SEQ ID NO: 2; Aminosäuren 358 bis 491 von SEQ ID NO: 2; Aminosäuren 358 bis 534 von SEQ ID NO: 2; Aminosäuren 426 bis 491 von SEQ ID NO: 2; Aminosäuren 426 bis 534 von SEQ ID NO: 2; und einer Kombination von wenigstens zwei von beliebigen der genannten Polypeptide.

6. Zusammensetzung zur Verwendung nach einem der Ansprüche 1 bis 5, wobei das genannte lösliche LINGO-4 Polypeptid ein cyclisches Peptid ist.

7. Zusammensetzung zur Verwendung nach Anspruch 6, wobei das genannte cyclische Polypeptid ferner ein Biotinmolekül oder ein mit dem N-Terminus verknüpftes acetyliertes Cystein oder ein mit dem C-Terminus verknüpftes Cysteinmolekül umfasst; wobei das genannte Biotinmolekül oder das genannte acetylierte Cysteinmolekül und das genannte Cysteinmolekül zu dem genannten cyclischen Molekül aneinander gefügt sind.

8. Polynukleotid, das das lösliche LINGO-4 Polypeptid, den LINGO-4 Antikörper oder ein Fragment davon oder das LINGO-4 Aptamer gemäß Definition in Anspruch 1 kodiert, zur Verwendung bei der Behandlung einer Krankheit, Störung oder Verletzung, die eine Demyelination oder Dysmyelination beinhaltet, ausgewählt aus der Gruppe bestehend aus multipler Sklerose (MS), progressiver multifokaler Leukenzephalopathie (PML), Enzephalomyelitis (EPL), zentraler pontiner Myelinolyse (CPM), Adrenoleukodystrophie, Alexander-Krankheit, Pelizaeus-Merzbacher-Krankheit, Wallerscher Degeneration, optischer Neuritis, transversaler Myelitis, amylotropher Lateralsklerose (ALS), Huntington-Krankheit, Alzheimer-Krankheit, Parkinson-Krankheit, Rückenmarksverletzung, traumatischer Hirnverletzung, Verletzung nach Bestrahlung, neurologischen Komplikationen von Chemotherapie, Schlaganfall, akuter ischämischer optischer Neuropathie, isoliertem Vitamin-E-Mangelsyndrom, Bassen-Kornzweig-Syndrom, Marchiafava-Bignami-Syndrom, metachromatischer Leukodystrophie, Trigeminus-Neuralgie und Bellscher Parese, wobei das Polynukleotid operativ mit einer Expressionskontrollsequenz verknüpft ist, die eine Expression des genannten LINGO-4 Antagonisten zulässt.

9. Expressionsvektor, der das Polynukleotid gemäß Definition in Anspruch 8 umfasst, zur Verwendung bei der Behandlung einer Krankheit, Störung oder Verletzung, die eine Demyelination oder Dysmyelination beinhaltet, ausgewählt aus der Gruppe bestehend aus multipler Sklerose (MS), progressiver multifokaler Leukenzephalopathie (PML), Enzephalomyelitis (EPL), zentraler pontiner Myelinolyse (CPM), Adrenoleukodystrophie, Alexander-Krankheit, Pelizaeus-Merzbacher-Krankheit, Wallerscher Degeneration, optischer Neuritis, transversaler Myelitis, amylotropher Lateralsklerose (ALS), Huntington-Krankheit, Alzheimer-Krankheit, Parkinson-Krankheit, Rückenmarksverletzung, traumatischer Hirnverletzung, Verletzung nach Bestrahlung, neurologischen Komplikationen von Chemotherapie, Schlaganfall, akuter ischämischer optischer Neuropathie, isoliertem Vitamin-E-Mangelsyndrom, Bassen-Kornzweig-Syndrom, Marchiafava-Bignami-Syndrom, metachromatischer Leukodystrophie, Trigeminus-Neuralgie und Bellscher Parese, wobei das Polynukleotid mit einer Expressionskontrollsequenz operativ verknüpft ist.

10. Expressionsvektor zur Verwendung nach Anspruch 9, wobei der Expressionsvektor ein Virusvektor ist, ausgewählt aus der Gruppe bestehend aus einem Adenovirusvektor, einem Alphavirusvektor, einem Enterovirusvektor, einem Pestivirusvektor, einem Lentivirusvektor, einem Baculovirusvektor, einem Herpesvirusvektor, einem Papovavirusvektor und einem Poxvirusvektor.

11. Zusammensetzung zur Verwendung nach einem der Ansprüche 1 bis 7, wobei die Zusammensetzung zur Verabreichung durch Bolusinjektion oder chronische Infusion, optional direkt in das zentrale Nervensystem formuliert ist.

12. *In-vitro*-Verfahren zum Fördern von Differenzierung oder Überleben von Oligodendrozyten, das das Inkontaktbringen von Oligodendrozyten mit einer Zusammensetzung beinhaltet, die einen LINGO-4-Antagonisten umfasst, der ausgewählt ist aus der Gruppe bestehend aus:
(i) einem löslichen LINGO-4-Polypeptid, optional an einen Non-LINGO-4-Anteil fusioniert;
(ii) einem LINGO-4-Antikörper oder einem Antigen-Bindungsfragment davon;
(iii) einem LINGO-4-Antagonisten-Polynukleotid, ausgewählt aus der Gruppe bestehend aus
(a) einem Antisense-Polynukleotid;
(b) einem Ribozym;
(c) einer kleinen eingreifenden RNA (siRNA); und
(d) einer kleinen Haarnadel-RNA (shRNA);
(iv) einem LINGO-4-Aptamer; und
(v) einer Kombination aus zwei oder mehr der genannten LINGO-4 Antagonisten;
wobei der Non-LINGO-4-Anteil ausgewählt ist aus der Gruppe bestehend aus Immunglobulin, Serumalbumin, einem Targetierungspolypeptid, einem Reporter-Polypeptid, einem Reinigungserleichterungspolypeptid, Polyalkylenglykol, einem Zuckerpolymer und einer Kombination von zwei oder mehr der genannten Non-LINGO-4-Anteile.

13. In-vitro-Verfahren zum Fördern von Myelination von Neuronen oder zum Verhüten von Demyelination von Neuronen, das das Inkontaktbringen eines Gemischs von Neuronen und Oligodendrozyten mit einer Zusammensetzung beinhaltet, die einen LINGO-4 Antagonisten umfasst, der ausgewählt ist aus der Gruppe bestehend aus:
(i) einem löslichen LINGO-4-Polypeptid, optional an einen Non-LINGO-4-Anteil fusioniert;
(ii) einem LINGO-4-Antikörper oder einem Antigen-Bindungsfragment davon;
(iii) einem LINGO-4-Antagonisten-Polynukleotid, ausgewählt aus der Gruppe bestehend aus
(a) einem Antisense-Polynukleotid;
(b) einem Ribozym;
(c) einer kleinen eingreifenden RNA (siRNA); und
(d) einer kleinen Haarnadel-RNA (shRNA);
(iv) einem LINGO-4-Aptamer; und
(v) einer Kombination aus zwei oder mehr der genannten LINGO-4 Antagonisten;
wobei der Non-LINGO-4-Anteil ausgewählt ist aus der Gruppe bestehend aus Immunglobulin, Serumalbumin, einem Targetierungspolypeptid, einem Reporter-Polypeptid, einem Reinigungserleichterungspolypeptid, Polyalkylenglykol, einem Zuckerpolymer und einer Kombination von zwei oder mehr der genannten Non-LINGO-4-Anteile.

## Revendications

1. Composition comprenant un antagoniste de LINGO-4, destinée à une utilisation dans le traitement d'une maladie, d'une affection ou d'une lésion mettant en jeu une démyélinisation ou une dysmyélinisation sélectionnée dans le groupe constitué de la sclérose en plaques (SEP), de la leuco-encéphalopathie multifocale progressive (LMP), de l'encéphalomyélite (EPL), de la myélinolyse centropontique (MCP), de l'adréno-leucodystrophie, de la maladie d'Alexander, de la maladie de Pelizaeus-Merzbacher, de la dégénérescence wallérienne, de la névrite optique, de la myélite transverse, de la sclérose latérale amylotrophique (SLA), de la maladie de Huntington, de la maladie d'Alzheimer, de la maladie de Parkinson, d'une lésion de la moelle épinière, d'une lésion cérébrale traumatique, d'une lésion consécutive à une irradiation, de complications neurologiques d'une chimiothérapie, d'un AVC, d'une neuropathie optique ischémique aiguë, d'un syndrome isolé lié à une carence en vitamine E, du syndrome de Bassen-Kornzweig, du syndrome de Marchiafava-Bignami, de la leucodystrophie métachromatique, de la névralgie du trijumeau, et de la paralysie de Bell, dans laquelle l'antagoniste de LINGO-4 est sélectionné dans le groupe constitué de :
(i) un polypeptide soluble de LINGO-4, optionnellement fixé par fusion sur une fraction non-LINGO-4 ;
(ii) un anticorps anti-LINGO-4 ou un fragment de celui-ci se liant à un antigène ;
(iii) un polynucléotide antagoniste de LINGO-4 sélectionné dans le groupe constitué de :
(a) un polynucléotide antisens ;
(b) un ribozyme ;
(c) un petit ARN interférent (ARNsi) ; et
(d) un petit ARN en épingle à cheveux (ARNsh) ;
(iv) un aptamère de LINGO-4 ; et
(v) une combinaison de deux ou plus desdits antagonistes de LINGO-4 ;
dans laquelle la fraction non-LINGO-4 est sélectionnée dans le groupe constitué d'une immunoglobuline, d'une sérumalbumine, d'un polypeptide de ciblage, d'un polypeptide reporter, d'un polypeptide facilitant la purification, d'un polyalkylène glycol, d'un polymère de sucres, et d'une combinaison de deux ou plus desdites fractions non-LINGO-4.

2. Composition destinée à une utilisation selon la revendication 1, dans laquelle ledit polypeptide soluble de LINGO-4 comprend une région de LINGO-4 sélectionnée dans le groupe constitué de :
(i) un domaine Ig de LINGO-4,
(ii) un domaine LRR de LINGO-4, et
(iii) une combinaison desdits domaines de LINGO-4.

3. Composition destinée à une utilisation selon la revendication 1 ou la revendication 2, dans laquelle il manque audit polypeptide soluble de LINGO-4 une région de LINGO-4 sélectionnée dans le groupe constitué de :
(i) un domaine transmembranaire de LINGO-4,
(ii) un domaine intracellulaire de LINGO-4, et
(iii) une combinaison desdits domaines de LINGO-4.

4. Composition destinée à une utilisation selon l'une quelconque des revendications 1 à 3, dans laquelle ledit polypeptide de LINGO-4 comprend une séquence d'acides aminés ayant une identité d'au moins 90 % ou ayant une identité d'au moins 95 % avec une séquence d'acides aminés de référence sélectionnée dans le groupe constitué des : acides aminés 30 à 411 de la SÉQ. ID n° 2 ; acides aminés 30 à 491 de la SÉQ. ID n° 2 ; et acides aminés 30 à 534 de la SÉQ. ID n° 2.

5. Composition destinée à une utilisation selon l'une quelconque des revendications 1 à 3, dans laquelle ledit polypeptide de LINGO-4 comprend une séquence d'acides aminés identique à une séquence d'acides aminés de référence sélectionnée dans le groupe constitué des : acides aminés 30 à 64 de la SÉQ. ID no 2 ; acides aminés 30 à 82 de la SÉQ. ID n° 2 ; acides aminés 30 à 106 de la SÉQ. ID n° 2 ; acides aminés 30 à 130 de la SÉQ. ID n° 2 ; acides aminés 30 à 154 de la SÉQ. ID n° 2 ; acides aminés 30 à 178 de la SÉQ. ID n° 2 ; acides aminés 30 à 202 de la SÉQ. ID n° 2 ; acides aminés 30 à 226 de la SÉQ. ID n° 2 ; acides aminés 30 à 298 de la SÉQ. ID n° 2 ; acides aminés 30 à 322 de la SÉQ. ID n° 2 ; acides aminés 30 à 346 de la SÉQ. ID n° 2 ; acides aminés 30 à 411 de la SÉQ. ID n° 2 ; acides aminés 30 à 491 de la SÉQ. ID n° 2 ; acides aminés 30 à 534 de la SÉQ. ID n° 2 ; acides aminés acides aminés 63 à 82 de la SÉQ. ID n° 2 ; acides aminés 63 à 106 de la SÉQ. ID n° 2 ; acides aminés 63 à 130 de la SÉQ. ID n° 2 ; acides aminés 63 à 154 de la SÉQ. ID n° 2 ; acides aminés 63 à 178 de la SÉQ. ID n° 2 ; acides aminés 63 à 202 de la SÉQ. ID n° 2 ; acides aminés 63 à 226 de la SÉQ. ID n° 2 ; acides aminés 63 à 298 de la SÉQ. ID n° 2 ; acides aminés 63 à 322 de la SÉQ. ID n° 2 ; acides aminés 63 à 346 de la SÉQ. ID n° 2 ; acides aminés 63 à 411 de la SÉQ. ID n° 2 ; acides aminés 63 à 491 de la SÉQ. ID n° 2 ; acides aminés 63 à 534 de la SÉQ. ID n° 2 ; acides aminés 83 à 106 de la SÉQ. ID n° 2 ; acides aminés 83 à 130 de la SÉQ. ID n° 2 ; acides aminés 83 à 154 de la SÉQ. ID n° 2 ; acides aminés 83 à 178 de la SÉQ. ID n° 2 ; acides aminés 83 à 202 de la SÉQ. ID n° 2 ; acides aminés 83 à 226 de la SÉQ. ID n° 2 ; acides aminés 83 à 298 de la SÉQ. ID n° 2 ; acides aminés 83 à 322 de la SÉQ. ID n° 2 ; acides aminés 83 à 346 de la SÉQ. ID n° 2 ; acides aminés 83 à 411 de la SÉQ. ID n° 2 ; acides aminés 83 à 491 de la SÉQ. ID n° 2 ; acides aminés 83 à 534 de la SÉQ. ID n° 2 ; acides aminés 107 à 130 de la SÉQ. ID n° 2 ; acides aminés 107 à 154 de la SÉQ. ID n° 2 ; acides aminés 107 à 178 de la SÉQ. ID n° 2 ; acides aminés 107 à 202 de la SÉQ. ID n° 2 ; acides aminés 107 à 226 de la SÉQ. ID n° 2 ; acides aminés 107 à 298 de la SÉQ. ID n° 2 ; acides aminés 107 à 322 de la SÉQ. ID n° 2 ; acides aminés 107 à 346 de la SÉQ. ID n° 2 ; acides aminés 107 à 411 de la SÉQ. ID n° 2 ; acides aminés 107 à 491 de la SÉQ. ID n° 2 ; acides aminés 107 à 534 de la SÉQ. ID n° 2 ; acides aminés 131 à 154 de la SÉQ. ID n° 2 ; acides aminés 131 à 178 de la SÉQ. ID n° 2 ; acides aminés 131 à 202 de la SÉQ. ID n° 2 ; acides aminés 131 à 226 de la SÉQ. ID n° 2 ; acides aminés 131 à 298 de la SÉQ. ID n° 2 ; acides aminés 131 à 322 de la SÉQ. ID n° 2 ; acides aminés 131 à 346 de la SÉQ. ID n° 2 ; acides aminés 131 à 411 de la SÉQ. ID n° 2 ; acides aminés 131 à 491 de la SÉQ. ID n° 2 ; acides aminés 131 à 534 de la SÉQ. ID n° 2 ; acides aminés 155 à 178 de la SÉQ. ID n° 2 ; acides aminés 155 à 202 de la SÉQ. ID n° 2 ; acides aminés 155 à 226 de la SÉQ. ID n° 2 ; acides aminés 155 à 298 de la SÉQ. ID n° 2 ; acides aminés 155 à 322 de la SÉQ. ID n° 2 ; acides aminés 155 à 346 de la SÉQ. ID n° 2 ; acides aminés 155 à 411 de la SÉQ. ID n° 2 ; acides aminés 155 à 491 de la SÉQ. ID n° 2 ; acides aminés 155 à 534 de la SÉQ. ID n° 2 ; acides aminés 179 à 202 de la SÉQ. ID n° 2 ; acides aminés 179 à 226 de la SÉQ. ID n° 2 ; acides aminés 179 à 298 de la SÉQ. ID n° 2 ; acides aminés 179 à 322 de la SÉQ. ID n° 2 ; acides aminés 179 à 346 de la SÉQ. ID n° 2 ; acides aminés 179 à 411 de la SÉQ. ID n° 2 ; acides aminés 179 à 491 de la SÉQ. ID n° 2 ; acides aminés 179 à 534 de la SÉQ. ID n° 2 ; acides aminés 203 à 226 de la SÉQ. ID n° 2 ; acides aminés 203 à 298 de la SÉQ. ID n° 2 ; acides aminés 203 à 322 de la SÉQ. ID n° 2 ; acides aminés 203 à 346 de la SÉQ. ID n° 2 ; acides aminés 203 à 411 de la SÉQ. ID n° 2 ; acides aminés 203 à 491 de la SÉQ. ID n° 2 ; acides aminés 203 à 534 de la SÉQ. ID n° 2 ; acides aminés 275 à 298 de la SÉQ. ID n° 2 ; acides aminés 275 à 322 de la SÉQ. ID n° 2 ; acides aminés 275 à 346 de la SÉQ. ID n° 2 ; acides aminés 275 à 411 de la SÉQ. ID n° 2 ; acides aminés 275 à 491 de la SÉQ. ID n° 2 ; acides aminés 275 à 534 de la SÉQ. ID n° 2 ; acides aminés 299 à 322 de la SÉQ. ID n° 2 ; acides aminés 299 à 346 de la SÉQ. ID n° 2 ; acides aminés 299 à 411 de la SÉQ. ID n° 2 ; acides aminés 299 à 491 de la SÉQ. ID n° 2 ; acides aminés 299 à 534 de la SÉQ. ID n° 2 ; acides aminés 323 à 346 de la SÉQ. ID n° 2 ; acides aminés 323 à 411 de la SÉQ. ID n° 2 ; acides aminés 323 à 491 de la SÉQ. ID n° 2 ; acides aminés 323 à 534 de la SÉQ. ID n° 2 ; acides aminés 358 à 411 de la SÉQ. ID n° 2 ; acides aminés 358 à 491 de la SÉQ. ID n° 2 ; acides aminés 358 à 534 de la SÉQ. ID n° 2 ; acides aminés 426 à 491 de la SÉQ. ID n° 2 ; acides aminés 426 à 534 de la SÉQ. ID n° 2 ; et d'une combinaison d'au moins deux de n'importe lesquels desdits polypeptides.

6. Composition destinée à une utilisation selon l'une quelconque des revendications 1 à 5, dans laquelle ledit polypeptide soluble de LINGO-4 est un peptide cyclique.

7. Composition destinée à une utilisation selon la revendication 6, dans laquelle ledit polypeptide cyclique comprend en outre une molécule de biotine ou une cystéine acétylée liée à l'extrémité N-terminale et une molécule de cystéine liée à l'extrémité C-terminale ; dans laquelle ladite molécule de biotine ou ladite molécule de cystéine acétylée et ladite molécule de cystéine sont jointes l'une à l'autre pour former ladite molécule cyclique.

8. Polynucléotide codant pour le polypeptide soluble de LINGO-4, l'anticorps anti-LINGO-4 ou le fragment de celui-ci, ou l'aptamère de LINGO-4 définis dans la revendication 1, destiné à une utilisation dans le traitement d'une maladie, d'une affection ou d'une lésion mettant en jeu une démyélinisation ou une dysmyélinisation sélectionnée dans le groupe constitué de la sclérose en plaques (SEP), de la leuco-encéphalopathie multifocale progressive (LMP), de l'encéphalomyélite (EPL), de la myélinolyse centropontique (MCP), de l'adréno-leucodystrophie, de la maladie d'Alexander, de la maladie de Pelizaeus-Merzbacher, de la dégénérescence wallérienne, de la névrite optique, de la myélite transverse, de la sclérose latérale amylotrophique (SLA), de la maladie de Huntington, de la maladie d'Alzheimer, de la maladie de Parkinson, d'une lésion de la moelle épinière, d'une lésion cérébrale traumatique, d'une lésion consécutive à une irradiation, de complications neurologiques d'une chimiothérapie, d'un AVC, d'une neuropathie optique ischémique aiguë, d'un syndrome isolé lié à une carence en vitamine E, du syndrome de Bassen-Kornzweig, du syndrome de Marchiafava-Bignami, de la leucodystrophie métachromatique, de la névralgie du trijumeau, et de la paralysie de Bell, le polynucléotide étant fonctionnellement lié à une séquence de contrôle d'expression, permettant l'expression dudit antagoniste de LINGO-4.

9. Vecteur d'expression comprenant le polynucléotide défini dans la revendication 8, destiné à une utilisation dans le traitement d'une maladie, d'une affection ou d'une lésion mettant en jeu une démyélinisation ou une dysmyélinisation sélectionnée dans le groupe constitué de la sclérose en plaques (SEP), de la leuco-encéphalopathie multifocale progressive (LMP), de l'encéphalomyélite (EPL), de la myélinolyse centropontique (MCP), de l'adrénoleucodystrophie, de la maladie d'Alexander, de la maladie de Pelizaeus-Merzbacher, de la dégénérescence wallérienne, de la névrite optique, de la myélite transverse, de la sclérose latérale amylotrophique (SLA), de la maladie de Huntington, de la maladie d'Alzheimer, de la maladie de Parkinson, d'une lésion de la moelle épinière, d'une lésion cérébrale traumatique, d'une lésion consécutive à une irradiation, de complications neurologiques d'une chimiothérapie, d'un AVC, d'une neuropathie optique ischémique aiguë, d'un syndrome isolé lié à une carence en vitamine E, du syndrome de Bassen-Kornzweig, du syndrome de Marchiafava-Bignami, de la leucodystrophie métachromatique, de la névralgie du trijumeau, et de la paralysie de Bell, le polynucléotide étant fonctionnellement lié à une séquence de contrôle d'expression.

10. Vecteur d'expression destiné à une utilisation selon la revendication 9, le vecteur d'expression étant un vecteur viral sélectionné dans le groupe constitué d'un vecteur adénoviral, d'un vecteur alphavirus, d'un vecteur entérovirus, d'un vecteur pestivirus, d'un vecteur lentivirus, d'un vecteur baculovirus, d'un vecteur herpèsvirus, d'un vecteur papovavirus, et d'un vecteur poxvirus.

11. Composition destinée à une utilisation selon l'une quelconque des revendications 1 à 7, la composition étant formulée pour une administration par injection bolus ou par perfusion chronique, optionnellement directement dans le système nerveux central.

12. Procédé *in vitro* pour favoriser la différenciation ou la survie d'oligodendrocytes, comprenant la mise en contact d'oligodendrocytes avec une composition comprenant un antagoniste de LINGO-4 sélectionné dans le groupe constitué de :
(i) un polypeptide soluble de LINGO-4, optionnellement fixé par fusion sur une fraction non-LINGO-4 ;
(ii) un anticorps anti-LINGO-4 ou un fragment de celuici se liant à un antigène ;
(iii) un polynucléotide antagoniste de LINGO-4 sélectionné dans le groupe constitué de :
(a) un polynucléotide antisens ;
(b) un ribozyme ;
(c) un petit ARN interférent (ARNsi) ; et
(d) un petit ARN en épingle à cheveux (ARNsh) ;
(iv) un aptamère de LINGO-4 ; et
(v) une combinaison de deux ou plus desdits antagonistes de LINGO-4 ;
dans lequel la fraction non-LINGO-4 est sélectionnée dans le groupe constitué d'une immunoglobuline, d'une sérumalbumine, d'un polypeptide de ciblage, d'un polypeptide reporter, d'un polypeptide facilitant la purification, d'un polyalkylène glycol, d'un polymère de sucres, et d'une combinaison de deux ou plus desdites fractions non-LINGO-4.

13. Procédé *in vitro* pour favoriser la myélinisation de neurones ou pour prévenir la démyélinisation de neurones, comprenant la mise en contact d'un mélange de neurones et d'oligodendrocytes avec une composition comprenant un antagoniste de LINGO-4 sélectionné dans le groupe constitué de :
(i) un polypeptide soluble de LINGO-4, optionnellement fixé par fusion sur une fraction non-LINGO-4 ;
(ii) un anticorps anti-LINGO-4 ou un fragment de celui-ci se liant à un antigène ;
(iii) un polynucléotide antagoniste de LINGO-4 sélectionné dans le groupe constitué de :
(a) un polynucléotide antisens ;
(b) un ribozyme ;
(c) un petit ARN interférent (ARNsi) ; et
(d) un petit ARN en épingle à cheveux (ARNsh) ;
(iv) un aptamère de LINGO-4 ; et
(v) une combinaison de deux ou plus desdits antagonistes de LINGO-4 ;
dans lequel la fraction non-LINGO-4 est sélectionnée dans le groupe constitué d'une immunoglobuline, d'une sérumalbumine, d'un polypeptide de ciblage, d'un polypeptide reporter, d'un polypeptide facilitant la purification, d'un polyalkylène glycol, d'un polymère de sucres, et d'une combinaison de deux ou plus desdites fractions non-LINGO-4.


### SEQUENCE LISTING

<110> Biogen Idee MA Inc.
<120> Use Of LINGO-4 Antagonists In The Treatment Of Conditions Involving Demyelination
<130> P34983EP-PCT
<140> EP08848257.5
   <141> 2008-11-10
<150> US 60/986,492
   <151> 2007-11-08
<160> 41
<170> PatentIn version 3.3
<210> 1
   <211> 2606
   <212> DNA
   <213> Homo sapiens
<400> 1
   gcggccgcag cagcaacagc agcagcagca gcggcaggca gcagccgggc agccaggcag      60
   cgggggttga ggcacacagg gaaggtgcag gggcctgagg tgcagctcga atgggacagg     120
   gcccccagcg ctggacagat gcagtgccaa acttgatgcc accttccagc ttctccggac     180
   tgaagaggga atggatgcag ccacagctcc aaagcaagcc tggcccccat ggcccccgct     240
   ccttttcctc ctcctcctac ctggagggag cggtggcagc tgccctgctg tgtgtgactg     300
   cacctcccag ccccaggctg tgctctgtgg ccacaggcaa ctggaggctg tacctggagg     360
   actcccactg gacactgagc tcctggacct gagtgggaac cgcctgtggg ggctccagca     420
   gggaatgctc tcccgcctga gcctgctcca ggaattggac ctcagctaca accagctctc     480
   aacccttgag cctggggcct tccatggcct acaaagccta ctcaccctga ggctgcaggg     540
   caatcggctc agaatcatgg ggcctggggt cttctcaggc ctctctgctc tgaccctgct     600
   ggacctccgc ctcaaccaga ttgttctctt cctagatgga gcttttgggg agctaggcag     660
   cctccagaag ctggaggttg gggacaacca cctggtattt gtggctccgg gggcctttgc     720
   agggctagcc aagttgagca ccctcaccct ggagcgctgc aacctcagca cagtgcctgg     780
   cctagccctt gcccgtctcc cggcactagt ggccctaagg cttagagaac tggatattgg     840
   gaggctgcca gctggggccc tgcgggggct ggggcagctc aaggagctgg agatccacct     900
   ctggccatct ctggaggctc tggaccctgg gagcctggtt gggctcaatc tcagcagcct     960
   ggccatcact cgctgcaatc tgagctcggt gcccttccaa gcactgtacc acctcagctt    1020
   cctcagggtc ctggatctgt cccagaatcc catctcagcc atcccagccc gaaggctcag    1080
   ccccctggtg cggctccagg agctacgcct gtcaggggca tgcctcacct ccattgctgc    1140
   ccatgccttc catggcttga ctgccttcca cctcctggat gtggcagata acgcccttca    1200
   gacactagag gaaacagctt tcccttctcc agacaaactg gtcaccttga ggctgtctgg    1260
   caacccccta acctgtgact gccgcctcct ctggctgctc cggctccgcc gccacctgga    1320
   ctttggcatg tccccccctg cctgtgctgg cccccatcat gtccagggga agagcctgaa    1380
   ggagttttca gacatcctgc ctccagggca cttcacctgc aaaccagccc tgatccgaaa    1440
   gtcggggcct cgatgggtca ttgcagagga gggcgggcat gcggttttct cctgctctgg    1500
   agatggagac ccagccccca ctgtctcctg gatgaggcct catggggctt ggctgggcag    1560
   ggctgggaga gtaagggtcc tagaggatgg gacactggag atccgctcag tgcagctacg    1620
   ggacagaggg gcctatgtct gtgtggttag caatgtcgct gggaatgact ccctgaggac    1680
   ctggctggaa gtcatccagg tggaaccacc aaacggcaca ctttctgacc ccaacatcac    1740
   cgtgccaggg atcccagggc ctttttttct ggatagcaga ggtgtggcca tggtgctggc    1800
   agtcggcttc ctccccttcc tcacctcagt gaccctctgc tttggcctga ttgccctttg    1860
   gagcaagggc aaaggtcggg tcaaacatca catgaccttt gactttgtgg cacctcggcc    1920
   ctctggggat aaaaactctg ggggtaaccg ggtcactgcc aagctcttct gacctttcct    1980
   tccccagtgg ggaacccacc aagtccgctt cagataccaa aggggaagac agaaccaagg    2040
   ctgcttgaac cagaacctag tcccgagcag caccgctctc ctgcacctcc cgcctgcgtt    2100
   gtgcctcctg ccggagagtc tgcttcctga gcttttccgg tctgaggata gcattgtcat    2160
   ttcttctctg agggtcccag ggagctgcag atgcagaccc cgttgttagt ccagcccccg    2220
   cttcaccccc tccacacaca aaacaggaaa cataatcaaa gcgctagtca gctagtctaa    2280
   ccactaggct ttcttcacac atgcttatat cctttaataa ccaattgcca accacggcta    2340
   taagattatt tcagaggtgg ggctgggaag tgccacttgc tccttagagt ctgtttgtca   2400
   accaggcaga gtccctttct tttctgctcc ccaccccaac cctgccccta tgtacaggaa   2460
   taagagcaaa ggacccacag gctacagaga agaggatggg gacagagtgt gggatggaga    2520
   ggacagacca tatactgcac tgtgtttgca tgagcctcta ccaccttcct ctatctacca    2580
   gatcattaaa cctgctgtca aagggc                                          2606
<210> 2
   <211> 593
   <212> PRT
   <213> Homo sapiens
<400> 2
   Met Asp Ala Ala Thr Ala Pro Lys Gln Ala Trp Pro Pro Trp Pro Pro
   1               5                   10                  15
   Leu Leu Phe Leu Leu Leu Leu Pro Gly Gly Ser Gly Gly Ser Cys Pro
               20                  25                  30
   Ala Val Cys Asp Cys Thr Ser Gln Pro Gln Ala Val Leu Cys Gly His
           35                  40                  45
   Arg Gln Leu Glu Ala Val Pro Gly Gly Leu Pro Leu Asp Thr Glu Leu
       50                  55                  60
   Leu Asp Leu Ser Gly Asn Arg Leu Trp Gly Leu Gln Gln Gly Met Leu
   65                  70                  75                  80
   Ser Arg Leu Ser Leu Leu Gln Glu Leu Asp Leu Ser Tyr Asn Gln Leu
                   85                  90                  95
   Ser Thr Leu Glu Pro Gly Ala Phe His Gly Leu Gln Ser Leu Leu Thr
               100                 105                 110
   Leu Arg Leu Gln Gly Asn Arg Leu Arg Ile Met Gly Pro Gly Val Phe
           115                 120                 125
   Ser Gly Leu Ser Ala Leu Thr Leu Leu Asp Leu Arg Leu Asn Gln Ile
       130                 135                 140
   Val Leu Phe Leu Asp Gly Ala Phe Gly Glu Leu Gly Ser Leu Gln Lys
   145                 150                 155                 160
   Leu Glu Val Gly Asp Asn His Leu Val Phe Val Ala Pro Gly Ala Phe
                   165                 170                 175
   Ala Gly Leu Ala Lys Leu Ser Thr Leu Thr Leu Glu Arg Cys Asn Leu
               180                 185                 190
   Ser Thr Val Pro Gly Leu Ala Leu Ala Arg Leu Pro Ala Leu Val Ala
           195                 200                 205
   Leu Arg Leu Arg Glu Leu Asp Ile Gly Arg Leu Pro Ala Gly Ala Leu
       210                 215                 220
   Arg Gly Leu Gly Gln Leu Lys Glu Leu Glu Ile His Leu Trp Pro Ser
   225                 230                 235                 240
   Leu Glu Ala Leu Asp Pro Gly Ser Leu Val Gly Leu Asn Leu Ser Ser
                   245                 250                 255
   Leu Ala Ile Thr Arg Cys Asn Leu Ser Ser Val Pro Phe Gln Ala Leu
               260                 265                 270
   Tyr His Leu Ser Phe Leu Arg Val Leu Asp Leu Ser Gln Asn Pro Ile
           275                 280                 285
   Ser Ala Ile Pro Ala Arg Arg Leu Ser Pro Leu Val Arg Leu Gln Glu
       290                 295                 300
   Leu Arg Leu Ser Gly Ala Cys Leu Thr Ser Ile Ala Ala His Ala Phe
   305                 310                 315                 320
   His Gly Leu Thr Ala Phe His Leu Leu Asp Val Ala Asp Asn Ala Leu
                   325                 330                 335
   Gln Thr Leu Glu Glu Thr Ala Phe Pro Ser Pro Asp Lys Leu Val Thr
               340                 345                 350
   Leu Arg Leu Ser Gly Asn Pro Leu Thr Cys Asp Cys Arg Leu Leu Trp
           355                 360                 365
   Leu Leu Arg Leu Arg Arg His Leu Asp Phe Gly Met Ser Pro Pro Ala
       370                 375                 380
   Cys Ala Gly Pro His His Val Gln Gly Lys Ser Leu Lys Glu Phe Ser
   385                 390                 395                 400
   Asp Ile Leu Pro Pro Gly His Phe Thr Cys Lys Pro Ala Leu Ile Arg
                   405                 410                 415
   Lys Ser Gly Pro Arg Trp Val Ile Ala Glu Glu Gly Gly His Ala Val
               420                 425                 430
   Phe Ser Cys Ser Gly Asp Gly Asp Pro Ala Pro Thr Val Ser Trp Met
           435                 440                 445
   Arg Pro His Gly Ala Trp Leu Gly Arg Ala Gly Arg Val Arg Val Leu
       450                 455                 460
   Glu Asp Gly Thr Leu Glu Ile Arg Ser Val Gln Leu Arg Asp Arg Gly
   465                 470                 475                 480
   Ala Tyr Val Cys Val Val Ser Asn Val Ala Gly Asn Asp Ser Leu Arg
                   485                 490                 495
   Thr Trp Leu Glu Val Ile Gln Val Glu Pro Pro Asn Gly Thr Leu Ser
               500                 505                 510
   Asp Pro Asn Ile Thr Val Pro Gly Ile Pro Gly Pro Phe Phe Leu Asp
           515                 520                 525
   Ser Arg Gly Val Ala Met Val Leu Ala Val Gly Phe Leu Pro Phe Leu
       530                 535                 540
   Thr Ser Val Thr Leu Cys Phe Gly Leu Ile Ala Leu Trp Ser Lys Gly
   545                 550                 555                 560
   Lys Gly Arg Val Lys His His Met Thr Phe Asp Phe Val Ala Pro Arg
                   565                 570                 575
   Pro Ser Gly Asp Lys Asn Ser Gly Gly Asn Arg Val Thr Ala Lys Leu
               580                 585                 590
   Phe
<210> 3
   <211> 2943
   <212> DNA
   <213> Mus sp.
<400> 3
   gagaagagga gggaaaaaaa aaaaaaaaga aaaaaatgct tcctggctct tttctctcct      60
   ttggtcttgg cagcgcgacc gcagtagcgg cggcagcaac agcagtcttg ccagccggct     120
   gatgcggcag gctgccgggc agtggggagt ggggactcag acacacgggg aaggtggaga     180
   ggccaaggtg cagctcggat gggacaggcc ccagccctgg agagatgcag cgcccaactt    240
   gatgccaccc cccagcttct ccggcctcag gggatggacg cagccactgc tccaaagcaa    300
   gcctggctcc catggtcccc actccttttc ctgctcctcc tgcctggagg gagcatcagt    360
   agctgcccca ctgtgtgtga ctgcacctcc cagacccggg cagtattctg tgcccacagg    420
   cgactggaca ctattcccgg agggcttcca ctggacacag aactcctgga tttgagtggg    480
   aaccgcctgt gggggcttca gcgtggcatg ctctcccgac tgggccagct ccaagaactg    540
   gacctcagct acaaccagct ttccaccctt gagcctgggg ctttccatgg cctacaaagt    600
   ctactcaccc tgaggctgca gggcaatcga ctgagaattg tgggtcctgg gatattctca    660
   ggcctgactg ccctcacact gctggacctc cgcctcaatc agattgtcct ctttctagat    720
   ggagccttta gtgagctagg tagtctccag cagctggagg ttggagataa ccacctggtg    780
   tttgtggctc cgggggcttt tgcagggctg gccaagttaa gtaccatcac tctggaacgt    840
   tgcaacctca gcacagtgcc tggcctagcc cttgcccagc tcccagcact agtagctctt    900
   aggcttcgag aactggatat tgagaggcta ccagctgggg cacttcgagg gctagggcag    960
   ctaaaggagc tggagatcca ccactggcca tctctggagg ctctggatcc agggagcctg   1020
   gttggcctca acctgagcag cctggctatc acccgctgca atctgagctc agtacccttc   1080
   caagcactgc accacttgag cttcctccgg atcttggatc tatctcagaa tcctatctca   1140
   gccatcccag ctcgaaggct cagccccctg gtacggctcc aggagctcag gctgtcagga   1200
   gcttgcctca cctcaatcgc tgctcatgcc ttccacggct tgactgcctt ccacttgctg   1260
   gatgtagcag acaatgctct tcagactcta gaggaaacag cctttccttc tccagacaaa   1320
   ctggtcaccc tgaggctgtc tggtaacccc ctaacctgtg attgccgcct cctctggctc   1380
   ctccgcctcc gccgccgcct ggacttcggc acatcccccc ctgcttgtgc tggcccccaa   1440
   catgtccaag ggaagagcct aagggagttt tcagacattc tgcctccagg ccacttcact   1500
   tgcaaaccag ccctgatccg aaagtcgggg cctcgttggg tcattgcaga ggagggcggg   1560
   catgctgttt tctcctgctc tggagatggg gacccagccc ccactgtttc ctggatgaga   1620
   ccacagggag cttggctagg aagggttggg agagtaaggg tactagagga tggtacactg   1680
   gagatccgct cggtacagct gcgggacagg ggggcctatg tctgtgtagt cagtaatgtc   1740
   gctgggaatg actctctgag aacctggctg gaagttatcc aagttgagcc accaaatggc   1800
   actctgtctg accccaacat cactatgcca gggatcccag ggcctttctt tctggacagc   1860
   aggggtgtgg ctatggtgct agcagtgggt ttcctcccct tcctcacctc agtgaccctc   1920
   tgctttggtc tgattgctct ttggagtaag ggcaagggcc gggtcaagca ccacatgact   1980
   tttgattttg tggcacctcg gccctcgggg gacaagaact ctgggggtaa tcgggtcact   2040
   gccaagttat tctgactttt ccatccatgc taaagaccac ccaagtccac ttcagaagcc   2100
   aaagggagaa gtaggactaa ggtctctgaa ccacagcttc atgccaaaca gcacagcctt   2160
   cccacacctg tcgcctgcat tatgattgct gctctagtct gagcatggca ttgctgcatc   2220
   ttctctgagg gacccaggga actgcagaca cagacctcat cgccagcaca tcccctgatc   2280
   ccaggcaccc actcacacaa ggcaggaaag ctgacaaggc tccggtctgc tctccatgtc   2340
   tgtatatcct ctaatagcca ggaccaggtg ccaaacacaa ccacaagatt gtttcagaag   2400
   tggagctgag aagcatctcc agctttttag agtctgctcc aaggcaggca ggcaggcagg   2460
   caggcaggca ggcaggctcc cgttcttttc tgctacccgg tacccaatcc agccagtgcc   2520
   cttaggtaca ggaagggatt ccagccaagg attccagtgc atgcagggga gtgtggcctc   2580
   tgcctgcagg agcctccacc accttcctga ctgtcacaag ccactgcagt ggcagcagaa   2640
   ggaaacatga tctctggaac ttcatttact tccacctact tcttcccatt ttagccactg   2700
   gtcatctagc ctccacctca caggtgagga gggccaggag cctgcagatg tcagcacttc   2760
   tcatcccctt ggtctgcatc ctttcccctt tcctctcctc tgttgagaca aagaaggcaa   2820
   gatgctgcta tctttggagg gattcctaca cagaactctc ctatttcaca ttgtccgcgg   2880
   ttcccagtgt tgtgtattcc aggcatgctt ggcaaaggga aagccagagg ggaactccta   2940
   ggg                                                                 2943
<210> 4
   <211> 618
   <212> PRT
   <213> Mus sp.
<400> 4
   Met Gly Gln Ala Pro Ala Leu Glu Arg Cys Ser Ala Gln Leu Asp Ala
   1               5                   10                  15
   Thr Pro Gln Leu Leu Arg Pro Gln Gly Met Asp Ala Ala Thr Ala Pro
               20                  25                  30
   Lys Gln Ala Trp Leu Pro Trp Ser Pro Leu Leu Phe Leu Leu Leu Leu
           35                  40                  45
   Pro Gly Gly Ser Ile Ser Ser Cys Pro Thr Val Cys Asp Cys Thr Ser
       50                  55                  60
   Gln Thr Arg Ala Val Phe Cys Ala His Arg Arg Leu Asp Thr Ile Pro
   65                  70                  75                  80
   Gly Gly Leu Pro Leu Asp Thr Glu Leu Leu Asp Leu Ser Gly Asn Arg
                   85                  90                  95
   Leu Trp Gly Leu Gln Arg Gly Met Leu Ser Arg Leu Gly Gln Leu Gln
               100                 105                 110
   Glu Leu Asp Leu Ser Tyr Asn Gln Leu Ser Thr Leu Glu Pro Gly Ala
           115                 120                 125
   Phe His Gly Leu Gln Ser Leu Leu Thr Leu Arg Leu Gln Gly Asn Arg
       130                 135                 140
   Leu Arg Ile Val Gly Pro Gly Ile Phe Ser Gly Leu Thr Ala Leu Thr
   145                 150                 155                 160
   Leu Leu Asp Leu Arg Leu Asn Gln Ile Val Leu Phe Leu Asp Gly Ala
                   165                 170                 175
   Phe Ser Glu Leu Gly Ser Leu Gln Gln Leu Glu Val Gly Asp Asn His
               180                 185                 190
   Leu Val Phe Val Ala Pro Gly Ala Phe Ala Gly Leu Ala Lys Leu Ser
           195                 200                 205
   Thr Ile Thr Leu Glu Arg Cys Asn Leu Ser Thr Val Pro Gly Leu Ala
       210                 215                 220
   Leu Ala Gln Leu Pro Ala Leu Val Ala Leu Arg Leu Arg Glu Leu Asp
   225                 230                 235                 240
   Ile Glu Arg Leu Pro Ala Gly Ala Leu Arg Gly Leu Gly Gln Leu Lys
                   245                 250                 255
   Glu Leu Glu Ile His His Trp Pro Ser Leu Glu Ala Leu Asp Pro Gly
               260                 265                 270
   Ser Leu Val Gly Leu Asn Leu Ser Ser Leu Ala Ile Thr Arg Cys Asn
   275 280 285
   Leu Ser Ser Val Pro Phe Gln Ala Leu His His Leu Ser Phe Leu Arg
   290 295 300
   Ile Leu Asp Leu Ser Gln Asn Pro Ile Ser Ala Ile Pro Ala Arg Arg
   305 310 315 320
   Leu Ser Pro Leu Val Arg Leu Gln Glu Leu Arg Leu Ser Gly Ala Cys
   325 330 335
   Leu Thr Ser Ile Ala Ala His Ala Phe His Gly Leu Thr Ala Phe His
   340 345 350
   Leu Leu Asp Val Ala Asp Asn Ala Leu Gln Thr Leu Glu Glu Thr Ala
   355 360 365
   Phe Pro Ser Pro Asp Lys Leu Val Thr Leu Arg Leu Ser Gly Asn Pro
   370 375 380
   Leu Thr Cys Asp Cys Arg Leu Leu Trp Leu Leu Arg Leu Arg Arg Arg
   385 390 395 400
   Leu Asp Phe Gly Thr Ser Pro Pro Ala Cys Ala Gly Pro Gln His Val
   405 410 415
   Gln Gly Lys Ser Leu Arg Glu Phe Ser Asp Ile Leu Pro Pro Gly His
   420 425 430
   Phe Thr Cys Lys Pro Ala Leu Ile Arg Lys Ser Gly Pro Arg Trp Val
   435 440 445
   Ile Ala Glu Glu Gly Gly His Ala Val Phe Ser Cys Ser Gly Asp Gly
   450 455 460
   Asp Pro Ala Pro Thr Val Ser Trp Met Arg Pro Gln Gly Ala Trp Leu
   465 470 475 480
   Gly Arg Val Gly Arg Val Arg Val Leu Glu Asp Gly Thr Leu Glu Ile
   485 490 495
   Arg Ser Val Gln Leu Arg Asp Arg Gly Ala Tyr Val Cys Val Val Ser
   500 505 510
   Asn Val Ala Gly Asn Asp Ser Leu Arg Thr Trp Leu Glu Val Ile Gln
           515                 520                 525
   Val Glu Pro Pro Asn Gly Thr Leu Ser Asp Pro Asn Ile Thr Met Pro
       530                 535                 540
   Gly Ile Pro Gly Pro Phe Phe Leu Asp Ser Arg Gly Val Ala Met Val
   545                 550                 555                 560
   Leu Ala Val Gly Phe Leu Pro Phe Leu Thr Ser Val Thr Leu Cys Phe
                   565                 570                 575
   Gly Leu Ile Ala Leu Trp Ser Lys Gly Lys Gly Arg Val Lys His His
               580                 585                 590
   Met Thr Phe Asp Phe Val Ala Pro Arg Pro Ser Gly Asp Lys Asn Ser
           595                 600                 605
   Gly Gly Asn Arg Val Thr Ala Lys Leu Phe
       610                 615
<210> 5
   <211> 15
   <212> PRT
   <213> Artificial
<220>
   <223> Synthetic linker sequence
<400> 5
   Gly Gly Gly Gly Ser Gly Gly Gly Gly Ser Gly Gly Gly Gly Ser
   1               5                   10                  15
<210> 6
   <211> 10
   <212> PRT
   <213> Artificial
<220>
   <223> Synthetic linker sequence
<400> 6
   Gly Gly Gly Gly Ser Gly Gly Gly Gly Ser
   1               5                   10
<210> 7
   <211> 15
   <212> PRT
   <213> Artificial
<220>
   <223> Synthetic linker sequence
<400> 7
   Glu Ser Gly Arg Ser Gly Gly Gly Gly Ser Gly Gly Gly Gly Ser
   1               5                   10                  15
<210> 8
   <211> 14
   <212> PRT
   <213> Artificial
<220>
   <223> Synthetic linker sequence
<400> 8
   Glu Gly Lys Ser Ser Gly Ser Gly Ser Glu Ser Lys Ser Thr
   1               5                   10
<210> 9
   <211> 15
   <212> PRT
   <213> Artificial
<220>
   <223> Synthetic linker sequence
<400> 9
   Glu Gly Lys Ser Ser Gly Ser Gly Ser Glu Ser Lys Ser Thr Gln
   1               5                   10                  15
<210> 10
   <211> 14
   <212> PRT
   <213> Artificial
<220>
   <223> Synthetic linker sequence
<400> 10
   Glu Gly Lys Ser Ser Gly Ser Gly Ser Glu Ser Lys Val Asp
   1               5                   10
<210> 11
   <211> 14
   <212> PRT
   <213> Artificial
<220>
   <223> Synthetic linker sequence
<400> 11
   Gly Ser Thr Ser Gly Ser Gly Lys Ser Ser Glu Gly Lys Gly
   1 5 10
<210> 12
   <211> 18
   <212> PRT
   <213> Artificial
<220>
   <223> Synthetic linker sequence
<400> 12 Leu Asp
<210> 13
   <211> 16
   <212> PRT
   <213> Artificial
<220>
   <223> Synthetic linker sequence
<400> 13
   Glu Ser Gly Ser Val Ser Ser Glu Glu Leu Ala Phe Arg Ser Leu Asp
   1 5 10 15
<210> 14
   <211> 8
   <212> PRT
   <213> Artificial
<220>
   <223> Synthetic FLAG peptide
<400> 14
   Asp Tyr Lys Asp Asp Asp Asp Lys
   1 5
<210> 15
   <211> 8
   <212> PRT
   <213> Artificial
<220>
   <223> Synthetic FLAG peptide
<400> 15
   Asp Tyr Lys Asp Glu Asp Asp Lys
   1 5
<210> 16
   <211> 9
   <212> PRT
   <213> Artificial
<220>
   <223> Synthetic STREP epitope
<400> 16
   Ala Trp Arg His Pro Gln Phe Gly Gly
   1 5
<210> 17
   <211> 11
   <212> PRT
   <213> Artificial
<220>
   <223> Synthetic VSV-G epitope
<400> 17
   Tyr Thr Asp Ile Glu Met Asn Arg Leu Gly Lys
   1 5 10
<210> 18
   <211> 6
   <212> PRT
   <213> Artificial
<220>
   <223> Synthetic poly-His
<400> 18
   His His His His His His
   1 5
<210> 19
   <211> 13
   <212> PRT
   <213> Artificial
<220>
   <223> Synthetic influenza HA
<400> 19
   Tyr Pro Tyr Asp Val Pro Asp Tyr Ala Ile Glu Gly Arg
   1 5 10
<210> 20
   <211> 11
   <212> PRT
   <213> Artificial
<220>
   <223> Synthetic c-myc
<400> 20
   Glu Gln Lys Leu Leu Ser Glu Glu Asp Leu Asn
   1               5                   10
<210> 21
   <211> 18
   <212> DNA
   <213> Artificial
<220>
   <223> Synthetic LINGO-4 forward primer
<400> 21
   gagcctggtt ggcctcaa 18
<210> 22
   <211> 20
   <212> DNA
   <213> Artificial
<220>
   <223> Synthetic LINGO-4 reverse primer
<400> 22
   gcagtgcttg gaagggtact 20
<210> 23
   <211> 16
   <212> DNA
   <213> Artificial
<220>
   <223> Synthetic LINGO-4 FAB-labeled probe
<400> 23
   cagcctggct atcacc 16
<210> 24
   <211> 22
   <212> DNA
   <213> Artificial
<220>
   <223> Synthetic LINGO-1 forward primer
<400> 24
   ctttcccctt cgacatcaag ac 22
<210> 25
   <211> 18
   <212> DNA
   <213> Artificial
<220>
   <223> Synthetic LINGO-1 reverse primer
<400> 25
   cagcagcacc aggcagaa 18
<210> 26
   <211> 23
   <212> DNA
   <213> Artificial
<220>
   <223> Synthetic LINGO-1 FAM-labeled probe
<400> 26
   atcgccacca ccatgggctt cat 23
<210> 27
   <211> 25
   <212> DNA
   <213> Artificial
<220>
   <223> Synthetic LINGO-2 forward primer
<400> 27
   accttgtata cctgacccac cttaa 25
<210> 28
   <211> 25
   <212> DNA
   <213> Artificial
<220>
   <223> Synthetic LINGO-2 reverse primer
<400> 28
   agagaacatg ccagcttcaa tagtg 25
<210> 29
   <211> 16
   <212> DNA
   <213> Artificial
<220>
   <223> Synthetic LINGO-2 FAM-labeled probe
<400> 29
   cctctcctac aatccc 16
<210> 30
   <211> 17
   <212> DNA
   <213> Artificial
<220>
   <223> Synthetic LINGO-3 forward primer
<400> 30
   cgcggctcct tcagaga 17
<210> 31
   <211> 17
   <212> DNA
   <213> Artificial
<220>
   <223> Synthetic LINGO-3 reverse primer
<400> 31
   ggctcctgct aggtgca 17
<210> 32
   <211> 16
   <212> DNA
   <213> Artificial
<220>
   <223> Synthetic LINGO-3 FAM-labeled probe
<400> 32
   ctggtgcgcc tgcgtg 16
<210> 33
   <211> 47
   <212> DNA
   <213> Artificial
<220>
   <223> Synthetic LINGO-4 forward primer
<400> 33
   tttttgcggc cgccaccatg gatgcagcca cagctccaaa gcaagcc 47
<210> 34
   <211> 48
   <212> DNA
   <213> Artificial
<220>
   <223> Synthetic LINGO-4 reverse primer
<400> 34
   tttttgcggc cgctcagaag agcttggcag tgacccggtt acccccag 48
<210> 35
   <211> 88
   <212> DNA
   <213> Artificial
<220>
   <223> Synthetic pJST1011 forward primer
<400> 35
   ctcctcctac ctggagggag cggtggctac ccttacgacg tccctgatta cgctagctgc 60
   cctgctgtgt gtgactgcac ctcccagc 88
<210> 36
   <211> 88
   <212> DNA
   <213> Artificial
<220>
   <223> Synthetic pJST1011 reverse primer
<400> 36
   gctgggaggt gcagtcacac acagcagggc agctagcgta atcagggacg tcgtaagggt 60
   agccaccgct ccctccaggt aggaggag 88
<210> 37
   <211> 20
   <212> DNA
   <213> Artificial
<220>
   <223> Synthetic pJST1037 forward primer
<400> 37
   aggaaacagc tatgaccatg 20
<210> 38
   <211> 47
   <212> DNA
   <213> Artificial
<220>
   <223> Synthetic pJST1037 reverse primer
<400> 38
   tttttgcggc cgctcaacct ttgcccttgc tccaaagggc aatcagg 47
<210> 39
   <211> 20
   <212> DNA
   <213> Artificial
<220>
   <223> Synthetic pJST1011 forward primer
<400> 39
   aggaaacagc tatgaccatg 20
<210> 40
   <211> 36
   <212> DNA
   <213> Artificial
<220>
   <223> Synthetic pJST1011 reverse primer
<400> 40
   aaaaaggtcg accatggcca cacctctgct atccag 36
<210> 41
   <211> 8
   <212> PRT
   <213> Artificial
<220>
   <223> Synthetic cyclic LINGO-4 polypeptide
<220>
   <221> variant
   <222> (5)..(5)
   <223> Can be Arg, His, Gin, or Asn
<220>
   <221> variant
   <222> (6)..(6)
   <223> Can be Arg, His, Gln, or Asn
<220>
   <221> variant
   <222> (7)..(7)
   <223> Can be Arg, His, Gln, or Asn
<400> 41
   Cys Leu Ser Pro Lys Lys Lys Cys
   1               5